# EUROPEAN PATENT APPLICATION

(11) **EP 2 338 490 A2**
(43) Date of publication of application: **29.06.2011**
(21) Application number: 10177019.6
(22) Date of filing: 29.10.2004
(51) Int. Cl.: A61K 31/473, A61K 31/4164, A61K 31/4184, A61P 25/00, A61P 25/02, A61P 25/08, A61P 25/14, A61P 25/16, A61P 25/18, A61P 25/20, A61P 25/22, A61P 25/24, A61P 25/28, A61P 25/32, A61P 9/02

(54) **Combinations Useful for the Treatment of Neuronal Disorders**

(30) Priority: 03.11.2003 US 516717 P
(62) Divisional of application: 04791058.3
(71) Applicant: Probiodrug AG, 06120 Halle/Saale (DE)
(72) Inventor: Demuth, Hans-Ulrich, 06120, Halle / Saale (DE); Niestroj, André J., 06193, Sennewitz (DE); Schilling, Stephan, 06130, Halle / Saale (DE); Rossner, Steffen, 04849, Bad Dueben (DE); Schulz, Ingo, 06120, Halle / Saale (DE)
(74) Representative: Hoffmann, Matthias

(57) **Abstract**

The present invention provides a method for the treatment of neuronal disorders, in a mammal such as a human, which method comprises administering an effective, nontoxic and pharmaceutically acceptable amount of at least one QC-inhibitor, optionally in combination with at least one agent, selected from the group consisting of PEP-inhibitors, inhibitors of DP IV/DP IV-like enzymes, NPY-receptor ligands, NPY agonists, NPY antagonists, ACE-inhibitors, PIMT enhancers, inhibitors of beta secretases, inhibitors of gamma secretases and inhibitors of neutral endopeptidase, to a mammal in need thereof.

SEQUENCE LISTING

<110>  Probiodrug AG

<120>  Combinations useful for the treatment of neuronal disorders

<130>  PBD 00037/EPT/D1

<140>  PCT/EP 2004/012301
<141>  2004-10-29

<150>  US 60/516,717
<151>  2003-11-03

<160>  15

<170>  PatentIn version 3.1

<210>  1
<211>  42
<212>  PRT
<213>  Homo sapiens

<400>  1

<210>  2
<211>  40
<212>  PRT
<213>  Homo sapiens

<400>  2

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

<210>  3

<211>  40

<212>  PRT

<213>  Homo sapiens

<400>  3

Glu Phe Arg His Asp Ser Gly Tyr Glu Val His His Gln Lys Leu Val
1               5                   10                  15

Phe Phe Ala Glu Asp Val Gly Ser Asn Lys Gly Ala Ile Ile Gly Leu
            20                  25                  30

Met Val Gly Gly Val Val Ile Ala
        35                  40

<210>  4

<211>  38

<212>  PRT

<213>  Homo sapiens

<400>  4

Glu Phe Arg His Asp Ser Gly Tyr Glu Val His His Gln Lys Leu Val
1               5                   10                  15

Phe Phe Ala Glu Asp Val Gly Ser Asn Lys Gly Ala Ile Ile Gly Leu
            20                  25                  30

Met Val Gly Gly Val Val
        35

<210>  5

<211>  11

<212>  PRT

<213>  Artificial sequence

<220>
<223>  Synthetic peptide
<220>
<221>  MOD_RES
<222>  (11)..(11)
<223>  AMIDATION

<400>  5
Asp Ala Glu Phe Arg His Asp Ser Gly Tyr Glu
1               5                   10

<210>  6
<211>  9
<212>  PRT
<213>  Artificial sequence

<220>
<223>  synthetic peptide
<220>
<221>  MOD_RES
<222>  (9)..(9)
<223>  AMIDATION

<400>  6
Glu Phe Arg His Asp Ser Gly Tyr Glu
1               5

<210>  7
<211>  21
<212>  PRT
<213>  Artificial sequence

<220>
<223>  synthetic peptide
<220>

<221>  MOD_RES

<222>  (21)..(21)

<223>  AMIDATION


<400>  7

Asp Ala Glu Phe Arg His Asp Ser Gly Tyr Glu Val His His Gln Lys
1               5                   10                  15

Leu Val Phe Phe Ala
            20


<210>  8

<211>  19

<212>  PRT

<213>  Artificial sequence


<220>

<223>  synthetic peptide

<220>

<221>  MOD_RES

<222>  (19)..(19)

<223>  AMIDATION


<400>  8

Glu Phe Arg His Asp Ser Gly Tyr Glu Val His His Gln Lys Leu Val
1               5                   10                  15

Phe Phe Ala


<210>  9

<211>  38

<212>  PRT

<213>  Artificial sequence


<220>

<223>  Synthetic peptide

<400>  9

Gln Phe Arg His Asp Ser Gly Tyr Glu Val His His Gln Lys Leu Val
1               5                   10                  15

Phe Phe Ala Glu Asp Val Gly Ser Asn Lys Gly Ala Ile Ile Gly Leu
            20                  25                  30

Met Val Gly Gly Val Val
        35

<210>  10

<211>  19

<212>  PRT

<213>  Artificial sequence


<220>

<223>  Synthetic peptide

<220>

<221>  MOD_RES

<222>  (19)..(19)

<223>  AMIDATION


<400>  10

Gln Phe Arg His Asp Ser Gly Tyr Glu Val His His Gln Lys Leu Val
1               5                   10                  15

Phe Phe Ala


<210>  11

<211>  11

<212>  PRT

<213>  Artificial sequence


<220>

<223>  Synthetic peptide

<220>

<221>  MOD_RES

<222>  (11)..(11)

<223>  AMIDATION

<400>  11

Asp Ala Gln Phe Arg His Asp Ser Gly Tyr Glu
1               5                   10


<210>  12

<211>  9

<212>  PRT

<213>  Artificial sequence



<220>

<223>  Synthetic peptide

<220>

<221>  MOD_RES

<222>  (9)..(9)

<223>  AMIDATION


<400>  12

Gln Phe Arg His Asp Ser Gly Tyr Glu
1               5


<210>  13

<211>  32

<212>  PRT

<213>  Artificial sequence



<220>

<223>  synthetic peptide

<400>  13

Glu Val His His Gln Lys Leu Val Phe Phe Ala Glu Asp Val Gly Ser
1               5                   10                  15


Asn Lys Gly Ala Ile Ile Gly Leu Met Val Gly Gly Val Val Ile Ala
            20                  25                  30


<210>  14

<211>  30

<212>  PRT

<213>  Artificial sequence

<220>

<223>  synthetic peptide

<400>  14

Glu Val His His Gln Lys Leu Val Phe Phe Ala Glu Asp Val Gly Ser
1               5                   10                  15

Asn Lys Gly Ala Ile Ile Gly Leu Met Val Gly Gly Val Val
            20                  25                  30

<210>  15

<211>  33

<212>  PRT

<213>  Artificial sequence

<220>

<223>  synthetic peptide

<400>  15

Glu Val His His Gln Lys Leu Val Phe Phe Ala Glu Asp Val Gly Ser
1               5                   10                  15

Asn Lys Gly Ala Ile Ile Gly Leu Met Val Gly Gly Val Val Ile Ala
            20                  25                  30

Thr

## Description

### Field of the Invention

The invention relates to combinations of inhibitors of glutaminyl cyclase and prolyl endopeptidase and their use for treating neuronal disorders (e.g., Alzheimer's disease, Down Syndrome, Parkinson disease, Chorea Huntington, pathogenic psychotic conditions, schizophrenia, impaired food intake, sleep-wakefulness, impaired homeostatic regulation of energy metabolism, impaired autonomic function, impaired hormonal balance, impaired regulation, body fluids, hypertension, fever, sleep dysregulation, anorexia, anxiety related disorders including depression, seizures including epilepsy, drug withdrawal and alcoholism, neurodegenerative disorders including cognitive dysfunction and dementia).

### Background of the Invention

Glutaminyl cyclase (QC, EC 2.3.2.5) catalyzes the intramolecular cyclization of N-terminal glutamine residues and N-terminal glutamate residues of peptides and proteins into pyroglutamic acid (pGlu*) liberating ammonia or water, respectively (Schilling, S. et al. 2004 FEBS Lett 563, 191-196). A QC was first isolated by Messer from the latex of the tropical plant Carica papaya in 1963 (Messer, M. 1963 Nature 4874, 1299). 24 years later, a corresponding enzymatic activity was discovered in animal pituitary (Busby, W. H. J. et al. 1987 J Biol Chem 262, 8532-8536; Fischer, W. H. and Spiess, J. 1987 Proc Natl Acad Sci U S A 84, 3628-3632). For the mammalian QC, the conversion of Gln into pGlu by QC could be shown for the precursors of TRH and GnRH (Busby, W. H. J. et al. 1987 J Biol Chem 262, 8532-8536; Fischer, W. H. and Spiess, J. 1987 Proc Natl Acad Sci U S A 84, 3628-3632). In addition, initial localization experiments of QC revealed a co-localization with its putative products of catalysis in bovine pituitary, further improving the suggested function in peptide hormone synthesis (Bockers, T. M. et al. 1995 J Neuroendocrinol 7, 445-453). In contrast, the physiological function of the plant QC is less clear. In case of the enzyme from C. papaya, a role in the plant defense against pathogenic microorganisms was suggested (El Moussaoui, A. et al.2001 Cell Mol Life Sci 58, 556-570). Putative QCs from other plants were identified by sequence comparisons recently (Dahl, S. W. et al.2000 Protein Expr Purif 20, 27-36). The physiological function of these enzymes, however, is still ambiguous.

The QCs known from plants and animals show a strict specificity for L-Glutamine in the N-terminal position of the substrates and their kinetic behavior was found to obey the Michaelis-Menten equation (Pohl, T. et al. 1991 Proc Natl Acad Sci U S A 88, 10059-10063; Consalvo, A. P. et al. 1988 Anal Biochem 175, 131-138; Gololobov, M. Y. et al. 1996 Biol Chem Hoppe Seyler 377, 395-398). A comparison of the primary structures of the QCs from C. papaya and that of the highly conserved QC from mammals, however, did not reveal any sequence homology (Dahl, S. W. et al. 2000 Protein Expr Purif 20, 27-36). Whereas the plant QCs appear to belong to a new enzyme family (Dahl, S. W. et al. 2000 Protein Expr Purif 20, 27-36), the mammalian QCs were found to have a pronounced sequence homology to bacterial aminopeptidases (Bateman, R. C. et al. 2001 Biochemistry 40, 11246-11250), leading to the conclusion that the QCs from plants and animals have different evolutionary origins.

EP 02 011 349.4 discloses polynucleotides encoding insect glutaminyl cyclase, as well as polypeptides encoded thereby. This application further provides host cells comprising expression vectors comprising polynucleotides of the invention. Isolated polypeptides and host cells comprising insect QC are useful in methods of screening for agents that reduce glutaminyl cyclase activity. Such agents are useful as pesticides.

Peptide bonds linked to proline appear to be relatively resistant to the broad-specificity peptidases (Mentlein, 1988), suggesting that peptidases that hydrolyze peptide bonds containing proline may be important in the metabolism of proline-containing peptides (Atack, et al., Eur. J. of Pharm., 205, 157-163 (1991)). Prolyl endopeptidase appears to play such a role in the metabolism of biologically active proline containing peptides. The enzyme hydrolyzes many biologically active peptides containing proline, such as oxytocin, thyrotropin releasing hormone, luteinizing hormone releasing hormone, angiotensin II, bradykinin, substance P, neurotensin and vasopressin.

Prolyl endopeptidase acts to degrade active peptides as a carboxy terminal proline cleaving enzyme. Specifically, prolyl endopeptidase acts by hydrolyzing peptide bonds on the carboxy side of proline residues. Prolyl endopeptidase is thought mechanistically to act as a serine protease, cleaving peptide bonds by a mechanism similar to other serine proteases such as α-chymotrypsin, trypsin, and subtilisins. Although the enzyme universally acts at peptide bonds containing proline derivatives, the enzyme form appears to vary in different tissue sources, wherein the enzyme shows differences in substrate specificity. Prolyl endopeptidase has been purified from a number of plant (carrots, mushrooms), microbial (Flavobacterium menigosepticum) and animal tissues. In animals, the enzyme is found ubiquitously throughout the body, however, prolyl endopeptidase is generally found in highest concentrations within the CNS (Wilk, 1983). Common sources of the enzyme for testing substrates against animal sources have been bovine, rat, and mouse brain.

Low molecular weight inhibitors of prolyl endopeptidase have been studied. These inhibitors are generally chemical derivatives of proline or small peptides containing terminal prolines. Benzyloxycarbonyl-prolyl-prolinal has been shown to be a specific transition state inhibitor of the enzyme (Wilk, S. and Orloeski, M., J. Neurochem., 41, 69 (1983), Friedman, et al., Neurochem., 42, 237 (1984)). N-terminal substitutions of L-proline or L-prolylpyrrolidine (Atack, et al., Eur. J. of Pharm., 205, 157-163 (1991), JP 03 56,460, EP 384,341), as well as variations of N-benzyloxycarbonyl (Z) dipeptides containing prolinal at the carboxy terminus have been synthesized as prolyl endopeptidase inhibitors (Nishikata, et al., Chem. Pharm. Bull. 34(7), 2931-2936 (1986), Baker, A. et al., Bioorganic & Medicinal Chem. Letts., 1(11), 585-590 (1991)). Thioproline, thiazolidine, and oxopyrrolidine substitutions of the core structure have been reported to inhibit prolyl endopeptidase (Tsuru, et al., J. Biochem., 94, 1179 (1988), Tsuru, et al., J. Biochem., 104, 580-586 (1988), Saito et al., J. Enz. Inhib. 5, 51-75 (1991), Uchida, I., et al. PCT Int. Appl. WO 90 12,005, JP 03 56,461, JP 03 56,462). Similarly, various modifications of the carboxy terminal proline have been made, including various fluorinated ketone derivatives (Henning, EP 4,912,127). General syntheses of fluorinated ketone derivatives has been described (Angelastro, M.R., et al., Tetrahedron Letters 33(23), 3265-3268 (1992)). Other compounds such as chloromethyl ketone derivatives of acyl-proline or acylpeptide-proline (Z-Gly-Pro-CH₂Cl) have been demonstrated to inhibit the enzyme by alkylating the enzyme's active site (Yoshimoto, T., et al., Biochemistry 16, 2942 (1977)).

EP-A-0 286 928 discloses 2-acylpyrrolidine derivatives useful as propyl endopeptidase inhibitors.

Further known prolyl endopeptidase inhibitors are, e.g. Fmoc-Ala-Pyrr-CN and those listed below:

| **Z-321** | **ONO-1603** |
|---|---|
| **Zeria Pharmaceutical Co Ltd** | **Ono Pharmaceutical Co Ltd** |
| 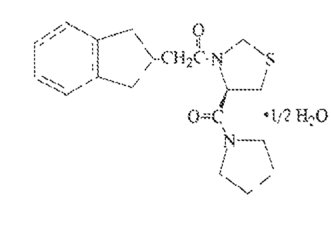 | 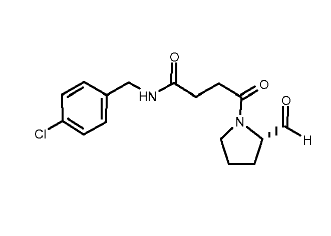 |
| **(4R)-3-(indan-2-ylacetyl)-4-(1-pyrrolidinyl-carbonyl)-1,3-thiazolidin** | **(S)-1-[N-(4-chlorobenzyl)-succinamoyl]pyrrolidin-2-carbaldehyd** |

| **JTP-4819** | **S-17092** |
|---|---|
| **Japan Tobacco Inc** | **Servier** |
| 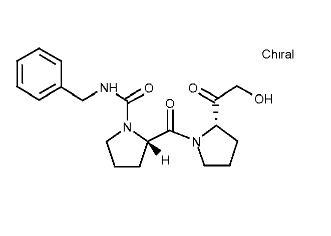 | 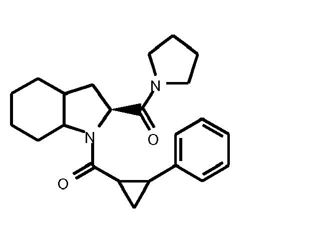 |
| **(S)-2-{[(S).(hydroxyacatyl)-1-pyrrolidinyl] carbonyl}-N-(phenylmethyl)-1-pyrrolidin-carboxamid** | **(2S, 3aS, 7aS)-1{[(R,R)-2-phenylcyclopropyl] carbonyl}-2-[(thiazolidin-3-yl)carbonyl] octahydro-*1H*-indol** |

Further prolyl endopeptidase inhibitors are disclosed in JP 01042465, JP 03031298, JP 04208299, WO 0071144, US 5847155; JP 09040693, JP 10077300, JP 05331072, JP 05015314, WO 9515310, WO 9300361, EP 0556482, JP 06234693, JP 01068396, EP 0709373, US 5965556, US 5756763, US 6121311, JP 63264454, JP 64000069, JP 63162672, EP 0268190, EP 0277588, EP 0275482, US 4977180, US 5091406, US 4983624, US 5112847, US 5100904, US 5254550, US 5262431, US 5340832, US 4956380, EP 0303434, JP 03056486, JP 01143897, JP 1226880, EP 0280956, US 4857537, EP 0461677, EP 0345428, 4JP 02275858, US 5506256, JP 06192298, EP 0618193, JP 03255080, EP 0468469, US 5118811, JP 05025125, WO 9313065, JP 05201970, WO 9412474, EP 0670309, EP 0451547, JP 06339390, US 5073549, US 4999349, EP 0268281, US 4743616, EP 0232849, EP 0224272, JP 62114978, JP 62114957, US 4757083, US 4810721, US 5198458, US 4826870, EP 0201742, EP 0201741, US 4873342, EP 0172458, JP 61037764, EP 0201743, US 4772587, EP 0372484, US 5028604, WO 9118877, JP 04009367, JP 04235162, US 5407950, WO 9501352, JP 01250370, JP 02207070, US 5221752, EP 0468339, JP 04211648 and WO 9946272, the teachings of which are herein incorporated by reference in their entirety, especially concerning these inhibitors, their definition, uses and their production.

Suitable DP IV-inhibitors are those, disclosed e.g. in US 6,380,398, US 6,011,155; US 6,107,317; US 6,110,949; US 6,124,305; US 6,172,081; WO 95/15309, WO 99/61431, WO 99/67278, WO 99/67279, DE 198 34 591, WO 97/40832, DE 196 16 486 C 2, WO 98/19998, WO 00/07617, WO 99/38501, WO 99/46272, WO 99/38501, WO 01/68603, WO 01/40180, WO 01/81337, WO 01/81304, WO 01/55105, WO 02/02560 and WO 02/14271, WO 02/04610, WO 02/051836, WO 02/068420, WO 02/076450; WO 02/083128, WO 02/38541, WO 03/000180, WO 03/000181, WO 03/000250, WO 03/002530, WO 03/002531, WO 03/002553,WO 03/002593, WO 03/004496, WO 03/004498, WO 03/024965, WO 03/024942, WO 03/035067, WO 03/037327, WO 03/035057, WO 03/045977, WO 03/055881, WO 03/68748, WO 03/68757, WO 03/057666, WO 03057144, WO 03/040174, WO 03/033524 and WO 03/074500.

Further suitable DP IV-inhibitors include valine pyrrolidide (Novo Nordisk), NVP-DPP728A (1-[ [ [ 2-[ {5-cyanopyridin-2-yl}amino]ethyl]amino]acetyl]-2-cyano-(S)-pyrrolidine) (Novartis) as disclosed by Hughes et al., Biochemistry, 38 (36), 11597-11603, 1999, LAF-237 (1-[(3-hydroxy-adamant-1-ylamino)-acetyl]-pyrrolidine-2(S)-carbonitrile); disclosed by Hughes et al., Meeting of the American Diabetes Association 2002, Abstract no. 272 or (Novartis), TSL-225 (tryptophyl-1,2,3,4-tetrahydroisoquinoline-3-carboxylic acid), disclosed by Yamada et. al., Bioorg. & Med. Chem. Lett. 8 (1998), 1537-1540, 2-cyanopyrrolidides and 4-cyanopyrrolidides as disclosed by Asworth et al., Bioorg. & Med. Chem. Lett., 6, No. 22, pp 1163-1166 and 2745-2748 (1996), FE-999011 ( [(2S)-1-([2'S]-2'-amino-3',3'dimethyl-butanoyl)-pyrrolidine-2-carbonitrile] ), disclosed by Sudre et al., Diabetes 51 (5), pp 1461-1469 (2002) (Ferring), GW-229A (GlaxoSmithKline), disclosed by Randhawa SA, et al, ACS Meeting 2003, 226th:New York (MEDI 91) and the compounds disclosed in WO 01/34594 (Guilford), employing dosages as set out in the above references.

For the avoidance of doubt, the examples disclosed in each of the above mentioned publications are specifically incorporated herein by reference in their entirety, as individually disclosed compounds, especially concerning their structure, their definition, uses and their production.

### Definitions

The term "DP IV-inhibitor" or "dipeptidyl peptidase IV inhibitor" is generally known to a person skilled in the art and means enzyme inhibitors, which inhibit the catalytical activity of DP IV or DP IV-like enzymes.

"DP IV-activity" is defined as the catalytical activity of dipeptidyl peptidase IV (DP IV) and DP IV-like enzymes. These enzymes are post-proline (to a lesser extent post-alanine, post-serine or post-glycine) cleaving serine proteases found in various tissues of the body of a mammal including kidney, liver, and intestine, where they remove dipeptides from the N-terminus of biologically active peptides with a high specificity when proline or alanine form the residues that are adjacent to the N-terminal amino acid in their sequence.

The term "PEP-inhibitor" or "prolyl endopeptidase inhibitor" is generally known to a person skilled in the art and means enzyme inhibitors, which inhibit the catalytical activity of prolyl endopeptidase (PEP).

The term "QC" as used herein comprises glutaminyl cyclase (QC) and QC-like enzymes. QC and QC-like enzymes have identical or similar enzymatic activity, further defined as QC activity. In this regard, QC-like enzymes can fundamentally differ in their molecular structure from QC.

The term "QC activity" as used herein is defined as intramolecular cyclization of N-terminal glutamine residues into pyroglutamic acid (pGlu*) or of N-terminal L-homoglutamine or L-β-homoglutamine to a cyclic pyro-homoglutamine derivative under liberation of ammonia. See therefore schemes 1 and 2.

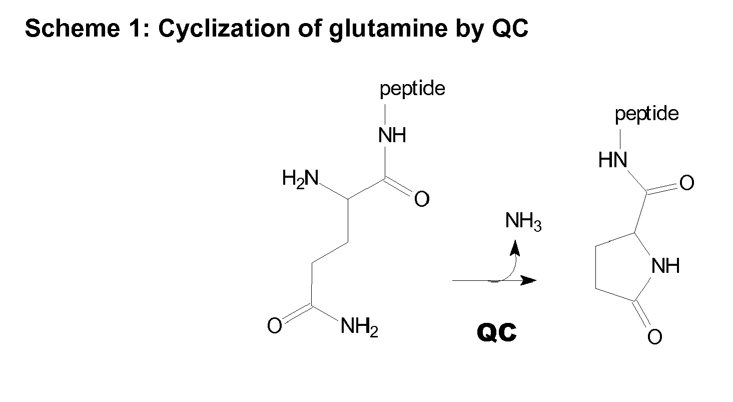

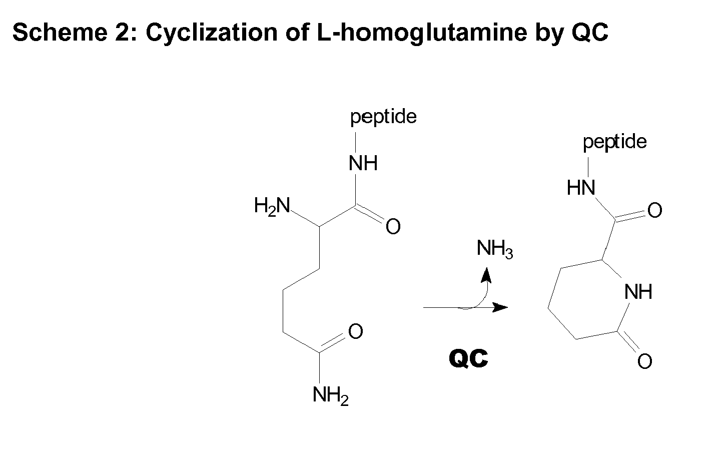

The term "EC" as used herein comprises the side activity of QC and QC-like enzymes as glutamate cyclase (EC), further defined as EC activity.

The term "EC activity" as used herein is defined as intramolecular cyclization of N-terminal glutamate residues into pyroglutamic acid (pGlu*) by QC. See therefore scheme 3.

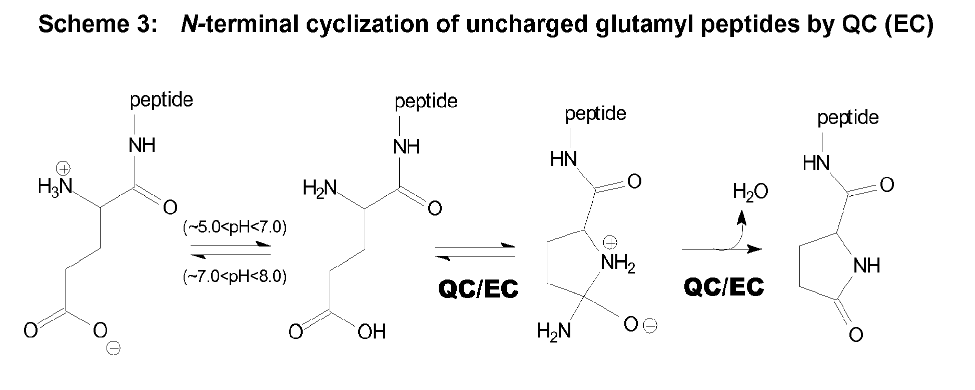

The term "QC-inhibitor" "glutaminyl cyclase inhibitor" is generally known to a person skilled in the art and means enzyme inhibitors, which inhibit the catalytical activity of glutaminyl cyclase (QC) or its glutamyl cyclase (EC) activity.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human being sought by a researcher, veterinarian, medical doctor or other clinician, which includes alleviation of the symptoms of the disease or disorder being treated.

As used herein, the term "pharmaceutically acceptable" embraces both human and veterinary use: for example the term "pharmaceutically acceptable" embraces a veterinarily acceptable compound or a compound acceptable in human medicine a health care.

Throughout the description and the claims the expression "acyl" can denote a C₁₋₂₀ acyl residue, preferably a C₁₋₈ acyl residue and especially preferred a C₁₋₄ acyl residue; "cycloalkyl" can denote a C₃₋₁₂ cycloalkyl residue, preferably a C₄, C₅ or C₆ cycloalkyl residue; and "carbocyclic" can denote a C₃₋₁₂ carbocyclic residue, preferably a C₄, C₅ or C₆ carbocyclic residue. "Heteroaryl" is defined as an aryl residue, wherein 1 to 4, and more preferably 1, 2 or 3 ring atoms are replaced by heteroatoms like N, S or O. "Heterocyclic" is defined as a cycloalkyl residue, wherein 1, 2 or 3 ring atoms are replaced by heteroatoms like N, S or O. "Peptides" are selected from dipeptides to decapeptides, preferred are dipeptides, tripeptides, tetrapeptides and pentapeptides. The amino acids for the formation of the "peptides" can be selected from those listed above.

Throughout the description and the claims the expression "alkyl" can denote a C₁₋₅₀ alkyl group, preferably a C₆₋₃₀ alkyl group, especially a C₈₋₁₂ alkyl group; for example, an alkyl group may be a methyl, ethyl, propyl, isopropyl or butyl group. The expression "alk", for example in the expression "alkoxy", and the expression "alkan", for example in the expression "alkanoyl", are defined as for "alkyl"; aromatic compounds are preferably substituted or optionally unsubstituted phenyl, benzyl, naphthyl, biphenyl or anthracene groups, which preferably have at least 8 C atoms; the expression "alkenyl" can denote a C₂₋₁₀ alkenyl group, preferably a C₂₋₆ alkenyl group, which has the double bond(s) at any desired location and may be substituted or unsubstituted; the expression "alkynyl" can denote a C₂₋₁₀ alkynyl group, preferably a C₂₋₆ alkynyl group, which has the triple bond(s) at any desired location and may be substituted or unsubstituted; the expression "substituted" or substituent can denote any desired substitution by one or more, preferably one or two, alkyl, alkenyl, alkynyl, mono- or multi-valent acyl, alkanoyl, alkoxyalkanoyl or alkoxyalkyl groups; the afore-mentioned substituents may in turn have one or more (but preferably zero) alkyl, alkenyl, alkynyl, mono- or multi-valent acyl, alkanoyl, alkoxyalkanoyl or alkoxyalkyl groups as side groups; organic amines, amides, alcohols or acids, each having from 8 to 50 C atoms, preferably from 10 to 20 C atoms, can have the formulae (alkyl)₂N- or alkyl-NH-, -CON(alkyl)₂ or -CO-NH(alkyl), -alkyl-OH or -alkyl-COOH.

Amino acids which can be used in the present invention are L and D-amino acids, N-methyl-amino acids, aza-amino acids; *allo*- and *threo*-forms of Ile and Thr, which can, e.g. be α-, β- or ω-amino acids, whereof α-amino acids are preferred.

### Examples of amino acids are:

aspartic acid (Asp), glutamic acid (Glu), arginine (Arg), lysine (Lys), histidine (His), glycine (Gly), serine (Ser), cysteine (Cys), threonine (Thr), asparagine (Asn), glutamine (Gln), tyrosine (Tyr), alanine (Ala), proline (Pro), valine (Val), isoleucine (Ile), leucine (Leu), methionine (Met), phenylalanine (Phe), tryptophan (Trp), hydroxyproline (Hyp), beta-alanine (beta-Ala), 2-aminooctanoic acid (Aoa), acetidine-(2)-carboxylic acid (Ace), pipecolic acid (Pip), 3-aminopropionic acid, 4-aminobutyric acid and so forth, alpha-aminoisobutyric acid (Aib), sarcosine (Sar), ornithine (Orn), citrulline (Cit), homoarginine (Har), t-butylalanine (t-butyl-Ala), t-butylglycine (t-butyl-Gly), N-methylisoleucine (N-Melle), phenylglycine (Phg), cyclohexylalanine (Cha), norleucine (Nle), cysteic acid (Cya) and methionine sulfoxide (MSO), acetyl-Lys, modified amino acids such as phosphoryl-serine (Ser(P)), benzyl-serine (Ser(Bzl)) and phosphoryl-tyrosine (Tyr(P)), 2-aminobutyric acid (Abu), aminoethylcysteine (AECys), carboxymethylcysteine (Cmc), dehydroalanine (Dha), dehydroamino-2-butyric acid (Dhb), carboxyglutaminic acid (Gla), homoserine (Hse), hydroxylysine (Hyl), cis-hydroxyproline (cisHyp), *trans*-hydroxyproline (transHyp), isovaline (Iva), pyroglutamic acid (Pyr), norvaline (Nva), 2-aminobenzoic acid (2-Abz), 3-aminobenzoic acid (3-Abz), 4- aminobenzoic acid (4-Abz), 4-(aminomethyl)benzoic acid (Amb), 4-(aminomethyl)cyclohexanecarboxylic acid (4-Amc), Penicillamine (Pen), 2-amino-4-cyanobutyric acid (Cba), cycloalkane-carboxylic aicds. Examples of ω̅-amino acids are e.g.: 5-Ara (aminoraleric acid), 6-Ahx (aminohexanoic acid), 8-Aoc (aminooctanoic aicd), 9-Anc (aminovanoic aicd), 10-Adc (aminodecanoic acid), 11-Aun (aminoundecanoic acid), 12-Ado (aminododecanoic acid). Further amino acids are: indanylglycine (Igl), indoline-2-carboxylic acid (Idc), octahydroindole-2-carboxylic acid (Oic), diaminopropionic acid (Dpr), diaminobutyric acid (Dbu), naphtylalanine (1-Nal) and (2-Nal), 4-aminophenylalanine (Phe(4-NH₂)), 4-benzoylphenylalanine (Bpa), diphenylalanine (Dip), 4-bromophenylalanine (Phe(4-Br)), 2-chlorophenylalanine (Phe(2-Cl)), 3-chlorophenylalanine (Phe(3-Cl)), 4-chlorophenylalanine (Phe(4-Cl)), 3,4-chlorophenylalanine (Phe (3,4-Cl₂)), 3-fluorophenylalanine (Phe(3-F)), 4-fluorophenylalanine (Phe(4-F)), 3,4-fluorophenylalanine (Phe(3,4-F₂)), pentafluorophenylalanine (Phe(F₅)), 4-guanidinophenylalanine (Phe(4-guanidino)), homophenylalanine (hPhe), 3-jodophenylalanine (Phe(3-J)), 4-jodophenylalanine (Phe(4-J)), 4-methylphenylalanine (Phe(4-Me)), 4-nitrophenylalanine (Phe-4-NO₂)), biphenylalanine (Bip), 4-phosphonomethylphenylalanine (Pmp), cyclohexylglycine (Ghg), 3-pyridinylalanine (3-Pal), 4-pyridinylalanine (4-Pal), 3,4-dehydroproline (A-Pro), 4-ketoproline (Pro(4-keto)), thioproline (Thz), isonipecotic acid (Inp), 1,2,3,4,-tetrahydroisoquinolin-3-carboxylic acid (Tic), propargylglycine (Pra), 6-hydroxynorleucine (NU(6-OH)), homotyrosine (hTyr), 3-jodotyrosine (Tyr(3-J)), 3,5-dijodotyrosine (Tyr(3,5-J₂)), methyltyrosine (Tyr(Me)), 2',6'-dimethyltyrosine (Dmt), 3-NO₂-tyrosine (Tyr(3-NO₂)), phosphotyrosine (Tyr(PO₃H₂)), alkylglycine, 1-aminoindane-1-carboxylic acid, 2-aminoindane-2-carboxylic acid (Aic), 4-amino-methylpyrrol-2-carboxylic acid (Py), 4-amino-pyrrolidine-2-carboxylic acid (Abpc), 2-aminotetraline-2-carboxylic acid (Atc), diaminoacetic acid (Gly(NH₂)), diaminobutyric acid (Dab), 1,3-dihydro-2H-isoinole-carboxylic acid (Disc), homocylcohexylalanine (hCha), homophenylalanine (hPhe or Hof), *trans*-3-phenyl-azetidine-2-carboxylic acid, 4-phenyl-pyrrolidine-2-carboxylic acid, 5-phenyl-pyrrolidine-2-carboxylic acid, 3-pyridylalanine (3-Pya), 4-pyridylalanine (4-Pya), styrylalanine, tetrahydroisoquinoline-1-carboxylic acid (Tiq), 1,2,3,4-tetrahydronorharmane-3-carboxylic acid (Tpi), β-(2-thienryl)-alanine (Tha).

"Peptides" are selected from dipeptides to decapeptides, preferred are dipeptides, tripeptides, tetrapeptides and pentapeptides. The amino acids for the formation of the "peptides" can be selected from those listed above.

An "aza-amino acid" is defined as an amino acid where the chiral α-CH group is replaced by a nitrogen atom, whereas an "aza-peptide" is defined as a peptide, in which the chiral α-CH group of one or more amino acid residues in the peptide chain is replaced by a nitrogen atom.

Other amino acid substitutions for those encoded in the genetic code can also be included in peptide compounds within the scope of the invention and can be classified within this general scheme. Proteinogenic amino acids are defined as natural protein-derived α-amino acids. Non-proteinogenic amino acids are defined as all other amino acids, which are not building blocks of common natural proteins.

"Peptide mimetics" per se are known to a person skilled in the art. They are preferably defined as compounds which have a secondary structure like a peptide and optionally further structural characteristics; their mode of action is largely similar or identical to the mode of action of the native peptide; however, their activity (e.g. as an antagonist or inhibitor) can be modified as compared with the native peptide, especially vis à vis receptors or enzymes. Moreover, they can imitate the effect of the native peptide (agonist). Examples of peptide mimetics are scaffold mimetics, non-peptidic mimetics, peptoides, peptide nucleic acids, oligopyrrolinones, vinylogpeptides and oligocarbamates. For the definitions of these peptide mimetics see Lexikon der Chemie, Spektrum Akademischer Verlag Heidelberg, Berlin, 1999.

The aim for using these mimetic structures is increasing the activity, increasing the selectivity to decrease side effects, protect the compound against enzymatic degradation for prolongation of the effect.

### Stereoisomers:

All possible stereoisomers of the claimed compounds are included in the present invention.

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention.

### Preparation and isolation of stereoisomers:

Where the processes for the preparation of the compounds according to the invention give rise to a mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their components enantiomers by standard techniques, such as the formation of diastereomeric pairs by salt formation with an optically active acid, such as (-)-di-p-toluoyl-d-tartaric acid and/or (+)-di-p-toluoyl-l-tartaric acid followed by fractional crystallization and regeneration of the free base. The compounds may also resolved by formation of diastereomeric esters or amides, followed by chromatographic separation and removal of the chiral auxiliary. Alternatively, the compounds may be resolved using a chiral HPLC column.

### Pharmaceutically acceptable salts:

In view of the close relationship between the free compounds and the compounds in the form of their salts, whenever a compound is referred to in this context, a corresponding salt is also intended, provided such is possible or appropriate under the circumstances.

The pharmaceutically acceptable salt generally takes a form in which an amino acids basic side chain is protonated with an inorganic or organic acid. Representative organic or inorganic acids include hydrochloric, hydrobromic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toulenesulfonic, cyclohexanesulfamic, salicylic, saccharinic or trifluoroacetic acid. All pharmaceutically acceptable acid addition salt forms of the compounds of the present invention are intended to be embraced by the scope of this invention.

### Polymorph crystal forms:

Furthermore, some of the crystalline forms of the compounds may exist as polymorphs and as such are intended to be included in the present invention. In addition, some of the compounds may form solvates with water (i.e. hydrates) or common organic solvents, and such solvates are also intended to be encompassed within the scope of this invention. The compounds, including their salts, can also be obtained in the form of their hydrates, or include other solvents used for their crystallization.

### Prodrugs:

The present invention further includes within its scope prodrugs of the compounds of this invention. In general, such prodrugs will be functional derivatives of the compounds which are readily convertible in vivo into the desired therapeutically active compound. Thus, in these cases, the methods of treatment of the present invention, the term "administering" shall encompass the treatment of the various disorders described with prodrug versions of one or more of the claimed compounds, but which converts to the above specified compound in vivo after administration to the subject. Conventional procedures for the selection and preparation of suitable prodrug derivatives are described, for example, in "Design of Prodrugs", ed. H. Bundgaard, Elsevier, 1985 and the patent applications DE 198 28 113, DE 198 28 114, WO 99/67228 and WO 99/67279 which are fully incorporated herein by reference.

### Protective Groups:

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, fully incorporated herein by reference. The protecting groups may be removed at a convenient subsequent stage using methods known from the art.

As used herein, the term "composition" is intended to encompass a product comprising the claimed compounds in the therapeutically effective amounts, as well as any product which results, directly or indirectly, from combinations of the claimed compounds (evtl. zu Definitionen).

### Carriers and Additives for galenic formulations:

Thus, for liquid oral preparations, such as for example, suspensions, elixirs and solutions, suitable carriers and additives may advantageously include water, glycols, oils, alcohols, flavoring agents, preservatives, coloring agents and the like; for solid oral preparations such as, for example, powders, capsules, gelcaps and tablets, suitable carriers and additives include starches, sugars, diluents, granulating agents, lubricants, binders, disintegrating agents and the like.

Carriers, which can be added to the mixture, include necessary and inert pharmaceutical excipients, including, but not limited to, suitable binders, suspending agents, lubricants, flavorants, sweeteners, preservatives, coatings, disintegrating agents, dyes and coloring agents.

Soluble polymers as targetable drug carriers can include polyvinylpyrrolidone, pyran copolymer, polyhydroxypropylmethacrylamidephenol, polyhydroxyethylaspartamide-phenol, or polyethyleneoxidepolyllysine substituted with palmitoyl residue. Furthermore, the compounds of the present invention may be coupled to a class of biodegradable polymers useful in achieving controlled release of a drug, for example, polyactic acid, polyepsilon caprolactone, polyhydroxy butyeric acid, polyorthoesters, polyacetals, polydihydropyrans, polycyanoacrylates and cross-linked or amphipathic block copolymers of hydrogels.

Suitable binders include, without limitation, starch, gelatin, natural sugars such as glucose or betalactose, corn sweeteners, natural and synthetic gums such as acacia, tragacanth or sodium oleate, sodium stearate, magnesium stearate, sodium benzoate, sodium acetate, sodium chloride and the like.

Disintegrators include, without limitation, starch, methyl cellulose, agar, bentonite, xanthan gum and the like.

### Peptide Sequences

The peptides mentioned and used herein have the following sequences:
**Aβ(1-42), amyloid β-peptide(1-42):**
   Asp-Ala-Glu-Phe-Arg-His-Asp-Ser-Gly-Tyr-Glu-Val-His-His-Gln-Lys-Leu-Val-Phe-Phe-Ala-Glu-Asp-Val-Gly-Ser-Asn-Lys-Gly-Ala-Ile-Ile-Gly-Leu-Met-Val-Gly-Gly-Val-Val-Ile-Ala
**Aβ(1-40), amyloid β-peptide(1-40):**
   Asp-Ala-Glu-Phe-Arg-His-Asp-Ser-Gly-Tyr-Glu-Val-His-His-Gln-Lys-Leu-Val-Phe-Phe-Ala-Glu-Asp-Val-Gly-Ser-Asn-Lys-Gly-Ala-Ile-Ile-Gly-Leu-Met-Val-Gly-Gly-Val-Val
**Aβ(3-42), amyloid β-peptide(3-42):**
   Glu-Phe-Arg-His-Asp-Ser-Gly-Tyr-Glu-Val-His-His-Gln-Lys-Leu-Val-Phe-Phe-Ala-Glu-Asp-Val-Gly-Ser-Asn-Lys-Gly-Ala-Ile-Ile-Gly-Leu-Met-Val-Gly-Gly-Val-Val-Ile-Ala
**Aβ(3-40), amyloid β-peptide(3-40):**
   GLu-Phe-Arg-His-Asp-Ser-Gly-Tyr-Glu-Val-His-His-Gln-Lys-Leu-Val-Phe-Phe-Ala-Glu-Asp-Val-Gly-Ser-Asn-Lys-Gly-Ala-Ile-Ile-Gly-Leu-Met-Val-Gly-Gly-Val-Val
**Aβ(1-11), amyloid β-peptide(1-11)a:**
   Asp-Ala-Glu-Phe-Arg-His-Asp-Ser-Gly-Tyr-Glu-NH₂
**Aβ(3-11), amyloid β-peptide(3-11)a:**
   Glu-Phe-Arg-His-Asp-Ser-Gly-Tyr-Glu-NH₂
**Aβ(1-21), amyloid β-peptide(1-21)a:**
   Asp-Ala-Glu-Phe-Arg-His-Asp-Ser-Gly-Tyr-Glu-Val-His-His-Gln-Lys-Leu-Val-Phe-Phe-Ala-NH₂
**Aβ(3-21), amyloid β-peptide(3-21)a:**
   Glu-Phe-Arg-His-Asp-Ser-Gly-Tyr-Glu-Val-His-His-Gln-Lys-Leu-Val-Phe-Phe-Ala-NH₂
**Gln**³**-Aβ(3-40), Gln³-amyloid β-peptide(3-40):**
   Gln-Phe-Arg-His-Asp-Ser-Gly-Tyr-Glu-Val-His-His-Gln-Lys-Leu-Val-Phe-Phe-Ala-Glu-Asp-Val-Gly-Ser-Asn-Lys-Gly-Ala-ile-ile-Gly-Leu-Met-Val-Gly-Gly-Val-Val
**Gln³-Aβ(3-21)a, Gln³-amyloid β-peptide(3-21)a:**
   Gln-Phe-Arg-His-Asp-Ser-Gly-Tyr-Glu-Val-His-His-Gln-Lys-Leu-Val-Phe-Phe-Ala-NH₂
**Gln³-Aβ(1-11)a, Gln³-amyloid β-peptide(1-11)a:**
   Asp-Ala-Gln-Phe-Arg-His-Asp-Ser-Gly-Tyr-Glu-NH₂
**Gln³-Aβ(3-11)a, Gln³-amyloid β-peptide(3-11)a:**
   Gln-Phe-Arg-His-Asp-Ser-Gly-Tyr-Glu-NH₂

### Summary of the Invention

The present invention provides novel physiological substrates of QC in mammals, [Glu³] amyloid β-protein (3-40/42), [Gln³] amyloid β-protein (3-40/42), [Glu¹¹] amyloid β-protein (11-40/42), [Gln¹¹] amyloid β-protein (11-40/42), and [Gln⁵]-substance P(5-11) and the use of effectors of QC and pharmaceutical compositions comprising effectors of QC for the treatment of conditions that can be treated by modulation of QC activity.

Unexpectedly, it was shown that recombinant human QC as well as QC-activity from brain extracts catalyze both, the N-terminal glutaminyl as well as glutamate cyclization. Most striking is the finding, that cyclase-catalyzed Glu¹-conversion is favored around pH 6.0 while Gin¹-conversion to pGlu-derivatives occurs with a pH-optimum of around 8.0. Since the formation of pGlu-Aβ-related peptides can be suppressed by inhibition of recombinant human QC and QC-activity from pig pituitary extracts, the enzyme QC (and its EC activity) is a target in drug development for treatment of Alzheimer's disease.

By administering effectors of QC (EC) activity to a mammal it can be possible to prevent or alleviate or treat neuronal disorders (Alzheimer's disease, Down Syndrome, Parkinson disease, Chorea Huntington, pathogenic psychotic conditions, schizophrenia, impaired food intake, sleep-wakefulness, impaired homeostatic regulation of energy metabolism, impaired autonomic function, impaired hormonal balance, impaired regulation, body fluids, hypertension, fever, sleep dysregulation, anorexia, anxiety related disorders including depression, seizures including epilepsy, drug withdrawal and alcoholism, neurodegenerative disorders including cognitive dysfunction and dementia).

In a preferred embodiment, the present invention provides the use of effectors of QC activity in combination with inhibitors of PEP for the treatment or alleviation of conditions that can be treated by modulation of QC- and/or PEP-activity.

In a further preferred embodiment, the present invention provides the use of effectors of QC activity in combination with inhibitors of DP IV or DP IV-like enzymes for the treatment or alleviation of conditions that can be treated by modulation of QC- and/or DP IV-activity.

Further preferred for the treatment of neuronal diseases is the use of at least one QC-effector in combination with NPY-receptor-ligands, NPY agonists and/or NPY antagonists.

Further preferred for the treatment of neuronal diseases is the use of at least one QC-effector in combination with at least one acetylcholinesterase (ACE) inhibitor.

The present invention provides pharmaceutical compositions for parenteral, enteral or oral administration, comprising at least one effector of QC optionally in combination with customary carriers and/or excipients; or comprising at least one effector of QC in combination with at least one PEP-inhibitor and/or at least one DP IV-inhibitor and/or at least one NPY-receptor-ligand, optionally in combination with customary carriers and/or excipients.

These combinations provide a particularly beneficial effect on behavioral conditions and such combinations are therefore shown to be effective and useful for the treatment of neuronal disorders (Alzheimer's disease, Down Syndrome, Parkinson disease, Chorea Huntington, pathogenic psychotic conditions, schizophrenia, impaired food intake, sleep-wakefulness, impaired homeostatic regulation of energy metabolism, impaired autonomic function, impaired hormonal balance, impaired regulation, body fluids, hypertension, fever, sleep dysregulation, anorexia, anxiety related disorders including depression, seizures including epilepsy, drug withdrawal and alcoholism, neurodegenerative disorders including cognitive dysfunction and dementia).

Accordingly, the invention provides a method for the treatment of of neuronal disorders (Alzheimer's disease, Down Syndrome, Parkinson disease, Chorea Huntington, pathogenic psychotic conditions, schizophrenia, impaired food intake, sleep-wakefulness, impaired homeostatic regulation of energy metabolism, impaired autonomic function, impaired hormonal balance, impaired regulation, body fluids, hypertension, fever, sleep dysregulation, anorexia, anxiety related disorders including depression, seizures including epilepsy, drug withdrawal and alcoholism, neurodegenerative disorders including cognitive dysfunction and dementia).

The method comprises either co-administration of a QC-inhibitor and/or at least one PEP-inhibitor and/or at least one DP IV-inhibitor and/or at least one NPY-receptor-ligand and/or at least one ACE-inhibitor or the sequential administration thereof.

Co-administration includes administration of a formulation which includes at least one QC-inhibitor and/or at least one PEP-inhibitor and/or at least one DP IV-inhibitor and/or at least one NPY-receptor-ligand and/or at least one ACE-inhibitor or the essentially simultaneous administration of separate formulations of each agent.

In another aspect the invention provides the use of at least one QC-inhibitor and/or at least one PEP-inhibitor and/or at least one DP IV-inhibitor and/or at least one NPY-receptor-ligand and/or at least one ACE-inhibitor for use in the manufacture of a composition for the treatment of neuronal disorders.

### Brief description of the drawings

Further understanding of these and other aspects of the present invention will be had by reference to the figures wherein:

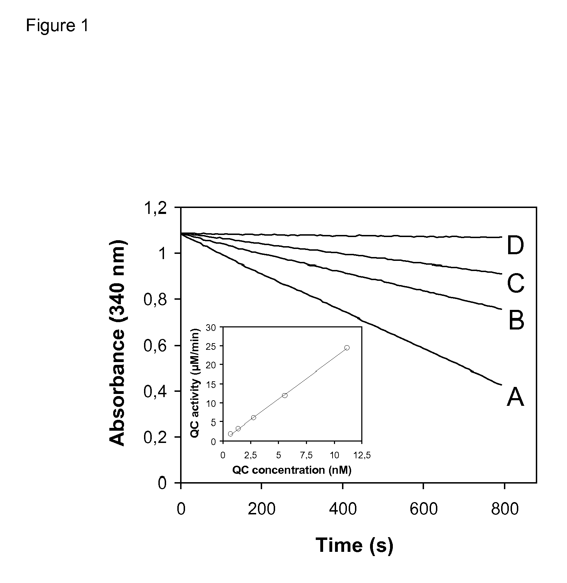

**Figure 1** shows progress curves of the cyclization of H-Gln-Ala-OH, catalyzed by human QC, monitoring the decrease in absorbance at 340 nm. The samples contained 0.3 mM NADH/H⁺, 14 mM α-Ketoglutaric acid, 30 U/ml glutamic dehydrogenase and 1 mM H-Gln-Ala-OH. From curve A-D, varying concentrations of QC were applied: A, 10 mU/ml, B, 5 mU/ml, C, 2.5 mU/ml. In case of curve D, QC was omitted. A linear relationship was obtained between the QC concentration and the observed activity (inset).

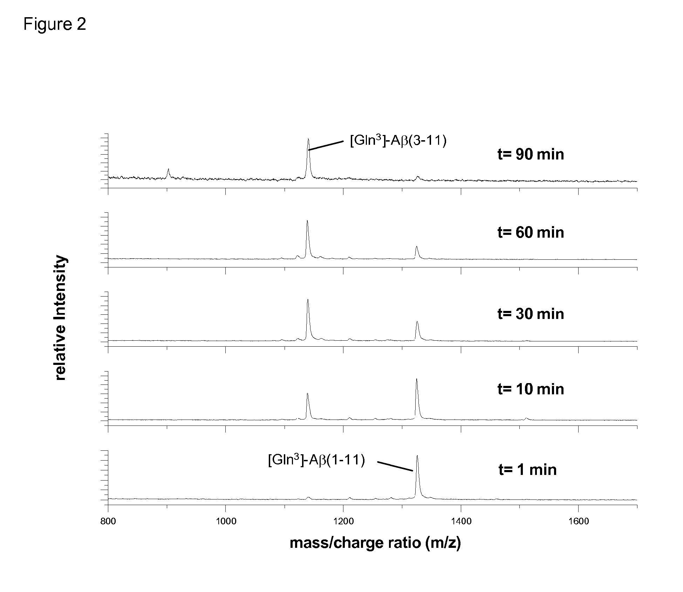

**Figure 2** shows the formation of Gln³-amyloid β-peptide(3-11) from Gln³-amyloid β-peptide(1-11) catalysed by DPIV. At the times indicated, samples were removed, from the assay tube, mixed with matrix solution (1:1 v/v) and subsequently the mass spectra recorded.

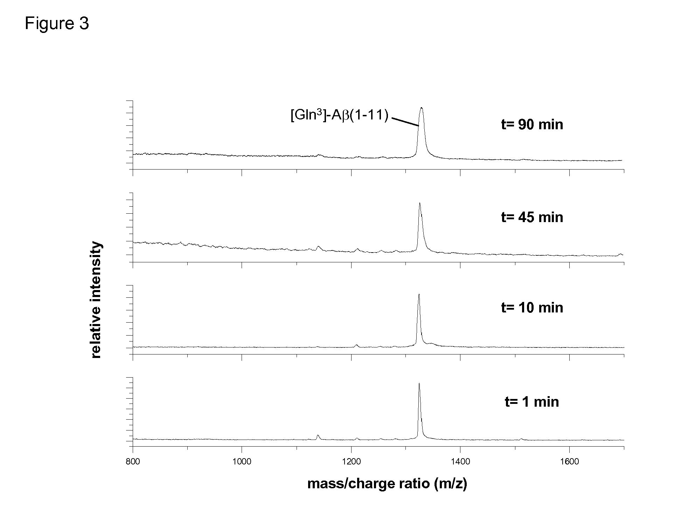

**Figure 3** shows the prevention of the cleavage of Gln³-amyloid β-peptide(1-11) by the DP IV-inhibitor Val-Pyrrolidide (Val-Pyrr). At the times indicated, samples were removed from the assay tube, mixed with matrix solution (1:1 v/v) and subsequently the mass spectra recorded.

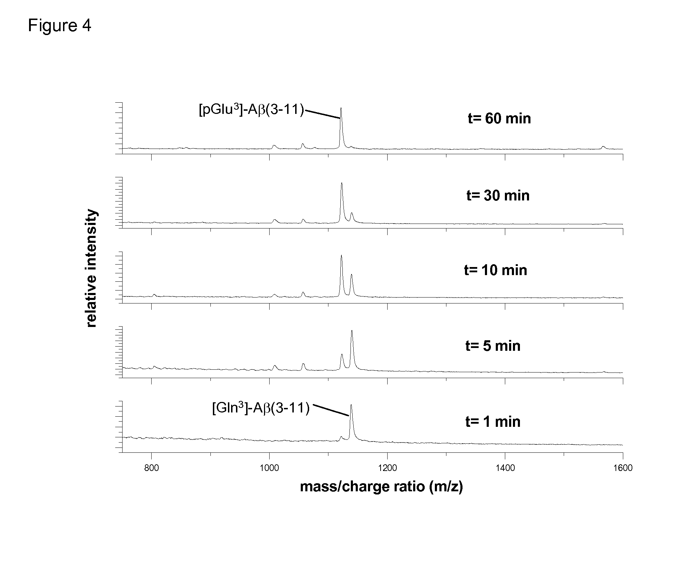

**Figure 4** shows the formation of pGlu³-amyloid β-peptide(3-11) from Gln³-amyloid β-peptide(3-11) catalysed by QC. At the times indicated, samples were removed from the assay tube, mixed with matrix solution (1:1 v/v) and subsequently the mass spectra recorded.

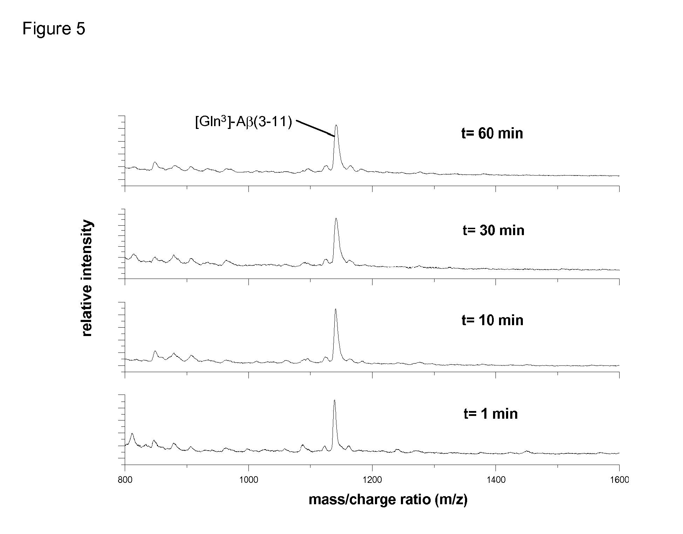

**Figure 5** shows the inhibition of the formation of pGlu³-amyloid β-peptide (3-11) from [Gln³]-amyloid β-peptide(3-11) by the QC-inhibitor 1,10-phenanthroline. At the times indicated from the assay tube, samples were removed, mixed with matrix solution (1:1 v/v) and subsequently the mass spectra recorded.

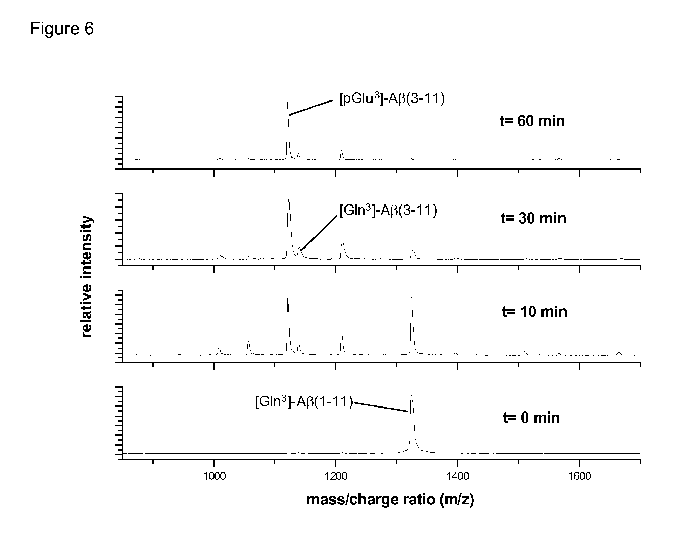

**Figure 6** shows the formation of pGlu³-amyloid β-peptide(3-11) from Gln³-amyloid β-peptide(1-11) after consecutive catalysis by DP IV and QC. At the times indicated, samples were removed from the assay tube, mixed with matrix solution (1:1 v/v) and subsequently the mass spectra recorded.

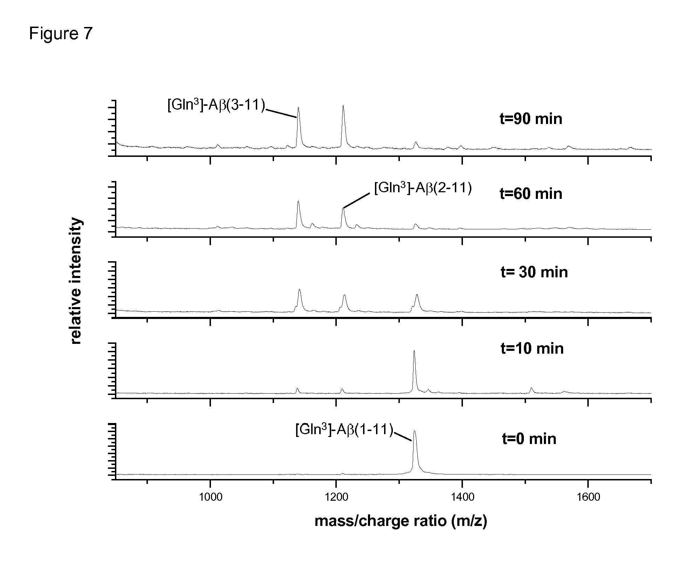

**Figure 7** shows the inhibition of pGlu³-amyloid β-peptide(3-11) formation from Gln³-amyloid β-peptide(1-11) by the QC-inhibitor 1,10-phenanthroline in the presence of catalytically active DP IV and QC. At the times indicated, samples were removed from the assay tube, mixed with matrix solution (1:1 v/v) and subsequently the mass spectra recorded.

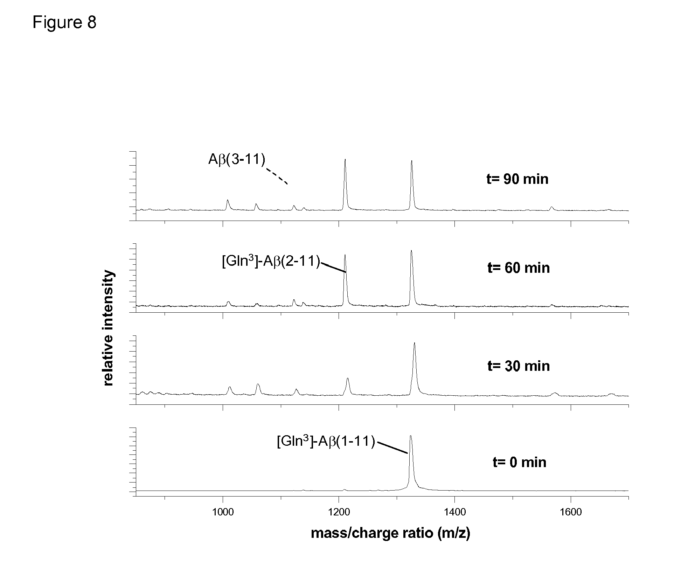

**Figure 8** shows the reduction of pGlu³-amyloid β-peptide(3-11) formation from Gln³-amyloid β-peptide(1-11) by the DP IV-inhibitor Val-Pyrr in the presence of catalytically active DP IV and QC. At the times indicated, samples were removed from the assay mixture, mixed with matrix solution (1:1 v/v) and subsequently the mass spectra recorded.

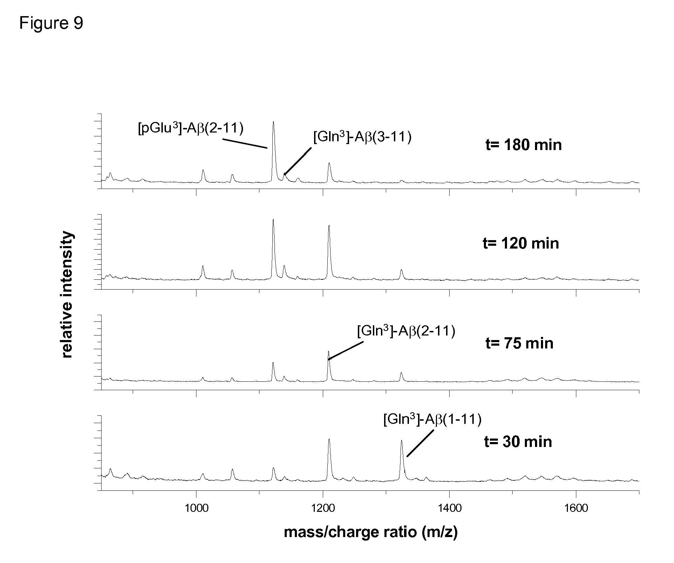

**Figure 9** shows the formation of pGlu³-amyloid β-peptide(3-11) from Gln³-amyloid β-peptide(1-11) after consecutive catalysis by aminopeptidase(s) and QC that are present in porcine pituitary homogenate. At the times indicated, samples were removed from the assay tube, mixed with matrix solution (1:1 v/v) and subsequently the mass spectra recorded.

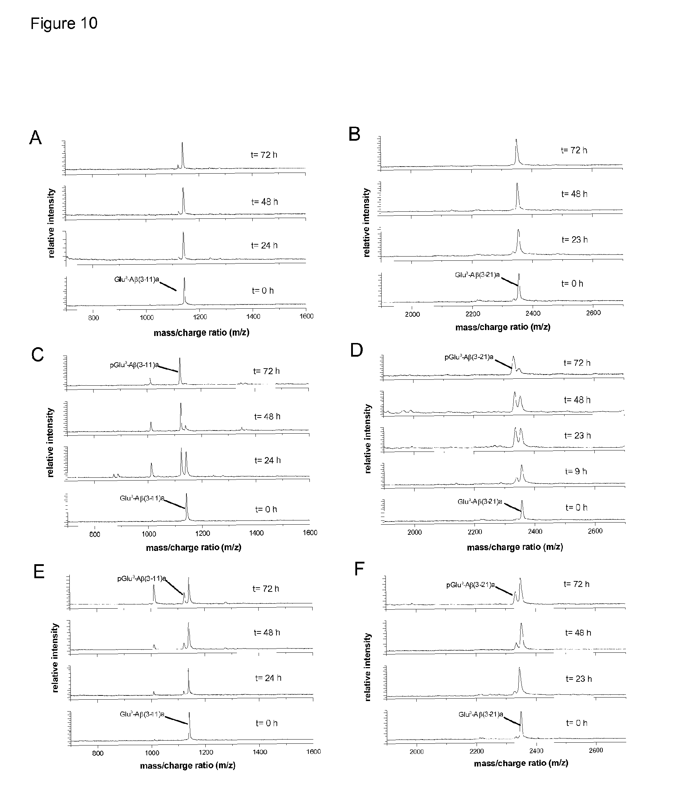

**Figure 10** **A** and **B** show Mass spectra of Glu³-Aβ(3-11)a and Glu³-Aβ(3-21)a incubated with recombinant human QC, that was boiled for 10 min before use. **C** and **D** show Mass spectra of Glu³-Aβ(3-11)a and Glu³-Aβ(3-21)a in presence of active human QC resulting in the formation of pGlu³-Aβ(3-11)a and pGlu³-Aβ(3-21)a, respectively. **E** and F show Mass spectra of Glu³-Aβ(3-11)a and Glu³-Aβ(3-21)a in presence of active QC and 5 mM Benzimidazole suppressing the formation of pGlu³-formation.

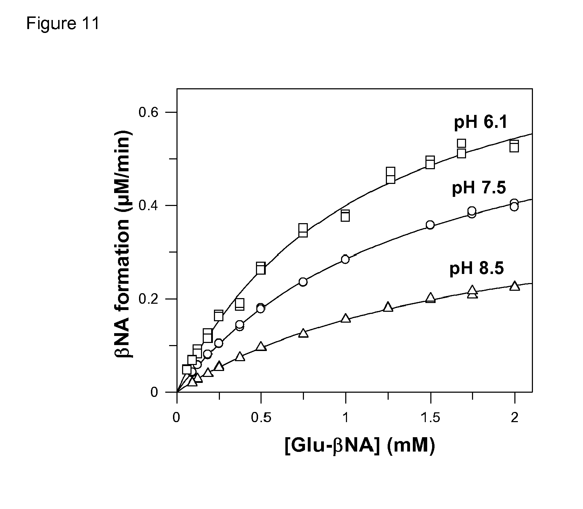

**Figure 11** shows reaction rates of papaya QC- catalyzed Glu-βNA-conversion plotted against the substrate concentration. The initial rates were measured in 0.1 M pyrophosphate buffer, pH 6.1 (squares), 0.1 M phosphate buffer, pH 7.5 (circles) and 0.1 M borate buffer, pH 8.5 (triangles). The kinetic parameters were as follows: K_{M}= 1.13 ±0.07 mM, k_{cat}= 1.13 ±0.04 min⁻¹ (pH 6.1); K_{M}= 1.45 ±0.03 mM, k_{cat}= 0.92 ±0.01 min⁻¹ (pH 7.5); K_{M}= 1.76 ±0.06 mM, k_{cat}= 0.56 ±0.01 min⁻¹ (pH 8.5).

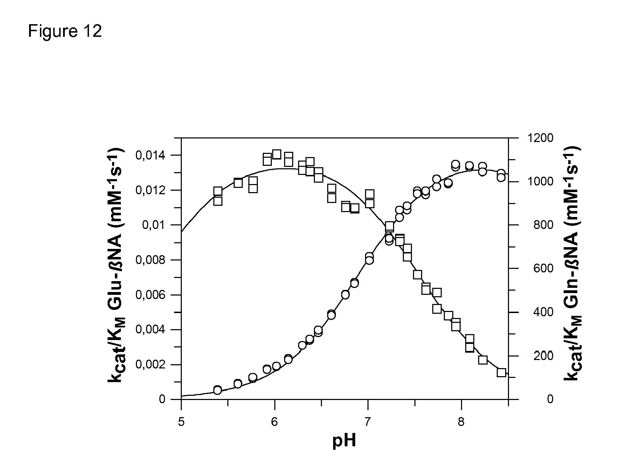

**Figure 12** shows the pH-dependence of the conversion of Gln-βNA (circles) and Glu-βNA (squares), determined under first-order rate-law conditions (S<<K_{M}). Substrate concentration was 0.01 mM and 0.25 mM, respectively. For both determinations, a three-component buffer system was applied consisting of 0.05 M acetic acid, 0.05 M pyrophosphoric acid and 0.05 M Tricine. All buffers were adjusted to equal conductivity by addition of NaCl, in order to avoid differences in ionic strength. The data were fitted to equations that account for two dissociating groups revealing pKₐ-values of 6.91 ±0.02 and 9.5 ±0.1 for Gln-βNA and 4.6 ±0.1 and 7.55 ±0.02 for Glu-βNA. The pKₐ-values of the respective substrate amino groups, determined by titration, were 6.97 ±0.01 (Gln-βNA) and 7.57 ±0.05 (Glu-βNA). All determinations were carried out at 30 °C.

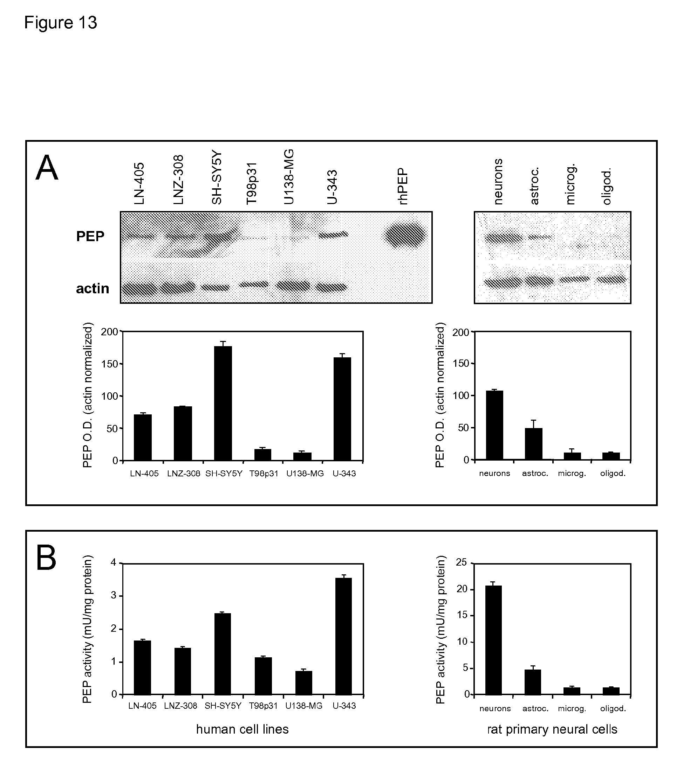

**Figure 13****: A)** Western blot analysis of PEP in cellular extracts of different cell lines normalized for actin content. PEP protein was detected by PEP-specific polyclonal antibody S449 (probiodrug, 1:400) using 10µg total protein/lane. The highest protein concentration for PEP was found in U-343 cells, followed by SH-SY5Y cells. All other cell types analysed displayed a significantly lower PEP content. In rat brain primary cultures, the highest PEP protein content was detected in neurons, followed by astrocytes, microglial cells and oligodendroglial cells.
   **B)** Quantification of PEP enzymatic activity in human cell lines and in rat primary neuronal and glial cells as indicated. PEP activity was highest in rat primary neurons, followed by astrocytes, microglia and oligodendroglial cells. Human neuroblastoma and glioma cell lines exhibited PEP activity in the range between the levels present in rat primary astrocytes and microglial cells.

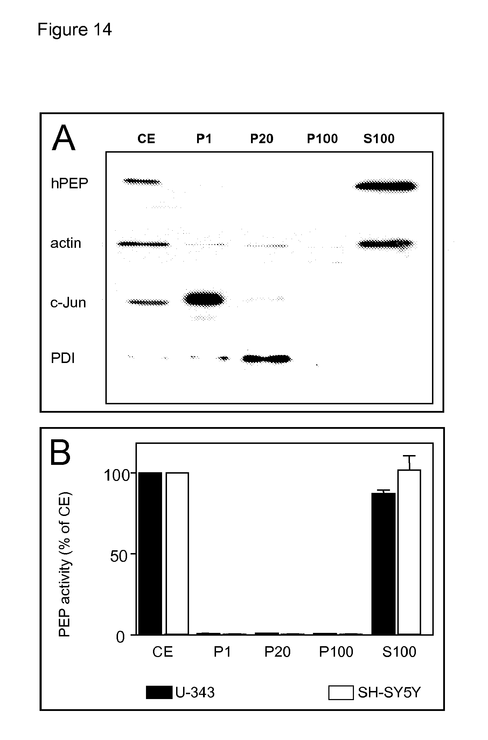

**Figure 14****: A)** Characterization of the endogenous subcellular PEP expression in the human glial cell line U-343. The quality of the separated cell fractions CE (crude extract), P1 (nucleus fraction), P20 (lysosomal fraction), P100 (microsomal fraction) and S100 (soluble cytosolic fraction) were validated by detection of different cell compartment specific proteins using antibodies against actin (1:1000, Sigma), c-fos (1:50, Oncogene) and protein-disulfidisomerase, PDI (1:100, Stressgen). PEP protein was detected only in the CE and in the S100 fraction using the polyclonal PEP-specific antibody S449 (1:400, probiodrug).
   **B)** Percentage of the specific PEP activity in separated cell fractions of the human glial and neuroblastoma cell line U-343 and SH-SY5Y, respectively. The separated cell fractions CE (crude extract), P1 (nucleus fraction), P20 (lysosomal fraction), P100 (microsomal fraction) and S100 (soluble cytosolic fraction) were screened for PEP activity as indicated. Nearly 100 % of the total specific PEP activity in the CE was detected in the S100 fraction of both cell lines investigated. Only small traces of PEP activity were measured in the particular fractions P20 and P100 as well in the nucleus fraction, P1.

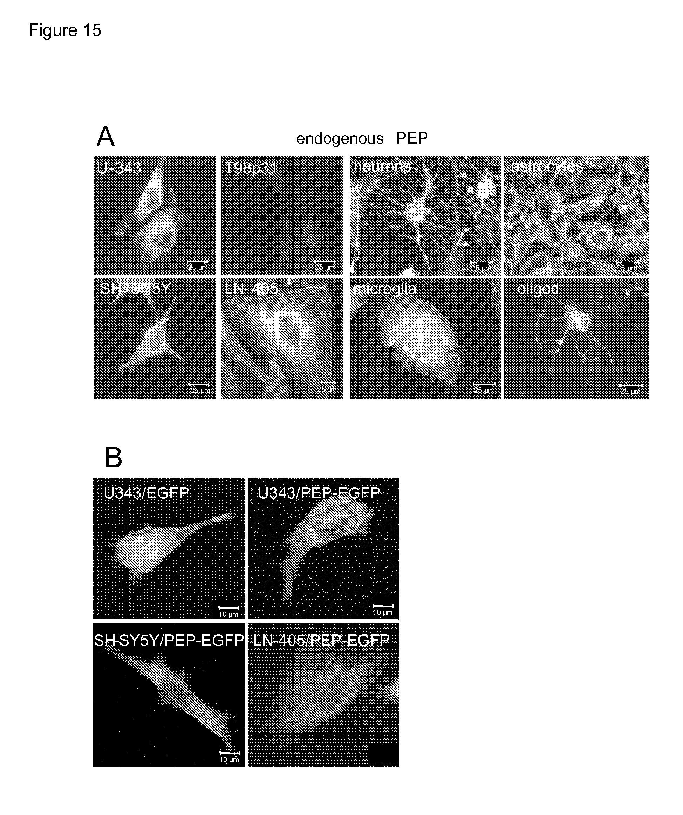

**Figure 15****: A)** Immunofluorescent labeling of PEP protein in human neuronal and glial cell lines as well in rat primary neuronal and glial cells. Different human cell lines and rat primary cells were labeled with the specific monoclonal PEP antibody 4D4D6 for confocal laser scanning microscopy (LSM510, Zeiss). In all investigated human cell lines and rat primary cells PEP protein was mainly found in the perinuclear space. In all LN-405 cells as well as in a significant number of SH-SY5Y and U-343 cells, a filamentous, cytoskeleton-like PEP distribution was observed.
   **B)** Distribution of PEP-EGFP fusion proteins in human cell lines. The human cell lines U-343, SH-SY5Y and LN-405 were transfected with the expression vector pEGFP (Clontech) and with PEP/EGFP fusion constructs pIS-7-MP7 using POLYFECTIN (Biontex) according to the manufacturer's instructions. After cultivation for 12 to 24 hours, cells were fixed with 4% (w/v) PFA in PBS and images were taken by laser scanning microscopy (LSM510, Zeiss, Oberkochen, Germany). As already observed for endogenous PEP distribution, PEP-EGFP fusion protein displayed both, a perinuclear and a filamentous cytoskeleton-like labeling in SH-SY5Y and LN-405 cells.

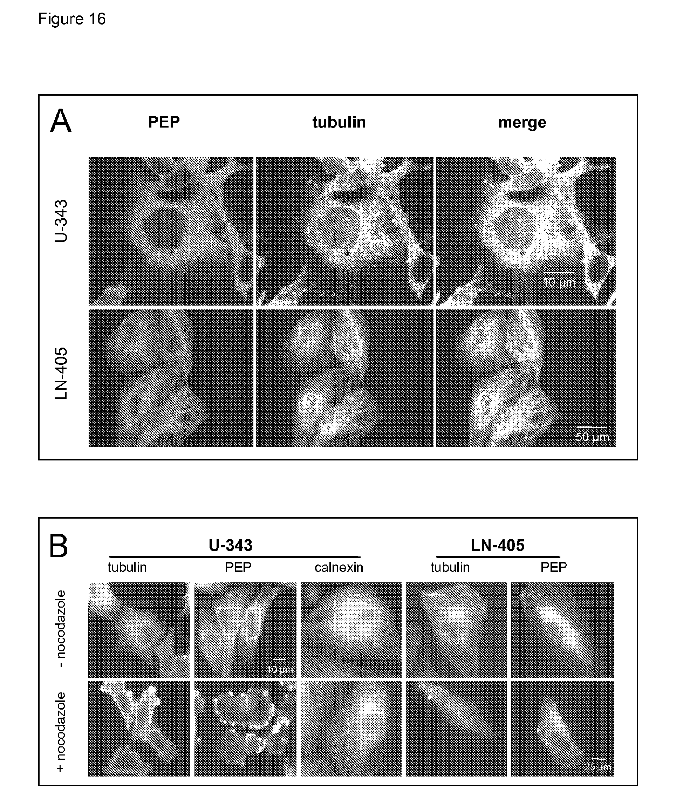

**Figure 16****: A)** Co-localization of PEP and tubulin in human glioma cell lines. U-343 and LN-405 cells were double-labeled with monoclonal tubulin (Sigma) and PEP antibodies (4D4D6) for confocal laser scanning microscopy (LSM510, Zeiss) as indicated. Yellow color (right row) indicates co-localization of tubulin and PEP immunofluorescence.
   **B)** Similar intracellular distribution of PEP and tubulin in native human glial cell lines U-343 and LN-405 (upper row). After depolymerisation of the microtubuli network by nocodazole treatment a complete loss of both, the tubulin and the PEP filamentous distribution pattern in U-343 and LN-405 cells observed (lower row).

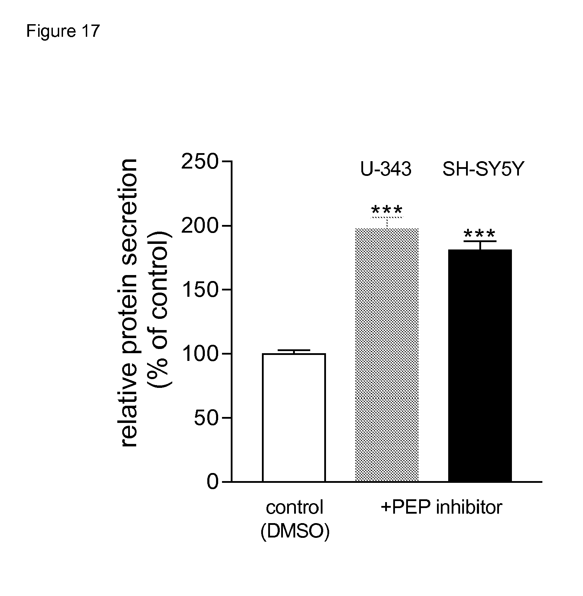

**Figure 17****:** Quantification of protein secretion by metabolic labeling experiments in U-343 and SH-SY5Y cells.
   Basal protein secretion from U-343 and SH-SY5Y cells was compared to protein secretion under conditions of inhibition of PEP enzymatic activity. The treatment of human U-343 and SH-SY5Y cells with PEP inhibitor over 24 hours resulted in a 2fold (197±27%) and 1,8fold (181±19%) higher protein content in the conditioned medium than in non-treated control cells, respectively Data are mean ± SEM and were tested for statistical significance by Analysis of variance (ANOVA) followed by two-tailed student's t-test. * Differences are statistically significant at P<0.05.

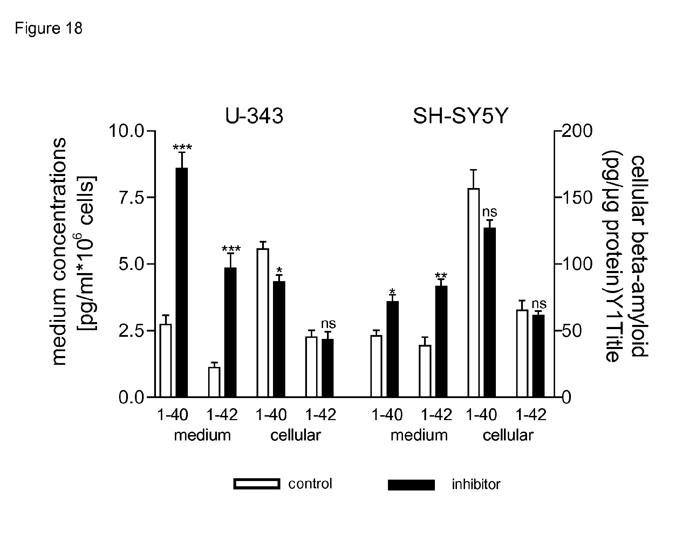

**Figure 18****:** Quantification of intracellular beta-amyloid concentrations in U-343 and SH-SY5Y cells and β-amyloid peptides secreted into the culture medium under conditions of PEP inhibition.
   Completely inhibition of PEP in human U-343 and SH-SY5Y cells resulted in an up to 4,3fold increase of beta-amyloid peptides in the conditioned medium. In both cell lines used, the intracellular amount of beta-amyloid 1-42 peptides were unaffected. In contrast, the amount of beta-amyloid 1-40 peptides were lowered at 20 % in PEP inhibitor treated U343 and SH-SY5Y cells. Due to the large variance in background levels, decrease in beta-amyloid 1-40 was not significant in SH-SY5Y cells
   Data are mean ± SEM from two independent experiments with samples run in triplicate and were tested for statistical significance by Analysis of variance (ANOVA) followed by two-tailed student's t-test. * Differences are statistically significant at P<0.05.

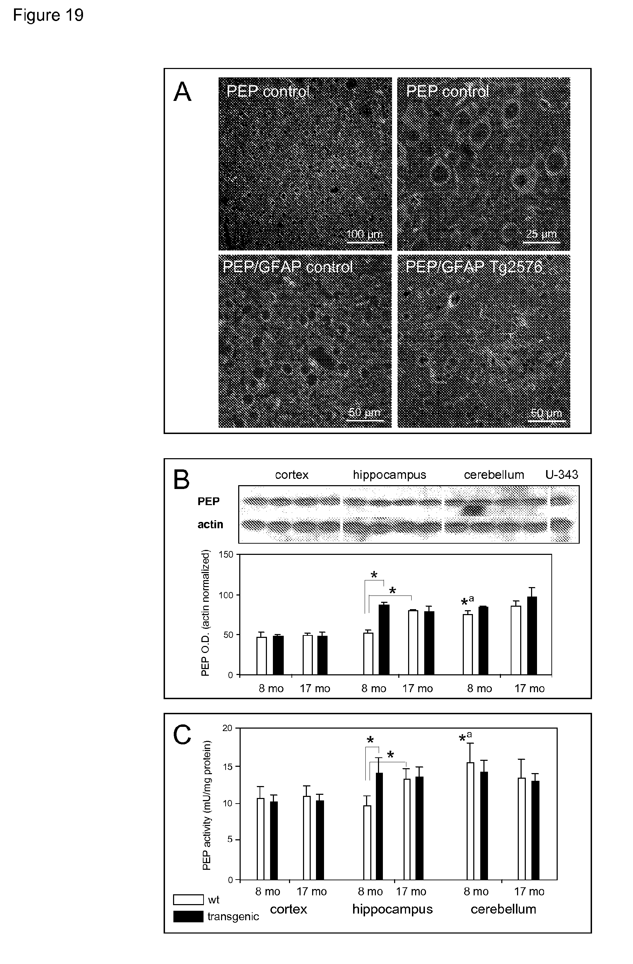

**Figure 19****: A)** In the upper row, the typical neuronal PEP immunofluorescent labeling of wild-type mouse brain is shown at low (left) and higher (right) magnification. The higher magnification image reveals the in the perinuclear and cytoskeletal localization of PEP in parietal cortex of wild-type mouse brain. In the bottom row, PEP (Cy2-labeled; green fluorescence) and GFAP (Cy3-labeled, red fluorescence) immunoreactivities are shown in parietal cortex of 17-months-old wild-type and age-matched APP transgenic Tg2576 mouse brain as indicated. Note the robust astrocytic activation in Tg2576 neocortex and the absence of PEP expression by these reactive astrocytes.
   **B)** Western blot analysis of PEP in brain homogenates from adult (8-months-old) and aged (17-months-old) wild-type and Tg2576 mice as indicated. This panel shows representative examples of Western blots and gives the quantification of optical density readings normalized for actin immunoreactivities. Data are mean ± SEM obtained from 7 animals per experimental group and were tested for statistical significance by ANOVA followed by two-tailed student's t-test.
      * Differences are statistically significant at P<0.05.
   **C)** Enzymatic activity of PEP in brain homogenates from adult (8-months-old) and aged (17-months-old) wild-type and Tg2576 mice as indicated. Data are mean ± SEM from 7 animals per experimental group and were tested for statistical significance by ANOVA followed by two-tailed student's t-test. * Differences as indicated are statistically significant at P<0.05. *^{a} PEP activity in cerebellum of 8-months old control mice is significantly higher than in parietal cortex and hippocampus of the same brains.

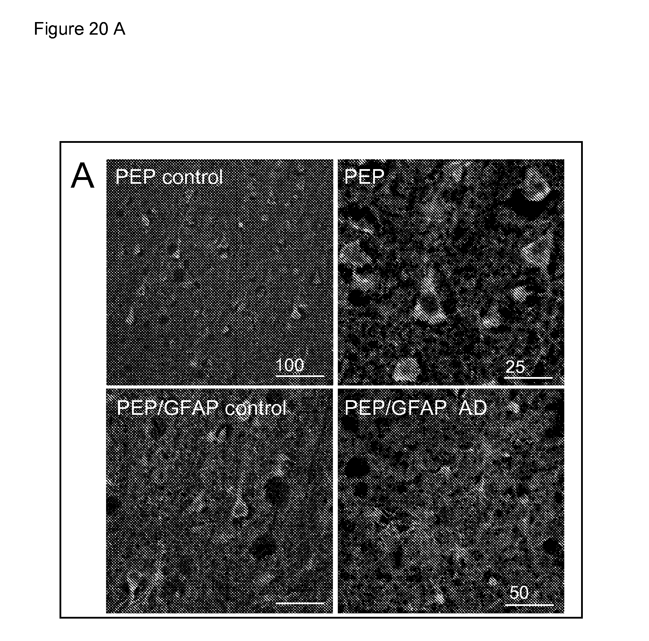

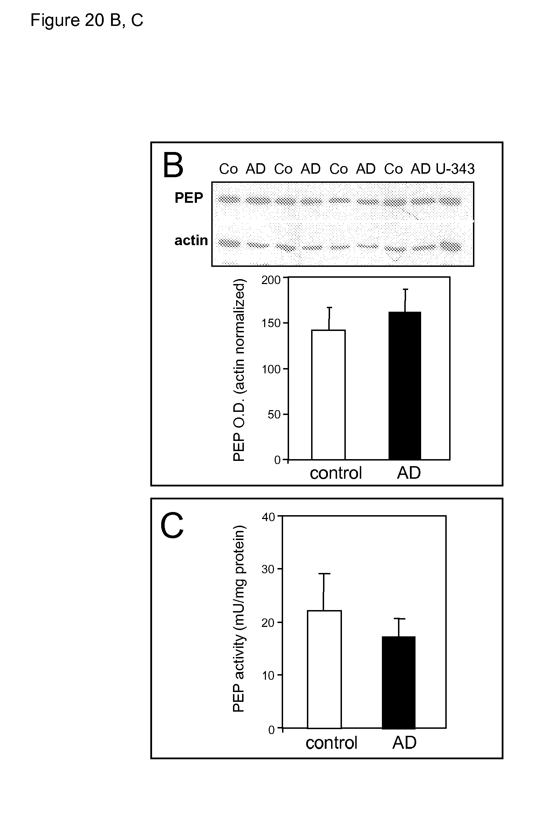

**Figure 20****: A)** PEP immunoreactivity in brain of a non-demented human control subject and in AD brain as indicated. PEP is neuronally expressed as shown at low magnification in parietal cortex (upper left). The higher magnification image (upper right) reveals the in the perinuclear and cytoskeletal localization of PEP in pyramidal neurons of parietal cortex in control brain. In bottom row, double immunofluorescent labelings for PEP (Cy2-labeled; green fluorescence) and GFAP (Cy3-labeled; red fluorescence) are shown for control (C) and AD (D) human parietal cortex. Note the intense PEP labeling in fewer neurons, which display shrunken morphology. PEP is not expressed by reactive astrocytes in AD brain.
   **B)** Western blot analysis of PEP in brain homogenates from non-demented human control subjects and AD patients as indicated. This panel shows representative examples of Western blots and gives the quantification of optical density readings normalized for actin content. Data are mean ± SEM from 7 AD patients and 8 control subjects and were tested for statistical significance by ANOVA followed by two-tailed student's t-test.
   **C)** Enzymatic activity of PEP in brain homogenates from control subjects and AD patients as indicated. Data are mean ± SEM from 7 AD patients and 8 control subjects and were tested for statistical significance by ANOVA followed by two-tailed student's t-test.
**Figure 21****:** Time response curves of a fluorescence quenched peptide substrate (RE(Edans)EVKMDAEFK(Dabcyl)Ra) mimicking the wild type (red squares) and the isoAsp containing (green circles) beta secretase cleavage site of APP incubated with a SY5Y cell extract.
**Figure 22****:** v-S-characteristic of the fluorescence quenched peptide substrate (RE(Edans)EVKMDAEFK(Dabcyl)Ra) mimicking the wild type (filled squares) and the isoAsp containing (open circles) beta secretase cleavage site of APP incubated with a SY5Y cell extract.

### Detailed Description of the Invention

The present invention provides new treatments of neuronal disorders, based on combinations of QC-inhibitors with at least one other compound selected from the group of PEP-inhibitors, DP IV-inhibitors, NPY-receptor ligands, NPY-agonists, NPY-antagonists and ACE inhibitors.

The present invention especially provides a new method for the treatment of Alzheimer's disease and Down Syndrome. The N-termini of amyloid β-peptides deposited in Alzheimer's disease and Down syndrome brain bear pyroglutamic acid. The pGlu formation is an important event in the development and progression of the disease, since the modified amyloid β-peptides show an enhanced tendency to β-amyloid aggregation and toxicity, likely worsening the onset and progression of the disease. (Russo, C. et al. 2002 J Neurochem 82,1480-1489).

In contrast, in the natural amyloid β-peptides (3-40/42), glutamic acid is present as an N-terminal amino acid. There was no enzymic conversion of Glu to pGlu known to date. Moreover, spontaneous cyclization of Glu-peptides to pGlu-peptides has not been observed as yet. Therefore one aspect of the present invention was to determine the role of QC in Alzheimer's disease and Down Syndrome. This aspect was addressed by the synthesis of amyloid β-peptide (3-11) and amyloid β-peptide (1-11), containing the amino acid glutamine instead of glutamic acid at position three, the determination of the substrate characteristics of these modified amyloid β-peptides against QC, DP IV and DP IV-like enzymes and aminopeptidases and the use of inhibitors of QC to prevent the formation of pGlu from a N-terminal glutaminyl residue of the amyloid β-derived peptides 1-11 and 3-11. The results are shown in example 8. The applied method is described in example 6.

To date, there are no hints indicating an involvement of QC in the progression of the disease, because glutamic acid is the N-terminal amino acid in amyloid β-peptide (3-40/42, or 11-40/42). But, QC is the only known enzyme capable of forming pGlu at the N-terminus of peptides. Other aspects of the present invention concern the following findings and discoveries:
a) In a side reaction, QC catalyzes the cyclization of glutamic acid to pyroglutamic acid at very low rates,
b) Glutamic acid of APP or its subsequently formed amyloid-β-peptides is converted into glutamine post-translationally by an unknown enzymatic activity and in a second step, QC catalyzes the cyclization of glutamine into pyroglutamic acid after processing of the amyloid-β-peptide N-terminus,
c) Glutamic acid is converted into glutamine post-translationally by a chemical catalysis or autocatalysis and subsequently, QC catalyzes the cyclization of glutamine to pyroglutamic acid after processing of the amyloid-β-peptide N-terminus,
d) There are mutations in the APP gene, which encode the amyloid-β-peptides, leading to Gln instead of Glu in position 3. After translation and processing of the N-terminus, QC catalyzes the cyclization of glutamine to pyroglutamic acid,
e) Glutamine is incorporated into the nascent peptide chain of APP, due to a malfunction of an unknown enzymatic activity and subsequently, QC catalyzes the cyclization of N-terminally glutamine to pyroglutamic acid after processing of the amyloid-β-peptide N-terminus.

QC is involved in the critical step in all five cases listed above, namely the formation of pyroglutamic acid that favors the aggregation of amyloid β-peptides. Thus, an inhibition of QC leads to a prevention of the precipitation of the plaque-forming amyloid-β-peptides 3-40/42 or amyloid-β-peptides 11-40/42, causing the onset and progression of Alzheimer's disease and Down Syndrome, independently of the mechanism by which cyclization occurs.

Glutamate is found in positions 3, 11 and 22 of the amyloid β-peptide. Among them the mutation from glutamic acid (E) to glutamine (Q) in position 22 (corresponding to amyloid precursor protein APP 693, Swissprot P05067) has been described as the so called Dutch type cerebroarterial amyloidosis mutation.

The β-amyloid peptides with a pyroglutamic acid residue in position 3, 11 and/or 22 have been described to be more cytotoxic and hydrophobic than the amyloid β-peptides 1-40(42/43) (Saido T.C. 2000 Medical Hypotheses 54(3): 427-429).

The multiple N-terminal variations can be generated by the β-secretase enzyme β-site amyloid precursor protein-cleaving enzyme (BACE) at different sites (Huse J.T. et al. 2002 J. Biol. Chem. 277 (18): 16278-16284), and/or by aminopeptidase processing. In all cases, cyclization can take place according to a)-e) as described above.

So far, there was no experimental evidence supporting the enzymatic conversion of Glu¹-peptides into pGlu¹-peptides by an unknown glutamyl cyclase (EC) corresponding to pathway a) (Garden, R. W., Moroz, T. P., Gleeson, J. M., Floyd, P. D., Li, L. J., Rubakhin, S. S., and Sweedler, J. V. (1999) J Neurochem 72, 676-681; Hosoda R. et al. (1998) J Neuropathol Exp Neurol. 57, 1089-1095). To date, no such enzyme activity has been identified, capable to cyclize Glu¹-peptides which are protonated N-terminally and possess a negatively charged Glu¹ γ-carboxylate moiety under mildly alkaline pH-conditions.

QC-activity against Gin¹-substrates is dramatically reduced below pH 7.0. In contrast, it appears that Glu¹-conversion can occur at acidic reaction conditions (Iwatsubo, T., Saido, T. C., Mann, D. M., Lee, V. M., and Trojanowski, J. Q. (1996) Am J Pathol 149, 1823-1830; Russo, C., Saido, T. C., DeBusk, L. M., Tabaton, M., Gambetti, P., and Teller, J. K. (1977) FEBS Lett 409, 411-416; Russo, C., Salis, S., Dolcini, V., Venezia, V., Song, X. H., Teller, J. K., and Schettini, G. (2001) Neurobiol Dis 8, 173-180; Tekirian, T. L., Saido, T. C., Markesbery, W. R., Russell, M. J., Wekstein, D. R., Patel, E., and Geddes, J. W. (1998) J Neuropathol Exp Neurol. 57, 76-94; Russo, C., Violani, E., Salis, S., Venezia, V., Dolcini, V., Damonte, G., Benatti, U., DArrigo, C., Patrone, E., Carlo, P., and Schettini, G. (2002) J Neurochem 82, 1480-1489; Hosoda, R., Saido, T. C., Otvos, L., Jr., Arai, T., Mann, D. M., Lee, V. M., Trojanowski, J. Q., and Iwatsubo, T. (1998) J Neuropathol Exp Neurol. 57, 1089-1095; Garden, R. W., Moroz, T. P., Gleeson, J. M., Floyd, P. D., Li, L. J., Rubakhin, S. S., and Sweedler, J. V. (1999) J Neurochem 72, 676-681).

According to the present invention, it was investigated whether QC is able to recognize and to turnover amyloid-β derived peptides under mild acidic conditions. Therefore, the peptides Gln³-Aβ(1-11)a, Aβ(3-11)a, Gln³-Aβ(3-11)a, Aβ(3-21)a, Gln³-Aβ(3-21)a and Gln³-Aβ(3-40) as potential substrates of the enzyme were synthesized and investigated. These sequences were chosen for mimicking natural N-terminally and C-terminally truncated Glu³-Aβ peptides and Gln³-Aβ peptides which could occur due to posttranslational Glu-amidation.

In the present invention it was shown that papaya and human QC catalyze both glutaminyl and glutamyl cyclization. Apparently, the primary physiological function of QC is to finish hormone maturation in endocrine cells by glutamine cyclization prior or during the hormone secretion process. Such secretory vesicles are known to be acidic in pH. Thus, a side activity of the enzyme in the narrow pH-range from 5.0 to 7.0 could be its newly discovered glutamyl cyclase activity (Scheme 3) transforming also Glu-Aβ peptides. However, due to the much slower occurring Glu-cyclization compared to Gln-conversion, it is questionable whether the glutamyl cyclization plays a significant physiological role. In the pathology of neurodegenerative disorders, however, the glutamyl cyclization is of relevance.

Investigating the pH-dependency of this enzymatic reaction, we found that the unprotonated N-terminus was essential for the cyclization of Gln¹-peptides and accordingly that the pKₐ-value of the substrate was identical to the pKₐ-value for QC-catalysis (see Figure 12). Thus, QC stabilizes the intramolecular nucleophilic attack of the unprotonated α-amino moiety at the γ-carbonyl carbon electrophilically activated by amidation (Scheme 1).

In contrast to the monovalent charge present on N-terminal glutamine containing peptides, the N-terminal Glu-residue in Glu-containing peptides is predominantly bivalently charged around neutral pH. Glutamate exhibits pKₐ-values of about 4.2 and 7.5 for the γ-carboxylic and for the α-amino moiety, respectively. I.e. at neutral pH and above, although the α-amino nitrogen is in part or fully unprotonated and nucleophilic, the γ-carboxylic group is unprotonated, and so exercising no electrophilic carbonyl activity. Hence, intramolecular cyclization is impossible.

However, in the pH-range of about 5.2-6.5, between their respective pKₐ-values, the two functional groups are present both in non-ionized forms, in concentrations of about 1-10% (-NH₂) or 10-1% (-COOH) of total N-terminal Glu-containing peptide. As a result, over a mildly acidic pH-range species of N-terminal Glu-peptides are present which carry both groups uncharged, and, therefore, it is possible that QC could stabilize the intermediate of intramolecular cyclization to pGlu-peptide. I.e. if the γ-carboxylic group is protonated, the carbonyl carbon is electrophilic enough to allow nucleophilic attack by the unprotonated α-amino group. At this pH the hydroxyl ion functions as a leaving group (Scheme 3). These assumptions are corroborated by the pH-dependence data obtained for the QC catalyzed conversion of Glu-βNA (see example 10). In contrast to glutamine conversion of Gln-βNA by QC, the pH-optimum of catalysis shifts to the acidic range around pH 6.0, i.e. the pH-range, in which substrate molecule species are simultaneously abundant carrying a protonated γ-carboxyl and unprotonated α-amino group. Furthermore, the kinetically determined pKₐ-value of 7.55 ±0.02 is in excellent agreement with that of the α-amino group of Glu-βNA, determined by titration (7.57 ±0.05).

Physiologically, at pH 6.0 the second-order rate constant (or specificity constant, k_{cat}/K_{M}) of the QC-catalyzed glutamate cyclization might be in the range of 8,000fold slower than the one for glutamine cyclization (Figure 11). However, the nonenzymatic turnover of both model substrates Glu-βNA and Gln-βNA is negligible, being conform with the observed negligible pGlu-peptide formation in the present invention. Hence, for the pGlu-formation by QC an acceleration of at least 10⁸ can be estimated from the ratio of the enzymatic versus non-enzymatic rate constants (comparing the second-order rate constants for the enzyme catalysis with the respective nonenzymatic cyclization first-order rate constants the catalytic proficiency factor is 10⁹-10¹⁰ M⁻¹ for the Gln- and the Glu-conversion, respectively). The conclusion from these data is, that *in vivo* only an enzymatic path resulting pGlu-formations seems conceivable.

Since QC is highly abundant in the brain and taking into account the high turnover rate of 0.9 min⁻¹ recently found for the maturation of 30 µM of (Gln-)TRH-like peptide (Prokai, L., Prokai-Tatrai, K., Ouyang, X., Kim, H. S., Wu, W. M., Zharikova, A., and Bodor, N. (1999) J Med Chem 42, 4563-4571), one can predict a cyclization half-life of about 100 hours for an appropriate glutamate-substrate, similar reaction conditions provided. Moreover, given compartmentalization and localization of brain QC/EC in the secretory pathway, the actual *in vivo* enzyme and substrate concentrations and reaction conditions might be even more favorable for the enzymatic cyclization in the intact cell. And, if N-terminal Glu is transformed to Gin a much more rapid pGlu-formation mediated by QC could be expected. *In vitro,* both reactions were suppressed by applying inhibitors of QC/EC-activity (Figures 4, 5 and 10).

In summary, the present invention shows that human QC/EC, which is highly abundant in the brain, is a likely catalyst to the formation of the amyloidogenic pGlu-Aβ peptides from Glu-Aβ and Gln-Aβ precursors which make up more than 50% of the plaque deposits found in Alzheimer's Disease. These findings identify QC/EC as a player in senile plaque formation and thus as a novel drug target in the treatment of Alzheimer's Disease.

In a second embodiment of the present invention, it was found that amyloid β- derived peptides are a substrate of dipeptidyl peptidase IV (DP IV) or DP IV-like enzymes. DP IV or DP IV-like enzymes release a dipeptide from the N-terminus of the modified amyloid β-peptide (1-11) generating amyloid β-peptide (3-11) with glutamine as the N-terminal amino acid residue. The results are shown in example 7.

In a third embodiment of the present invention, a combination of inhibitors of DP IV-activity and of inhibitors of QC can be used for the treatment of Alzheimer's disease and Down Syndrome.

The combined effect of DP IV and/or DP IV-like enzymes and of QC is illustrated as follows:
a) DP IV and/or DP IV-like enzymes cleave amyloid β-peptide (1-40/42), a dipeptide comprising H-Asp-Ala-OH and amyloid β- peptide (3-40/42) are released,
b) In a side reaction, QC catalyzes the cyclization of glutamic acid to pyroglutamic acid at very low rates,
c) Glutamic acid is converted into glutamine at the N-terminus post-translationally by an unknown enzymatic activity and subsequently, QC catalyzes the cyclization of glutamine into pyroglutamic acid after processing of the amyloid β-peptide N-terminus,
d) Glutamic acid is converted into glutamine post-translationally by a chemical catalysis or autocatalysis and in a second step, QC catalyzes the cyclization of glutamine into pyroglutamic acid after processing of the amyloid β-peptide N-terminus,
e) There are mutations in the APP gene, which encode the amyloid β-protein, leading to Gln instead of Glu in position 3 of Aβ, After translation and processing of the N-terminus, QC catalyzes the cyclization of glutamine to pyroglutamic acid,
f) Glutamine is incorporated into the nascent peptide chain of APP, due to a malfunction of an unknown enzymatic activity and subsequently, QC catalyzes the cyclization of N-terminally glutamine to pyroglutamic acid after processing of the amyloid β-peptide N-terminus,

The N-terminal Gln-exposure to QC-activity can be also triggered by different peptidase activities. Aminopeptidases can remove sequentially Asp and Ala from the N-terminus of amyloid β-peptides (1-40/41/43), thus unmasking amino acid three that is prone to cyclization. Dipeptidyl peptidases, such as DP I, DP II, DP IV, DP 8, DP 9 and DP 10, remove the dipeptide Asp-Ala in one step. Hence, inhibition of aminopeptidase- or dipeptidylpeptidase-activity is useful to prevent the formation of amyloid β-peptides (3-40/41/43).

The combined effect of inhibitors of DP IV and/or DP IV-like enzymes and of activity lowering effectors of QC is illustrated in the following way:
a) The inhibitors of DP IV and/or DP IV-like enzymes inhibit the conversion of amyloid β-peptide (1-40/42) to amyloid β-peptide (3-40/42).
b) An N-terminal exposure of glutamic acid is thereby prevented and no conversion to glutamine, either by enzymatic or by chemical catalysis, subsequently leading to pyroglutamic acid formation, is possible.
c) Inhibitors of QC prevent in addition the formation pyroglutamic acid from any residual modified amyloid β-peptide (3-40/42) molecules and those modified amyloid β-peptide (3-40/42) molecules, which are generated by mutations of the APP gene.

Prolyl endopeptidase (PEP) is believed to inactivate neuropeptides that are present in the extracellular space. However, the intracellular presence of PEP suggests additional, yet unidentified physiological functions for this enzyme.

The present invention comprises the following unexpected findings:
1) PEP is localized to the perinuclear space and to the cytoskeleton of rat primary neuronal and glial cells and human cell lines indicating novel functions for PEP in axonal transport and/or protein secretion.
2) In metabolic labeling experiments performed in U-343 and SH-SY5Y cells an increased global protein secretion under conditions of PEP inhibition occurs.
3) Among the proteins more abundantly secreted were β-amyloid peptides, which were accumulated in the culture medium.
4) In mouse brain PEP was exclusively expressed by neurons and displayed region- and age-specific differences in expression levels.
5) In brains of amyloid precursor protein transgenic Tg2576 mice, hippocampal PEP activity increased in the pre-plaque phase but not in aged mice with β-amyloid plaque pathology.
6) PEP expression was not detected in activated glial cells surrounding β-amyloid plaques in brains from Tg2576 mice and Alzheimer's disease patients.

The observations according to the present invention indicate that the reported neuroprotective and cognition-enhancing effects of PEP inhibition might be due to increased protein secretion - including β-amyloid peptides - and are most likely supported by a rise in intracellular IP₃ concentrations.

A further aspect of the present invention considers inhibitors of acetylcholinesterase (ACE). ACE inhibitors were shown to increase basal and K⁺-stimulated brain pyrrolidone carboxyl peptidase activity in a dose dependent manner. Because of that, these drugs are able to ameliorate Alzheimer type dementia (ATD) cognitive deficits acting not only facilitating cholinergic transmission but also avoiding the formation of pyroglutamyl-ended amyloid-b-peptides deposition trough the activation of brain pyrrolidone carboxyl peptidase (Ramírez-Expósito et al. (2001), European Neuropsychopharmcology 11, 381-383). A preferred ACE-inhibitor is SDZ ENA 713 (rivastigmine (+)-(*S*)-*N*-ethyl-3-[(1-dimethylamino)ethyl]-*N*-methylphenylcarbamate hydrogen tartrate.

Summarizing the facts mentioned above, the enzymes QC, PEP, DP IV/DP IV-like enzymes and pyrrolidone carboxyl peptidase are involved in impaired neuronal conditions and are therefore targets for drug development. The specific effect of inhibitors of theses enzymes is shown in table 1.

**Table 1: Effect of inhibitors of QC, PEP and DP IV/DP IV-like enzymes in neuronal diseases**

| **Disease** | **Target-Enzyme** | **Drug Type** | **Effect** |
|---|---|---|---|
| Alzheimer/Down Syndrome, Parkinson, Chorea Huntington | QC | Inhibitor | Suppression of amyloid β-peptide formation (suppression of N-terminal pGlu formation) |
| Alzheimer/Down Syndrome, Parkinson, Chorea Huntington | Pyrrolidone carboxyl peptidase | Inhibitors of ACE | Suppression of amyloid β-peptide formation (suppression of N-terminal pGlu formation) |
| Dementia, Alzheimer/Down Syndrome | PEP | Inhibitor | Increase of amyloid β-peptide (1-40/42) secretion |
| Anxiety, Depression | DP IV/DP IV-like enzymes | Inhibitor | Augmentation of active NPY (see WO 02/34243 and WO 02/34242) |

More than 50% of all plaque peptides found in Alzheimer, Down Syndrome, Parkinson and Chorea Huntington patients start with pGlu. Such N-terminal pGlu renders the peptides degradation resistent and triggers plaque formation starting with intracellular deposition of, e.g. pGlu-Aβ 3-40(42/43), pGlu-Aβ 11-40(42/43) and pGlu-Aβ 2-40(42/43) in neuronal cells in the CNS. The formation and intracellular deposition of these pGlu-containing peptides can efficiently prevented or decreased by either
1) Inhibition of QC, thereby inhibiting the cyclization of N-terminal glutamine or glutamic acid residues of amyloid β-peptides;
2) Inhibition of PEP, thereby increasing the amyloid β-peptide (1-40/42/43) secretion into the extracellular space and thereby preventing QC to act subsequently on on the N-terminally truncated amyloid β-peptide (3-40/42/43) and amyloid β-peptide (11-40/42/43); or Administration of ACE inhibitors, thereby inhibiting the formation of pyroglutamyl-ended amyloid β-peptides; or
4) Simultaneous inhibition of both enzymes, QC and PEP, thereby combining the effects described in 1) and 2).In scheme 4, the respective target points for therapeutic intervention to prevent intracellular pE-Aβ3/11-42 formation and accumulation ar indicated with the numbers (1) for QC/EC inhbition, (2) for PEP inhibition and (3) for ACE inhibitor administration.

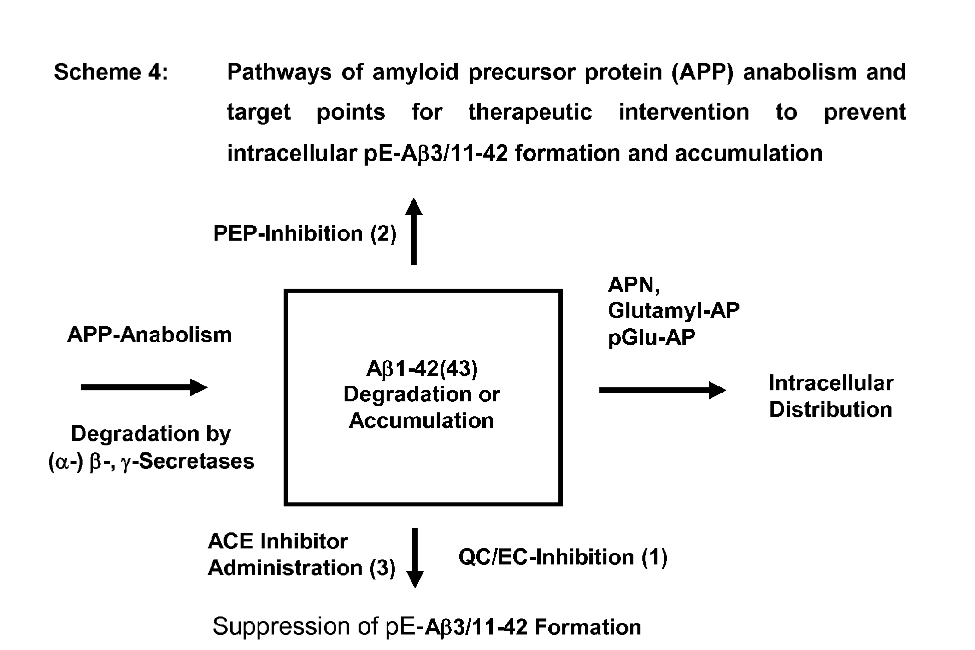

### Increase of Aβ1-42 Secretion

Further enzymes which are involved in the amyloid precursor protein (APP) anabolism are described as follows.

The type I transmembrane APP is the origin of the plaque forming β-amyloid peptides which contribute to the pathogenesis of Alzheimer's disease. The APP undergoes different processing pathways. The normal cleavage by the alpha-secretase occurs within the β-amyloid peptide sequence and results in soluble and non-toxic fragments. On the other hand the APP is also hydrolysed by a subsequent action of beta- and gamma-secretases which releases the highly amyloidogenic beta-A4 1-40 or 1-42 peptides.

In 1999 and 2000 two aspartic proteases BACE1 and BACE2 (Memapsin-2 and -1) were identified which are capable to cleave APP at the beta-secretase site (Vassar R. et al. 1999 Science 286 (5440):735-741, Acquati F. et al. 2000 FEBS Lett 468 (1):59-64). Especially when the so called Swedish mutation (K670M671 → NL) is present APP is 50fold better substrate for BACE1 (Grueninger-Leitch F. et al. 2002 J Biol Chem 277 (7):4687-4693). Furthermore also cysteine proteases are in discussion as potential candidates for beta site cleavage (Hook V. Y. et al. 2002 J Neurochem. 81 (2):237-256). After release of β-amyloid peptide (1-40/42) the peptide can be attacked by aminopeptidases or dipeptidyl aminopeptidases resulting in the formation of β-amyloid peptide (3-40/42) with an N-terminal glutamyl residue.

As demonstrated above, the N-terminal glutamyl residue of β-amyloid peptide (3-40/42) is accepted by glutaminyl cyclase which catalyzes its cyclization producing an N-terminal pyroglutamate residue. This pGlu³-β-amyloid peptide (3-40/42) is characterized by an increased proteolytic stability to aminopeptidases and by an enhancement of its amyloidogenic properties.

Spontaneous formation of an iso-aspartyl (isoAsp) or D-aspartyl (D-Asp) residues from intra-protein asparaginyl (Asn) or aspartyl (Asp) residues, which is a common process during aging of proteins can take place at position 672 of the APP, which corresponds to position 1 of the β-amyloid peptides. β-amyloid peptides containing an N-terminal isoAsp were indeed determined in the plaques of Alzheimer patients (Shimizu T. et al. 2000 Arch Biochem Biophys 381 (2):225-234).

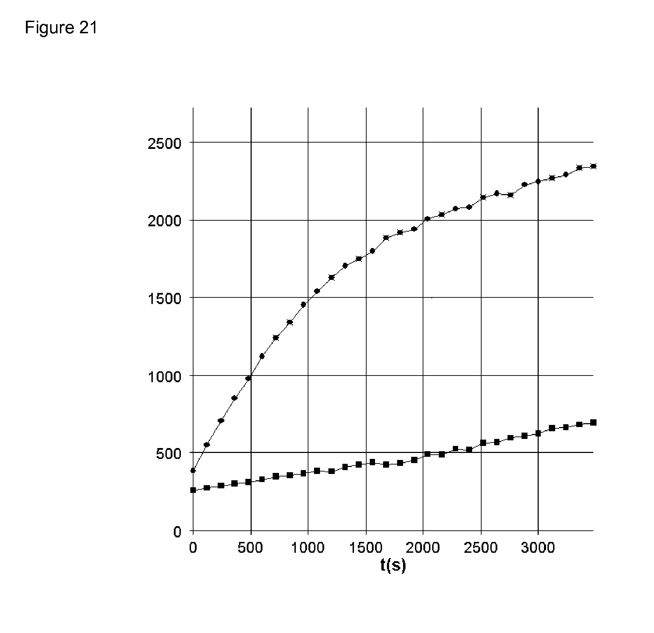

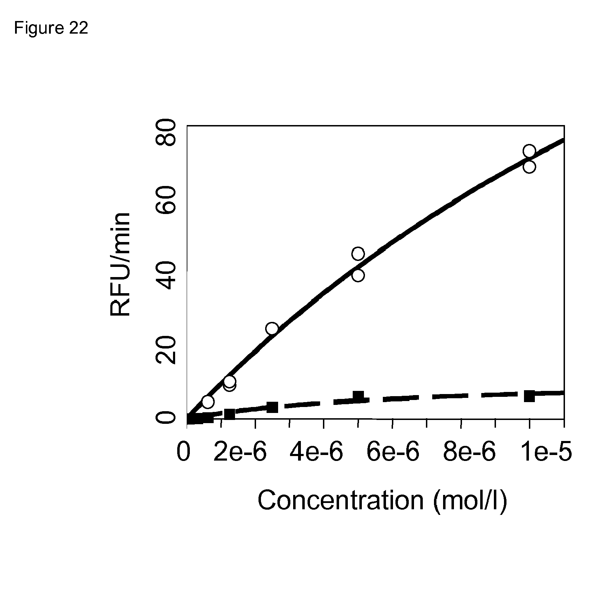

As a further preferred embodiment of the present invention, there is first experimental evidence, that substrates with an isoAsp residue at this position are much more sensitive to beta secretase like cleavage than the corresponding aspartyl containing peptides (Figures 21 and 22).

Protein isoaspartate carboxymethyl transferase (PIMT) is an enzyme capable to repair the spontaneous formed isoAsp or D-Asp residues inside of an polypeptide chain by methylation of this non-natural amino acids. This methylation results in the rapid formation of a succinimide intermediate which converts by chance either to Asp or isoAsp (Clarke S. 2003 Ageing Res Rev 2 (3):263-285). Repeated action of PIMT finally leads to a complete repair of the IsoAsp containing peptide chain back to the Asp containing peptides (Harigaya Y., T. C. et al. 2000 Biochem Biophys.Res Commun 276 (2):422-427, Russo C. et al. 2002 J Neurochem 82 (6):1480-1489). See scheme 5 for the mechanism of action catalyzed by PIMT.

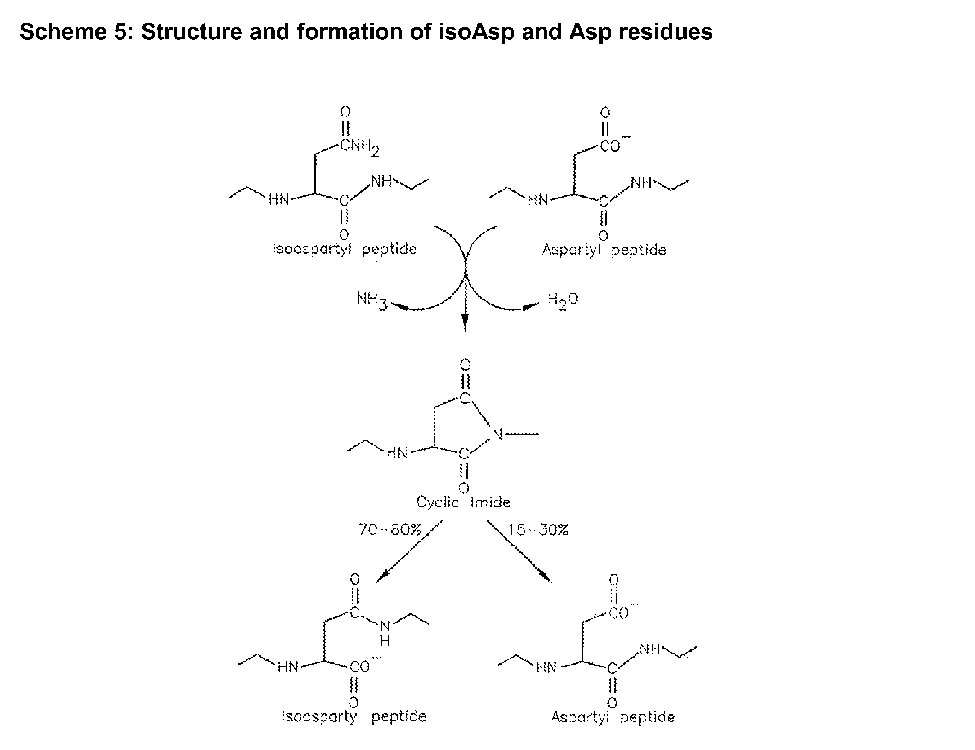

In summary all combinations of compounds preventing a single step in the cascade of pGlu³-β-amyloid peptide (3-40/42) formation are useful for treatment of Alzheimers disease. Such combinations include, e.g.
1. inhibitors of QC activity to prevent the formation of the N-terminal pGlu from β-amyloid peptides (3-40/42),
2. PIMT enhancers to repair the aged APP,
3. inhibitors of beta-secretases including but not restricted to BACE1, BACE2 and cysteine proteases, and inhibitors of gamma-secretase to prevent formation of β-amyloid peptides from APP,
4. inhibitors of aminopeptidases and inhibitors of dipeptidyl aminopeptidases including but not restricted to dipeptiyl peptidase II dipeptiyl peptidase IV to prevent formation of β-amyloid peptides (3-40/42), and
5. enhancers of activity of neutral endopeptidase which was found to be capable to cleave soluble β-amyloid peptides.

A schematic representation of these combinations is given in scheme 6. The APP is cleaved by beta and/or gamma secretase to β-amyloid peptides (1-40/42). The beta secretase cleavage may be enhanced by formation of isoAsp672. β-amyloid peptides (1-40/42) are hydrolysed by, e.g. aminopeptidases (AP) or dipeptidyl peptidases (DP) to β-amyloid peptides (3-40/42) containing an N-terminal Glu residue which can further be processed by glutaminyl cyclase resulting in the formation of the amyloidogenic pGlu³-β-amyloid peptides (3-40/42). The x in scheme 6 stands for 40/42.

According to the present invention, combinations comprising 2 to 5 compounds selected from 1. to 5. described above are preferred. More preferred combinations comprise 2 to 5 compounds selected from 1. to 5. described above. Most preferred are combinations comprising 2 compounds selected from 1. to 5. above.

Especially preferred are combinations comprising at least one QC inhibitor and at least 1 to 5 compounds selected from 2. to 5. described above. Most preferred are combinations comprising at least one QC inhibitor and at least one PIMT enhancer or combinations comprising at least one QC inhibitor and at least one beta secretase inhibitor or combinations comprising at least one QC inhibitor and at least one gamma secretase inhibitor.

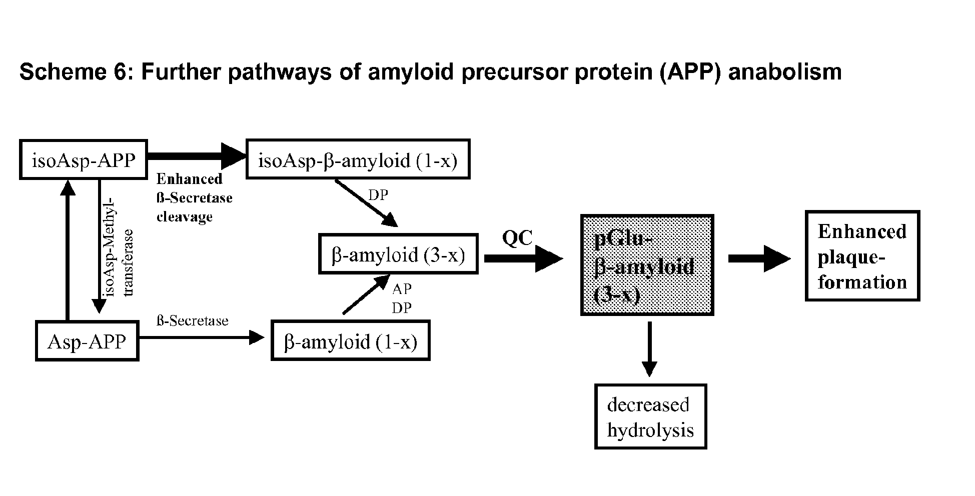

Suitable QC-inhibitors are those, e.g. having the general formula 1: 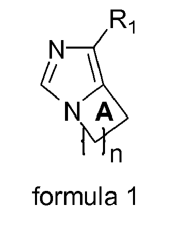 wherein n is 1, 2, 3 or 4, preferably 2 or 3, especially 2, and A can be any saturated or unsaturated heterocycle and may be substituted or unsubstituted, and wherein R₁ is H or a branched or unbranched alkyl chain, a branched or unbranched alkenyl chain, a branched or unbranched alkynyl chain, carbocyclica carbocycle, aryl, heteroaryl, heterocyclica heterocycle, aza-amino acid, amino acid or a mimetic thereof, aza-peptide, peptide or a mimetic thereof; all of the above residues R₁ optionally being substituted independently of each other.

Further suitable QC-inhibitors can be described generally by the formula 2 and the pharmaceutically acceptable salts thereof, including all stereoisomers: 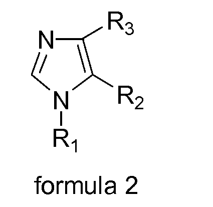 wherein R₁, R₂ and R₃ are independently H or a branched or unbranched alkyl chain, a branched or unbranched alkenyl chain, a branched or unbranched alkynyl chain, carbocyclica carbocycle, aryl, heteroaryl, heterocyclica heterocycle, aza-amino acid, amino acid or a mimetic thereof, aza-peptide, peptide or a mimetic thereof; all of the above residues R₁, R₂ and R₃ optionally being substituted independently of each other.

Furthermore, the present invention provides QC-inhibitors of the formula 3 and the pharmaceutically acceptable salts thereof, including all stereoisomers: 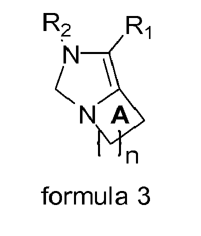 wherein n is 1, 2, 3 or 4, preferably 2 or 3, especially 2, and A can be any saturated or unsaturated heterocycle and may be substituted or unsubstituted, and wherein R₁ and R₂ are independently H or a branched or unbranched alkyl chain, a branched or unbranched alkenyl chain, a branched or unbranched alkynyl chain, carbocyclica carbocycle, aryl, heteroaryl, heterocyclica heterocycle, aza-amino acid, amino acid or a mimetic thereof, aza-peptide, peptide or a mimetic thereof; all of the above residues R₁ and R₂ optionally being substituted independently of each other.

Furthermore, the present invention provides QC-inhibitors which can be described generally by the formula 4 and the pharmaceutically acceptable salts thereof, including all stereoisomers: 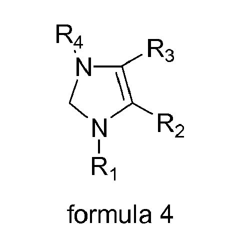 wherein R₁, R₂, R₃ and R₄ are independently H or a branched or unbranched alkyl chain, a branched or unbranched alkenyl chain, a branched or unbranched alkynyl chain, carbocyclic, aryl, heteroaryl, heterocyclic, aza-amino acid, amino acid or a mimetic thereof, aza-peptide, peptide or a mimetic thereof; all of the above residues optionally being substituted.

Furthermore, the present invention provides QC-inhibitors which can be described generally by the formula 5 and the pharmaceutically acceptable salts thereof, including all stereoisomers: 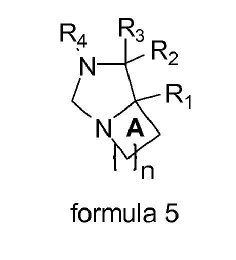 wherein n is 1, 2, 3 or 4, preferably 2 and 3, especially 2, and A can be any saturated or unsaturated heterocycle and may be substituted or unsubstituted, and wherein R₁, R₂, R₃ and R₄ are independently H or a branched or unbranched alkyl chain, a branched or unbranched alkenyl chain, a branched or unbranched alkynyl chain, carbocyclic, aryl, heteroaryl, heterocyclic, aza-amino acid, amino acid or a mimetic thereof, aza-peptide, peptide or a mimetic thereof; all of the above residues optionally being substituted.

Other suitable QC-inhibitors are compounds which can be described generally by the formula 6 and the pharmaceutically acceptable salts thereof, including all stereoisomers: 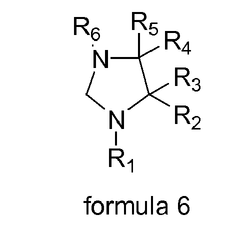 wherein R₁, R₂, R₃, R₄, R₅ and R₆ are independently H or a branched or unbranched alkyl chain, a branched or unbranched alkenyl chain, a branched or unbranched alkynyl chain, carbocyclic, aryl, heteroaryl, heterocyclic, aza-amino acid, amino acid or a mimetic thereof, aza-peptide, peptide or a mimetic thereof; all of the above residues optionally being substituted.

In addition, the present invention provides QC-inhibitors which can be described generally by the formula 7 and the pharmaceutically acceptable salts thereof, including all stereoisomers: 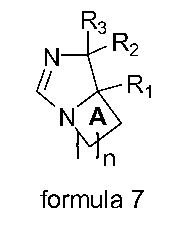 wherein n is 1, 2, 3 or 4, preferably 2 or 3, especially 2, and A can be any saturated or unsaturated heterocycle and may be substituted or unsubstituted, and wherein R₁, R₂ and R₃ are independently H or a branched or unbranched alkyl chain, a branched or unbranched alkenyl chain, a branched or unbranched alkynyl chain, carbocyclica carbocycle, aryl, heteroaryl, heterocyclica heterocycle, aza-amino acid, amino acid or a mimetic thereof, aza-peptide, peptide or a mimetic thereof; all of the above residues R₁, R₂ and R₃ optionally being substituted independently of each other.

Other QC-inhibitors according to the present invention are compounds which can be described generally by the formula 8 and the pharmaceutically acceptable salts thereof, including all stereoisomers: 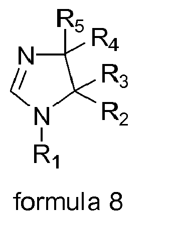 wherein R₁, R₂, R₃, R₄ and R₅ are independently H or a branched or unbranched alkyl chain, a branched or unbranched alkenyl chain, a branched or unbranched alkynyl chain, carbocyclica carbocycle, aryl, heteroaryl, heterocyclica heterocycle, aza-amino acid, amino acid or a mimetic thereof, aza-peptide, peptide or a mimetic thereof; all of the above residues R₁, R₂, R₃, R₄ and R₅ optionally being substituted independently of each other..

Furthermore, the present invention provides QC-inhibitors which can be described generally by the formula 9 or the pharmaceutically acceptable salts thereof, including all stereoisomers: 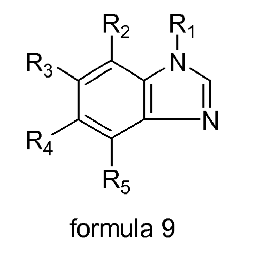 wherein R₁, R₂, R₃, R₄ and R₅ are independently H or a branched or unbranched alkyl chain, a branched or unbranched alkenyl chain, a branched or unbranched alkynyl chain, a carbocycle, aryl, heteroaryl, a heterocycle, aza-amino acid, amino acid or a mimetic thereof, aza-peptide, peptide or a mimetic thereof; all of the above residues R₁, R₂, R₃, R₄ and R₅ optionally being substituted independently of each other. Preferred QC-inhibitors relate to formula 10: 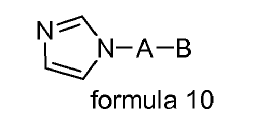
- wherein A is a branched or unbranched C₁-C₇ alkyl chain, a branched or unbranched C₁-C₇ alkenyl chain, a branched or unbranched C₁-C₇ alkynyl chain,
   or wherein A is a compound selected from the group consisting of : 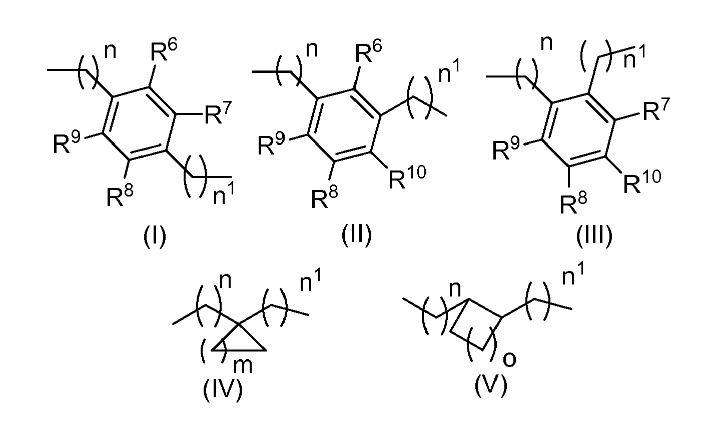
- wherein R⁶-R¹⁰ are independently H or a branched or unbranched alkyl chain, a branched or unbranched alkenyl chain, a branched or unbranched alkynyl chain, a carbocycle, aryl, heteroaryl, a heterocycle , preferably H or methyl,
- wherein n and n¹ are independently 1 - 5, m is 1 - 5 , o is 0 - 4,
   Preferably A is a C₃ alkyl chain, a C₃ methyl branched alkyl chain, cycloalkyl-1,1-dimethyl of formula (IV) with m = 1-4, 1,4-dimethylphenyl or 1,3-dimethylphenyl; and
- wherein B is a compound selected from the group consisting of 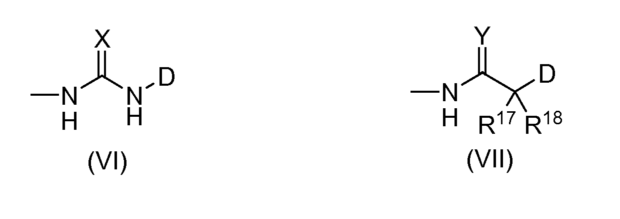 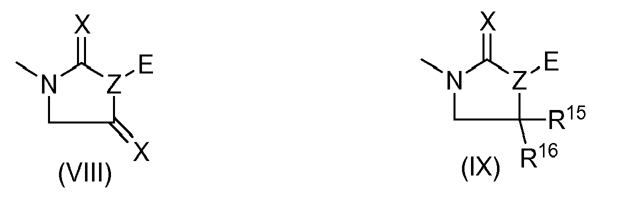 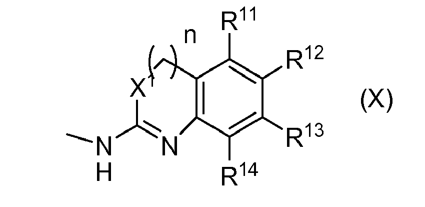 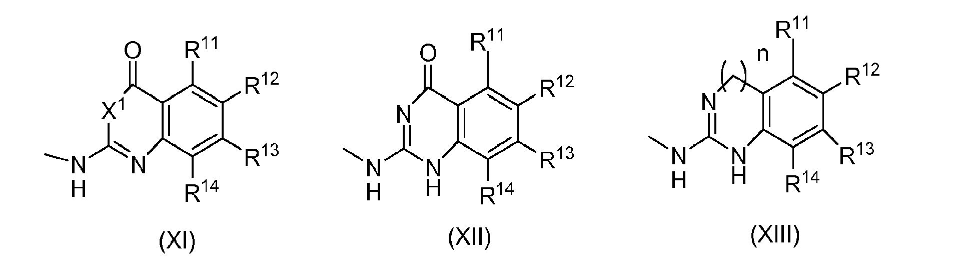 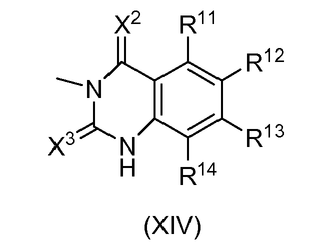
- wherein D and E are a branched or unbranched alkyl chain, a branched or unbranched alkenyl chain, a branched or unbranched alkynyl chain, a carbocycle, aryl, heteroaryl, a heterocycle,

Preferably D and E are a substituted phenyl, wherein substitution means oxyalkyl, thioalkyl, halogenyl, or carboxylic acid alkyl ester or aryl ester.

Further preferred are compounds, wherein D and E are a dihydrobenzodioxine, a benzodioxole, a benzodithiole, a dihydrobenzodithiine, a benzooxathiole, a dihydrobenzooxathiine.
- wherein Z is CH or N.

In a preferred embodiment, Z is N.
- wherein X can be O, S, N-CN, with the proviso for formulas (VIII) and (IX) that, if Z = CH, X is O or S,
- wherein X¹, X² and X³ are independently O or S,

In a preferred embodiment, X is S.
- wherein Y is O or S,
- wherein Z is CH or N.

In a preferred embodiment, Z is N.
- wherein R¹¹-R¹⁴ can beare independently of each other H or a branched or unbranched alkyl chain, a branched or unbranched alkenyl chain, a branched or unbranched alkynyl chain, a carbocycle, aryl, heteroaryl, a heterocycle, halogenyl, oxyalkyl, thioalkyl, carboxyl, carboxylic acid ester, carbonyl, carbamide, carbimide, thiocarbamide or thiocarbonyl.

In a preferred embodiment, R¹¹ and R¹⁴ are H.

In a further preferred embodiment, R¹² and R¹³ are independently of each other oxyalkyl or thioalkyl, halogenyl, or carboxylic acid alkyl ester or phenyl, or R¹² and R¹³ together are connected to form a dihydrobenzodioxine, a benzodioxole, a benzodithiole, a dihydrobenzodithiine, a benzooxathiole, a dihydrobenzooxathiine,
- wherein R¹⁵ and R¹⁶ are independently of each other H or a branched or unbranched alkyl chain, or a branched or unbranched alkenyl chain. In a preferred embodiment, at least one of R¹⁵ and R¹⁶ is H. Most preferably, R¹⁵ and R¹⁶ are both H.
- wherein R¹⁷ and R¹⁸ are independently of each other H or a branched or unbranched alkyl chain, a branched or unbranched alkenyl chain, a branched or unbranched alkynyl chain, a carbocycle, aryl or can be connected to form a carbocycle with up to 6 membersring atoms.

In a preferred embodiment, one of R¹⁷ and R¹⁸ is H and the other is Me.

Further preferred are compounds wherein one of R¹⁷ and R¹⁸ is H and the other is phenyl.

In a further preferred embodiment, R¹⁷ and R¹⁸ may form a carbocycle with up to 6 members in the ring atoms.
- wherein n is 0 or 1,
all of the above residues being optionally substituted independently of each other.

Furthermore, the present invention provides the use of the QC-inhibitors of the formula 10 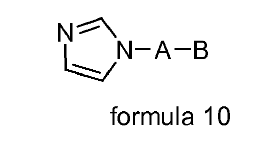 for the preparation of a medicament for the treatment of neuronal diseases optionally in combination with at least one agent, selected from the group consisting of PEP-inhibitors, inhibitors of dipeptidyl aminopeptidases, NPY-receptor ligands, NPY agonists, NPY antagonists, ACE inhibitors, PIMT enhancers, inhibitors of beta secretases, inhibitors of gamma secretases and inhibitors of neutral endopeptidase, wherein A and B are defined above.

Examples of suitable PIMT enhancers are 10-aminoaliphatyl-dibenz[b, f] oxepines of the general formula 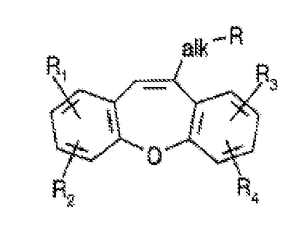 described in WO 98/15647 and WO 03/057204, respectively,
wherein alk is a divalent aliphatic radical, R is an amino group that is unsubstituted or mono- or di-substituted by monovalent aliphatic and/or araliphatic radicals or disubstituted by divalent aliphatic radicals, and R₁, R₂, R₃ and R₄ are each, independently of the others, hydrogen, lower alkyl, lower alkoxy, halogen or trifluoromethyl.

Further useful according to the present invention are modulators of PIMT activity of the general formulae I - IV: 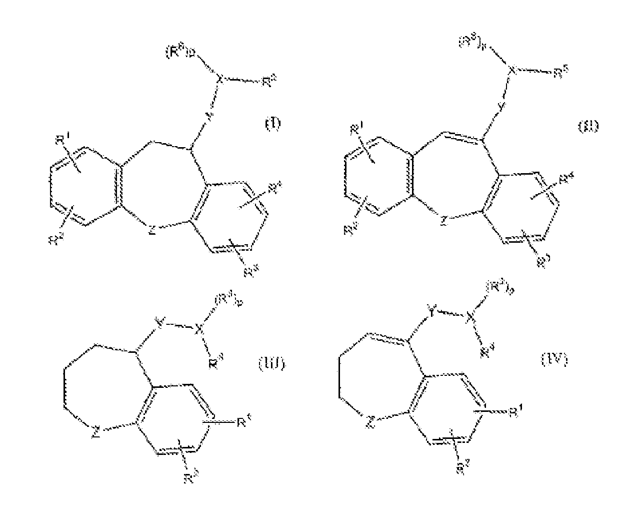 wherein the definition of the substituents R¹ - R⁵, (R³)p, (R⁶)p, X, Y and Z is described in WO 2004/039773.

WO 98/15647, WO 03/057204 and WO 2004/039773 are incorporated herein in their entirety and are part of this invention with regard to the synthesis and use of the compounds described therein.

Suitable inhibitors of beta and/or gamma secretases and compositions containing such inhibitors are described, e.g. in GB 2 385 124, GB 2 389 113, US 2002-115616, WO 01/87293, WO 03/057165, WO 2004/052348 and WO 2004/062652. These references are incorporated herein in their entirety and are part of this invention with regard to the synthesis, manufacture and use of the compounds and compositions described therein for the inhibition of beta and/or gamma secretases.

A potent selective and cell permeable gamma secretase inhibitor is (5S)-(t-Butoxycarbonylamino)-6-phenyl-(4R)hydroxy-(2R)benzylhexanoyl)-L-leu-L-phe-amide with the formula:

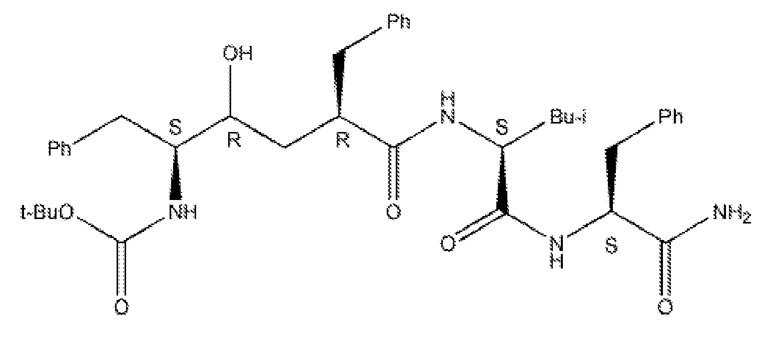

A potent beta secretase inhibitor is PNU-33312 of the formula:

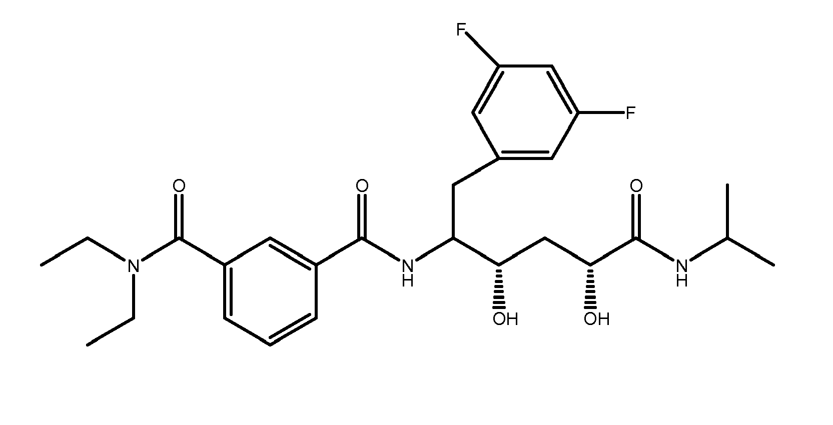

Suitable inhibitors of prolyl endopeptidase are, e.g. chemical derivatives of proline or small peptides containing terminal prolines. Benzyloxycarbonyl-prolyl-prolinal has been shown to be a specific transition state inhibitor of the enzyme (Wilk, S. and Orloeski, M., J. Neurochem., 41, 69 (1983), Friedman, et al., Neurochem., 42, 237 (1984)). N-terminal substitutions of L-proline or L-prolylpyrrolidine (Atack, et al., Eur. J. of Pharm., 205, 157-163 (1991), JP 03 56,460, EP 384,341), as well as variations of N-benzyloxycarbonyl (Z) dipeptides containing prolinal at the carboxy terminus have been synthesized as prolyl endopeptidase inhibitors (Nishikata, et al., Chem. Pharm. Bull. 34(7), 2931-2936 (1986), Baker, A. et al., Bioorganic & Medicinal Chem. Letts., 1(11), 585-590 (1991)). Thioproline, thiazolidine, and oxopyrrolidine substitutions of the core structure have been reported to inhibit prolyl endopeptidase (Tsuru, et al., J. Biochem., 94, 1179 (1988), Tsuru, et al., J. Biochem., 104, 580-586 (1988), Saito et al., J. Enz. Inhib. 5, 51-75 (1991), Uchida, I., et al. PCT Int. Appl. WO 90 12,005, JP 03 56,461, JP 03 56,462). Similarly, various modifications of the carboxy terminal proline have been made, including various fluorinated ketone derivatives (Henning, EP 4,912,127). General syntheses of fluorinated ketone derivatives has been described (Angelastro, M.R., et al., Tetrahedron Letters 33(23), 3265-3268 (1992)). Other compounds such as chloromethyl ketone derivatives of acyl-proline or acylpeptide-proline (Z-Gly-Pro-CH₂Cl) have been demonstrated to inhibit the enzyme by alkylating the enzyme's active site (Yoshimoto, T., et al., Biochemistry 16, 2942 (1977)).
EP-A-0 286 928 discloses 2-acylpyrrolidine derivatives useful as propyl endopeptidase inhibitors.

Further suitable prolyl endopeptidase inhibitors according to the present invention are, e.g. Fmoc-Ala-Pyrr-CN and those listed below:

| **Z-321** | **ONO-1603** |
|---|---|
| **Zeria Pharmaceutical Co Ltd** | **Ono Pharmaceutical Co Ltd** |
| 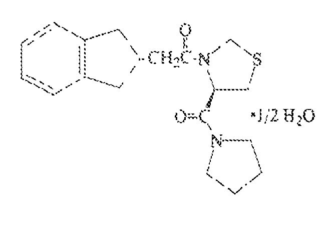 | 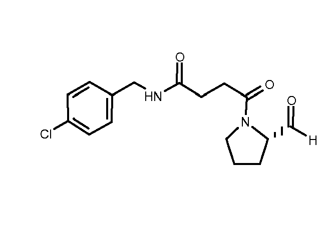 |
| **(4R)-3-(indan-2-ylacetyl)-4-(1-pyrrolidinyl-carbonyl)-1,3-thiazolidin** | **(S)-1-[N-(4-chlorobenzyl)-succinamoyl]pyrrolidin-2-carbaldehyd** |

| **JTP-4819** | **S-17092** |
|---|---|
| **Japan Tobacco Inc** | **Servier** |
| 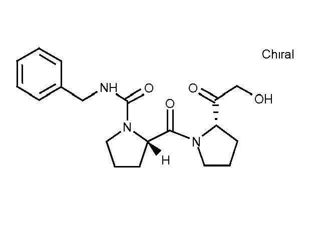 | 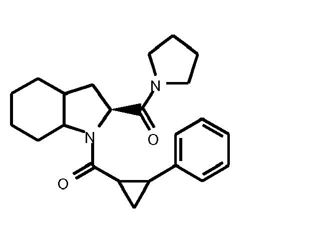 |
| **(S)-2-{[(S).(hydroxyacatyl)-1-pyrrolidinyl]carbony)}-N-(phenylmethyl)-1**-**pyrrolidin-carboxamid** | **(2S, 3aS, 7aS)-1{[(R,R)-2-phenylcyclopropyl] carbonyl}-2-[(thiazolidin-3-yl)carbonyl]octahydro-1*H*-indol** |

Further suitable prolyl endopeptidase inhibitors according to the present invention are disclosed in JP 01042465, JP 03031298, JP 04208299, WO 0071144, US 5847155; JP 09040693, JP 10077300, JP 05331072, JP 05015314, WO 9515310, WO 9300361, EP 0556482, JP 06234693, JP 01068396, EP 0709373, US 5965556, US 5756763, US 6121311, JP 63264454, JP 64000069, JP 63162672, EP 0268190, EP 0277588, EP 0275482, US 4977180, US 5091406, US 4983624, US 5112847, US 5100904, US 5254550, US 5262431, US 5340832, US 4956380, EP 0303434, JP 03056486, JP 01143897, JP 1226880, EP 0280956, US 4857537, EP 0461677, EP 0345428, 4JP 02275858, US 5506256, JP 06192298, EP 0618193, JP 03255080, EP 0468469, US 5118811, JP 05025125, WO 9313065, JP 05201970, WO 9412474, EP 0670309, EP 0451547, JP 06339390, US 5073549, US 4999349, EP 0268281, US 4743616, EP 0232849, EP 0224272, JP 62114978, JP 62114957, US 4757083, US 4810721, US 5198458, US 4826870, EP 0201742, EP 0201741, US 4873342, EP 0172458, JP 61037764, EP 0201743, US 4772587, EP 0372484, US 5028604, WO 9118877, JP 04009367, JP 04235162, US 5407950, WO 9501352, JP 01250370, JP 02207070, US 5221752, EP 0468339, JP 04211648 and WO 9946272, the teachings of which are herein incorporated by reference in their entirety, especially concerning these inhibitors, their definition, uses and their production.

Suitable DP IV-inhibitors are those, disclosed e.g. in US 6,380,398, US 6,011,155; US 6,107,317; US 6,110,949; US 6,124,305; US 6,172,081; WO 95/15309, WO 99/61431, WO 99/67278, WO 99/67279, DE 198 34 591, WO 97/40832, DE 196 16 486 C 2, WO 98/19998, WO 00/07617, WO 99/38501, WO 99/46272, WO 99/38501, WO 01/68603, WO 01/40180, WO 01/81337, WO 01/81304, WO 01/55105, WO 02/02560 and WO 02/14271, WO 02/04610, WO 02/051836, WO 02/068420, WO 02/076450; WO 02/083128, WO 02/38541, WO 03/000180, WO 03/000181, WO 03/000250, WO 03/002530, WO 03/002531, WO 03/002553,WO 03/002593, WO 03/004496, WO 03/004498, WO 03/024965, WO 03/024942, WO 03/035067, WO 03/037327, WO 03/035057, WO 03/045977, WO 03/055881, WO 03/68748, WO 03/68757, WO 03/057666, WO 03057144, WO 03/040174, WO 03/033524 and WO 03/074500.

Further suitable DP IV-inhibitors include valine pyrrolidide (Novo Nordisk), NVP-DPP728A (1-[ [ [ 2-[ {5-cyanopyridin-2-yl}amino]ethyl]amino]acetyl]-2-cyano-(S)-pyrrolidine) (Novartis) as disclosed by Hughes et al., Biochemistry, 38 (36), 11597-11603, 1999, LAF-237 (1-[(3-hydroxy-adamant-1-ylamino)-acetyl]-pyrrolidine-2(S)-carbonitrile); disclosed by Hughes et al., Meeting of the American Diabetes Association 2002, Abstract no. 272 or (Novartis), TSL-225 (tryptophyl-1,2,3,4-tetrahydroisoquinoline-3-carboxylic acid), disclosed by Yamada et. al., Bioorg. & Med. Chem. Lett. 8 (1998), 1537-1540, 2-cyanopyrrolidides and 4-cyanopyrrolidides as disclosed by Asworth et al., Bioorg. & Med. Chem. Lett., 6, No. 22, pp 1163-1166 and 2745-2748 (1996), FE-999011 ( [(2S)-1-([2'S]-2'-amino-3',3'dimethyl-butanoyl)-pyrrolidine-2-carbonitrile] ), disclosed by Sudre et al., Diabetes 51 (5), pp 1461-1469 (2002) (Ferring), GW-229A (GlaxoSmithKline), disclosed by Randhawa SA, et al, ACS Meeting 2003, 226th: New York (MEDI 91), MK-0431 ( (2R)-4-Oxo-4-[3-(trifluoromethyl)-5,6-dihydro[1,2,4]triazolo[4,3-*a*]pyrazin-7(8*H*)-yl]-1-(2,4,5-trifluorophenyl)butan-2-amine ) and the compounds disclosed in WO 01/34594 (Guilford), employing dosages as set out in the above references.

For the avoidance of doubt, the examples disclosed in each of the above mentioned publications are specifically incorporated herein by reference in their entirety, as individually disclosed compounds, especially concerning their structure, their definition, uses and their production.

Other suitable agents that can be used according to the present invention in combination with QC-inhibitors are NPY, a NPY mimetic or a NPY agonist or antagonist or a ligand of the NPY receptors.

Preferred according to the present invention are antagonists of the NPY receptors.

Suitable ligands or antagonists of the NPY receptors are 3a,4,5,9b-tetrahydro-1h-benz[e]indol-2-yl amine-derived compounds as disclosed in WO 00/68197.

NPY receptor antagonists which may be mentioned include those disclosed in European patent applications EP 0 614 911, EP 0 747 357, EP 0 747 356 and EP 0 747 378; international patent applications WO 94/17035, WO 97/19911, WO 97/19913, WO 96/12489, WO 97/19914, WO 96/22305, WO 96/40660, WO 96/12490, WO 97/09308, WO 97/20820, WO 97/20821, WO 97/20822, WO 97/20823, WO 97/19682, WO 97/25041, WO 97/34843, WO 97/46250, WO 98/03492, WO 98/03493, WO 98/03494 and WO 98/07420; WO 00/30674, US patents Nos. 5,552,411, 5,663,192 and 5,567,714; 6,114,336, Japanese patent application JP 09157253; international patent applications WO 94/00486, WO 93/12139, WO 95/00161 and WO 99/15498; US Patent No. 5,328,899; German patent application DE 393 97 97; European patent applications EP 355 794 and EP 355 793; and Japanese patent applications JP 06116284 and JP 07267988, the disclosures in all of which documents are hereby incorporated by reference. Preferred NPY antagonists include those compounds that are specifically disclosed in these patent documents. More preferred compounds include amino acid and non-peptide-based NPY antagonists. Amino acid and non-peptide-based NPY antagonists which may be mentioned include those disclosed in European patent applications EP 0 614 911, EP 0 747 357, EP 0 747 356 and EP 0 747 378; international patent applications WO 94/17035, WO 97/19911, WO 97/19913, WO 96/12489, WO 97/19914, WO 96/22305, WO 96/40660, WO 96/12490, WO 97/09308, WO 97/20820, WO 97/20821, WO 97/20822, WO 97/20823, WO 97/19682, WO 97/25041, WO 97/34843, WO 97/46250, WO 98/03492, WO 98/03493, WO 98/03494, WO 98/07420 and WO 99/15498 ; US patents Nos. 5,552,411, 5,663,192 and 5,567,714; and Japanese patent application JP 09157253. Preferred amino acid and non-peptide-based NPY antagonists include those compounds that are specifically disclosed in these patent documents.

Particularly preferred compounds include amino acid-based NPY antagonists. Amino acid-based compounds which may be mentioned include those disclosed in international patent applications WO 94/17035, WO 97/19911, WO 97/19913, WO 97/19914 or, preferably, WO 99/15498. Preferred amino acid-based NPY antagonists include those that are specifically disclosed in these patent documents, for example BIBP3226 and, especially, (R)-N2-(diphenylacetyl)-(R)-N-[1-(4-hydroxy- phenyl) ethyl] arginine amide (Example 4 of international patent application WO 99/15498).

For the avoidance of doubt, the examples disclosed in each of the above mentioned publications are specifically incorporated herein by reference in their entirety, as individually disclosed compounds, especially concerning their structure, their definition, uses and their production.

Preferred DP IV-inhibitors are dipeptide-like compounds and compounds analogous to dipeptide compounds that are formed from an amino acid and a thiazolidine or pyrrolidine group, and salts thereof, referred to hereinafter as dipeptide-like compounds. Preferably the amino acid and the thiazolidine or pyrrolidine group are bonded with an amide bond.

Especially suitable for that purpose according to the invention are dipeptide-like compounds in which the amino acid is preferably selected from a natural amino acid, such as, for example, leucine, valine, glutamine, glutamic acid, proline, isoleucine, asparagines and aspartic acid.

The dipeptide-like compounds used according to the invention exhibit at a concentration (of dipeptide compounds) of 10 µM, a reduction in the activity of plasma dipeptidyl peptidase IV or DP IV-analogous enzyme activities of at least 10 %, especially of at least 40 %. Frequently a reduction in activity of at least 60 % or at least 70 % is also required. Preferred agents may also exhibit a reduction in activity of a maximum of 20 % or 30 %.

Preferred compounds are N-valyl prolyl, O-benzoyl hydroxylamine, alanyl pyrrolidine, isoleucyl thiazolidine like L-allo-isoleucyl thiazolidine, L-threo-isoleucyl pyrrolidine and salts thereof, especially the fumaric salts, and L-allo-isoleucyl pyrrolidine and salts thereof.

Further preferred compounds are given in Table 2.

The salts of the dipeptide-like compounds can be present in a molar ratio of dipeptide (-analogous) component to salt component of 1 : 1 or 2 : 1. Such a salt is, for example, (Ile-Thia)₂ fumaric acid.

**Table 2: Structures of further preferred dipeptide compounds**

| **DP IV-inhibitor** |
|---|
| H-Asn-pyrrolidine |
| H-Asn-thiazolidine |
| H-Asp-pyrrolidine |
| H-Asp-thiazolidine |
| H-Asp(NHOH)-pyrrolidine |
| H-Asp(NHOH)-thiazolidine |
| H-Glu-pyrrolidine |
| H-Glu-thiazolidine |
| H-Glu(NHOH)-pyrrolidine |
| H-Glu(NHOH)-thiazolidine |
| H-His-pyrrolidine |
| H-His-thiazolidine |
| H-Pro-pyrrolidine |
| H-Pro-thiazolidine |
| H-Ile-azididine |
| H-Ile-pyrrolidine |
| H-L-*allo*-Ile-thiazolidine |
| H-Val-pyrrolidine |
| H-Val-thiazolidine |

In another preferred embodiment, the present invention provides the use of substrate-like peptide compounds of formula 11 useful for competitive modulation of dipeptidyl peptidase IV catalysis for combination therapy of neuronal diseases: 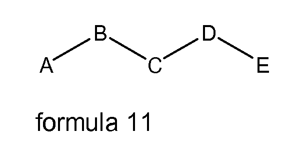 wherein

A, B, C, D and E are independently any amino acid moieties including proteinogenic amino acids, non-proteinogenic amino acids, L-amino acids and D-amino acids and wherein E and/or D may be absent.

Further definitions regarding formula 11:
A is an amino acid except a D-amino acid,
B is an amino acid selected from Pro, Ala, Ser, Gly, Hyp, acetidine-(2)-carboxylic acid and pipecolic acid,
C is any amino acid except Pro, Hyp, acetidine-(2)-carboxylic acid, pipecolic acid and except N-alkylated amino acids, e.g. N-methyl valine and sarcosine,
D is any amino acid or missing, and
E is any amino acid or missing,
or:
C is any amino acid except Pro, Hyp, acetidine-(2)-carboxylic acid, pipecolic acid, except N-alkylated amino acids, e.g. N-methyl valine and sarcosine, and except a D-amino-acid;
D is any amino acid selected from Pro, Ala, Ser, Gly, Hyp, acetidine-(2)-carboxylic acid and pipecolic acid, and
E is any amino acid except Pro, Hyp, acetidine-(2)-carboxylic acid, pipecolic acid and except N-alkylated amino acids, e.g. N-methyl valine and sarcosine.

Examples of amino acids which can be used in the present invention are: L and D-amino acids, N-methyl-amino-acids; allo- and threo-forms of Ile and Thr, which can, e.g. be α-, β- or ω-amino acids, whereof α-amino acids are preferred.

Examples of amino acids throughout the claims and the description are: aspartic acid (Asp), glutamic acid (Glu), arginine (Arg), lysine (Lys), histidine (His), glycine (Gly), serine (Ser) and cysteine (Cys), threonine (Thr), asparagine (Asn), glutamine (Gln), tyrosine (Tyr), alanine (Ala), proline (Pro), valine (Val), isoleucine (Ile), leucine (Leu), methionine (Met), phenylalanine (Phe), tryptophan (Trp), hydroxyproline (Hyp), beta-alanine (beta-Ala), 2-amino octanoic acid (Aoa), azetidine-(2)-carboxylic acid (Ace), pipecolic acid (Pip), 3-amino propionic, 4-amino butyric and so forth, alpha-aminoisobutyric acid (Aib), sarcosine (Sar), ornithine (Orn), citrulline (Cit), homoarginine (Har), t-butylalanine (t-butyl-Ala), t-butylglycine (t-butyl-Gly), N-methylisoleucine (N-Melle), phenylglycine (Phg), cyclohexylalanine (Cha), norleucine (Nle), cysteic acid (Cya) and methionine sulfoxide (MSO), Acetyl-Lys, modified amino acids such as phosphoryl-serine (Ser(P)), benzyl-serine (Ser(Bzl)) and phosphoryl-tyrosine (Tyr(P)), 2-aminobutyric acid (Abu), aminoethylcysteine (AECys), carboxymethylcysteine (Cmc), dehydroalanine (Dha), dehydroamino-2-butyric acid (Dhb), carboxyglutaminic acid (Gla), homoserine (Hse), hydroxylysine (Hyl), *cis-*hydroxyproline (cisHyp), *trans*-hydroxyproline (transHyp), isovaline (Iva), pyroglutamic acid (Pyr), norvaline (Nva), 2-aminobenzoic acid (2-Abz), 3- aminobenzoic acid (3-Abz), 4- aminobenzoic acid (4-Abz), 4-(aminomethyl)benzoic acid (Amb), 4-(aminomethyl)cyclohexanecarboxylic acid (4-Amc), Penicillamine (Pen), 2-Amino-4-cyanobutyric acid (Cba), cycloalkane-carboxylic aicds.

Examples of ω̅-amino acids are e.g.: 5-Ara (aminoraleric acid), 6-Ahx (aminohexanoic acid), 8-Aoc (aminooctanoic aicd), 9-Anc (aminovanoic aicd), 10-Adc (aminodecanoic acid), 11-Aun (aminoundecanoic acid), 12-Ado (aminododecanoic acid).

Further amino acids are: indanylglycine (Igl), indoline-2-carboxylic acid (Idc), octahydroindole-2-carboxylic acid (Oic), diaminopropionic acid (Dpr), diaminobutyric acid (Dbu), naphtylalanine (1-Nal), (2-Nal), 4-aminophenylalanin (Phe(4-NH₂)), 4-benzoylphenylalanine (Bpa), diphenylalanine (Dip), 4-bromophenylalanine (Phe(4-Br)), 2-chlorophenylalanine (Phe(2-Cl)), 3-chlorophenylalanine (Phe(3-Cl)), 4-chlorophenylalanine (Phe(4-Cl)), 3,4-chlorophenylalanine (Phe (3,4-Cl₂)), 3-fluorophenylalanine (Phe(3-F)), 4- fluorophenylalanine (Phe(4-F)), 3,4-fluorophenylalanine (Phe(3,4-F₂)), pentafluorophenylalanine (Phe(F₅)), 4-guanidinophenylalanine (Phe(4-guanidino)), homophenylalanine (hPhe), 3-jodophenylalanine (Phe(3-J)), 4 jodophenylalanine (Phe(4-J)), 4-methylphenylalanine (Phe(4-Me)), 4-nitrophenylalanine (Phe-4-NO₂)), biphenylalanine (Bip), 4-phosphonomehtylphenylalanine (Pmp), cyclohexyglycine (Ghg), 3-pyridinylalanine (3-Pal), 4-pyridinylalanine (4-Pal), 3,4-dehydroproline (A-Pro), 4-ketoproline (Pro(4-keto)), thioproline (Thz), isonipecotic acid (Inp), 1,2,3,4,-tetrahydroisoquinolin-3-carboxylic acid (Tic), propargylglycine (Pra), 6-hydroxynorleucine (NU(6-OH)), homotyrosine (hTyr), 3-jodotyrosine (Tyr(3-J)), 3,5-dijodotyrosine (Tyr(3,5-J₂)), d-methyl-tyrosine (Tyr(Me)), 3-NO₂-tyrosine (Tyr(3-NO₂)), phosphotyrosine (Tyr(PO₃H₂)), alkylglycine, 1-aminoindane-1-carboxy acid, 2-aminoindane-2-carboxy acid (Aic), 4-amino-methylpyrrol-2-carboxylic acid (Py), 4-amino-pyrrolidine-2-carboxylic acid (Abpc), 2-aminotetraline-2-carboxylic acid (Atc), diaminoacetic acid (Gly(NH₂)), diaminobutyric acid (Dab), 1,3-dihydro-2H-isoinole-carboxylic acid (Disc), homocylcohexylalanin (hCha), homophenylalanin (hPhe oder Hof), *trans*-3-phenyl-azetidine-2-carboxylic acid, 4-phenyl-pyrrolidine-2-carboxylic acid, 5-phenyl-pyrrolidine-2-carboxylic acid, 3-pyridylalanine (3-Pya), 4-pyridylalanine (4-Pya), styrylalanine, tetrahydroisoquinoline-1-carboxylic acid (Tiq), 1,2,3,4-tetrahydronorharmane-3-carboxylic acid (Tpi), β-(2-thienryl)-alanine (Tha).

Other amino acid substitutions for those encoded in the genetic code can also be included in peptide compounds within the scope of the invention and can be classified within this general scheme.

Proteinogenic amino acids are defined as natural protein-derived α-amino acids. Non-proteinogenic amino acids are defined as all other amino acids, which are not building blocks of common natural proteins.

The resulting peptides may be synthesized as the free C-terminal acid or as the C-terminal amide form. The free acid peptides or the amides may be varied by side chain modifications. Such side chain modifications include for instance, but are not restricted to, homoserine formation, pyroglutamic acid formation, disulphide bond formation, deamidation of asparagine or glutamine residues, methylation, t-butylation, t-butyloxycarbonylation, 4-methylbenzylation, thioanysilation, thiocresylation, benzyloxymethylation, 4-nitrophenylation, benzyloxycarbonylation, 2-nitrobencoylation, 2-nitrosulphenylation, 4-toluenesulphonylation, pentafluorophenylation, diphenylmethylation, 2-chlorobenzyloxycarbonylation, 2,4,5-trichlorophenylation, 2-bromobenzyloxycarbonylation, 9-fluorenylmethyloxycarbonylation, triphenylmethylation, 2,2,5,7,8,-pentamethylchroman-6-sulphonylation, hydroxylation, oxidation of methionine, formylation, acetylation, anisylation, benzylation, bencoylation, trifluoroacetylation, carboxylation of aspartic acid or glutamic acid, phosphorylation, sulphation, cysteinylation, glycolysation with pentoses, deoxyhexoses, hexosamines, hexoses or N-acetylhexosamines, farnesylation, myristolysation, biotinylation, palmitoylation, stearoylation, geranylgeranylation, glutathionylation, 5'-adenosylation, ADP-ribosylation, modification with N-glycolylneuraminic acid, N-acetylneuraminic acid, pyridoxal phosphate, lipoic acid, 4'-phosphopantetheine, or N-hydroxysuccinimide.

In the compounds of formula (3), the amino acid moieties A, B, C, D, and E are respectively attached to the adjacent moiety by amide bonds in a usual manner according to standard nomenclature so that the amino-terminus (N-terminus) of the amino acids (peptide) is drawn on the left and the carboxyl-terminus of the amino acids (peptide) is drawn on the right. (C-terminus).

Preferred peptide compounds are listed in table 3.

**Table 3: Examples of peptide substrates**

| **Peptide** | **Mass (calc.)** | **Mass (exp.)¹ [M+H⁺]** |
|---|---|---|
| 2-Amino octanoic acid-Pro-Ile | 369.5 | 370.2 |
| Abu-Pro-Ile | 313.4 | 314.0 |
| Aib-Pro-Ile | 313.4 | 314.0 |
| Aze-Pro-Ile | 311.4 | 312.4 |
| Cha-Pro-Ile | 381.52 | 382.0 |
| Ile-Hyp-Ile | 356.45 | 358.2 |
| Ile-Pro-*allo*-Ile | 341.4 | 342.0 |
| Ile-Pro-*t*-butyl-Gly | 341,47 | 342,36 |
| Ile-Pro-Val | 327.43 | 328.5 |
| Nle-Pro-Ile | 341.45 | 342.2 |
| Nva-Pro-Ile | 327.43 | 328.2 |
| Orn-Pro-Ile | 342.42 | 343.1 |
| Phe-Pro-Ile | 375.47 | 376.2 |
| Phg-Pro-Ile | 361.44 | 362.2 |
| Pip-Pro-Ile | 338.56 | 340.0 |
| Ser(Bzl)-Pro-Ile | 405.49 | 406.0 |
| Ser(P)-Pro-Ile | 395.37 | 396.0 |
| Ser-Pro-Ile | 315.37 | 316.3 |
| *t*-butyl-Gly-Pro-D-Val | 327.4 | 328.6 |
| *t*-butyl-Gly -Pro-Gly | 285.4 | 286.3 |
| *t*-butyl-Gly -Pro-Ile | 341.47 | 342.1 |
| *t*-butyl-Gly -Pro-Ile-amide | 340.47 | 341.3 |
| *t*-butyl-Gly-Pro-*t*-butyl-Gly | 341.24 | 342.5 |
| *t*-butyl-Gly-Pro-Val | 327.4 | 328.4 |
| Thr-Pro-Ile | 329.4 | 330.0 |
| Tic-Pro-Ile | 387.46 | 388.0 |
| Trp-Pro-Ile | 414.51 | 415.2 |
| Tyr(P)-Pro-Ile | 471.47 | 472.3 |
| Tyr-Pro-*allo*-Ile | 391.5 | 392.0 |
| Val-Pro-*allo*-Ile | 327.4 | 328.5 |
| Val-Pro-*t*-butyl-Gly | 327.4 | 328.15 |
| Val-Pro-Val | 313.4 | 314.0 |

| | | |
|---|---|---|
| ¹ [M+H⁺] were determined by Electrospray mass spectrometry in positive ionization mode. | | |

*t*-butyl-Gly is defined as:

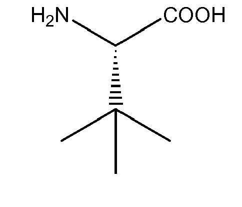

Ser(Bzl) and Ser(P) are defined as benzyl-serine and phosphoryl-serine, respectively. Tyr(P) is defined as phosphoryl-tyrosine.

Further preferred DP IV-inhibitors, which can be used according to the present invention for combination therapy of neuronal diseases, are peptidylketones of formula 12: 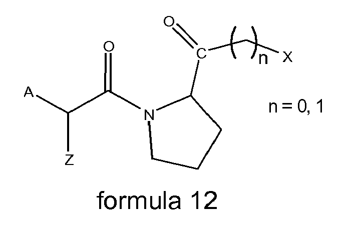 and pharmaceutically acceptable salts thereof, wherein:
A is selected from the following structures: 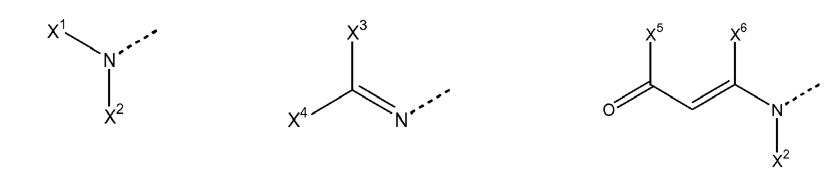 wherein
   X¹ is H or an acyl or oxycarbonyl group including an amino acid residue, N-protected amino acid residue, a peptide residue or a N-protected peptide residue,
   X² is H, -(CH)ₘ-NH-C₅H₃N-Y with m = 2-4 or -C₅H₃N-Y (a divalent pyridyl residue) and Y is selected from H, Br, Cl, I, NO₂ or CN,
   X³ is H or selected from an alkyl-, alkoxy-, halogen-, nitro-, cyano- or carboxy-substituted phenyl or from an alkyl-, alkoxy-, halogen-, nitro-, cyano- or carboxy-substituted pyridyl residue,
   X⁴ is H or selected from an alkyl-, alkoxy-, halogen-, nitro-, cyano- or carboxy-substituted phenyl or from an alkyl-, alkoxy-, halogen-, nitro-, cyano- or carboxy-substituted pyridyl residue,
   X⁵ is H or an alkyl, alkoxy or phenyl residue,
   X⁶ is H or an alkyl residue,
   for n = 1
X is selected from: H, OR², SR², NR²R³, N⁺R²R³R⁴, wherein:
   R² stands for acyl residues, which are optionally substituted with alkyl, cycloalkyl, aryl or heteroaryl residues, or for amino acid residues or peptidic residues, or alkyl residues, which are optionally substituted with alkyl, cycloalkyl, aryl or heteroaryl residues,
   R³ stands for alkyl or acyl residues, wherein R² and R³ may be part of a saturated or unsaturated carbocyclic or heterocyclic ring,
   R⁴ stands for alkyl residues, wherein R² and R⁴ or R³ and R⁴ may be part of a saturated or unsaturated carbocyclic or heterocyclic ring,
   for n = 0
X is selected from: 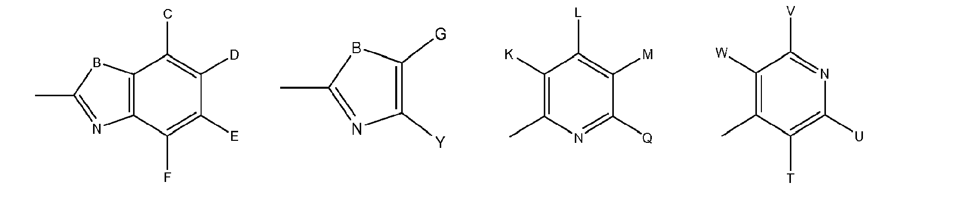 wherein
   B stands for: O, S or NR⁵, wherein R⁵ is H, alkyl or acyl,
   C, D, E, F, G, Y, K, L, M, Q, T, U, V and W are independently selected from alkyl and substituted alkyl residues, oxyalkyl, thioalkyl, aminoalkyl, carbonylalkyl, acyl, carbamoyl, aryl and heteroaryl residues, and
Z is selected from H, or a branched or straight chain alkyl residue from C₁-C₉, a branched or straight chain alkenyl residue from C₂-C₉, a cycloalkyl residue from C₃-C₈, a cycloalkenyl residue from C₅-C₇, an aryl or heteroaryl residue, or a side chain selected from all side chains of all natural amino acids or derivatives thereof.

In preferred compounds of formula 12, A is 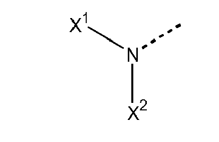 wherein
X¹ is H or an acyl or oxycarbonyl group including an amino acid residue, N-acylated amino acid residue, a peptide residue from di- to pentapeptides, preferably a dipeptide residue, or a N-protected peptide residue from di-to pentapeptides, preferably a N-protected dipeptide residue
X² is H, -(CH)ₘ-NH-C₅H₃N-Y with m = 2-4 or -C₅H₃N-Y (a divalent pyridyl residue) and Y is selected from H, Br, Cl, 1, NO₂ or CN,
   for n = 1
X is preferably selected from: H, OR², SR², NR²R³, wherein:
   R² stands for acyl residues, which are optionally substituted with alkyl, cycloalkyl, aryl or heteroaryl residues, or for amino acid residues or peptidic residues, or alkyl residues, which are optionally substituted with alkyl, cycloalkyl, aryl or heteroaryl residues,
   R³ stands for alkyl or acyl residues, wherein R² and R³ may be part of a saturated or unsaturated carbocyclic or heterocyclic ring,
   for n = 0
X is preferably selected from: 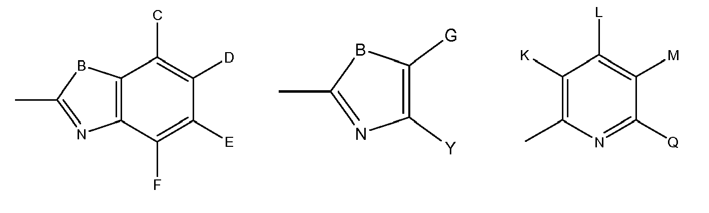 wherein
   B stands for: O, S or NR⁵, wherein R⁵ is H, alkyl or acyl,
   C, D, E, F, G, Y, K, L, M and Q are independently selected from alkyl and substituted alkyl residues, oxyalkyl, thioalkyl, aminoalkyl, carbonylalkyl, acyl, carbamoyl, aryl and heteroaryl residues, and
Z is selected from H, or a branched or straight chain alkyl residue from C₁-C₉, preferably C₂- C₆, a branched or straight chain alkenyl residue from C₂-C₉, a cycloalkyl residue from C₃-C₈, a cycloalkenyl residue from C₅-C₇, an aryl or heteroaryl residue, or a side chain selected from all side chains of all natural amino acids or derivatives thereof.

In more preferred compounds of formula 12, A is 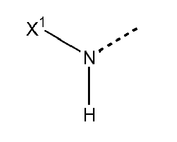 wherein
X¹ is H or an acyl or oxycarbonyl group including an amino acid residue, N-acylated amino acid residue or a peptide residue from di- to pentapeptides, preferably a dipeptide residue, or a N-protected peptide residue from di- to pentapeptides, preferably a N-protected dipeptide residue
for n = 1,
X is preferably selected from: H, OR², SR², wherein:
   R² stands for acyl residues, which are optionally substituted with alkyl or aryl residues,
   for n = 0
X is preferably selected from: 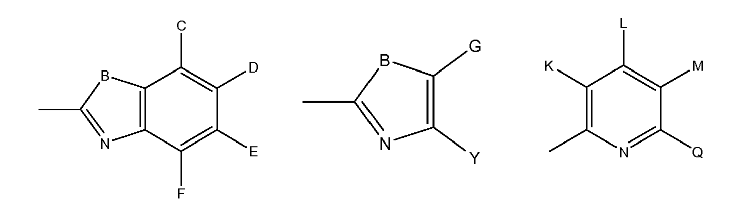 wherein
   B stands for: O, S or NR⁵, wherein R⁵ is H, alkyl or acyl,
   C, D, E, F, G, Y, K, L, M and Q are independently selected from alkyl and substituted alkyl residues, oxyalkyl, thioalkyl, aminoalkyl, carbonylalkyl, acyl, carbamoyl, aryl and heteroaryl residues, and
Z is selected from H, or a branched or straight chain alkyl residue from C₁-C₉, preferably C₂- C₆, a branched or straight chain alkenyl residue from C₂-C₉, a cycloalkyl residue from C₃-C₈, a cycloalkenyl residue from C₅-C₇, an aryl or heteroaryl residue, or a side chain selected from all side chains of all natural amino acids or derivatives thereof.

In most preferred compounds of formula 12, A is 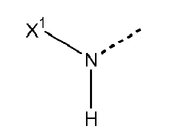 wherein
X¹ is H or an acyl or oxycarbonyl group including an amino acid residue, N-acylated amino acid residue or a dipeptide residue, containing a Pro or Ala in the penultimate position, or a N-protected dipeptide residue containing a Pro or Ala in the penultimate position,
forn=1,
X is H,
   for n = 0
X is preferably selected from: 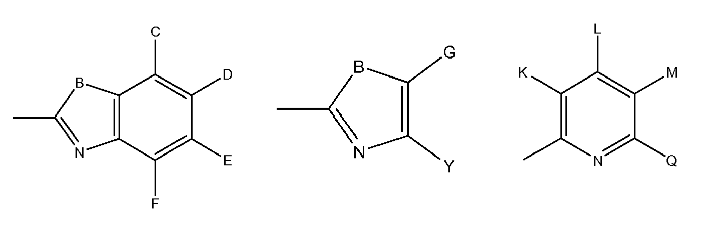 wherein
   B stands for: O or S, most preferably for S
   C, D, E, F, G, Y, K, L, M, Q, are H and
Z is selected from H, or a branched or straight chain alkyl residue from C₃-C₅, a branched or straight chain alkenyl residue from C₂-C₉, a cycloalkyl residue from C₅-C₇, a cycloalkenyl residue from C₅-C₇, an aryl or heteroaryl residue, or a side chain selected from all side chains of all natural amino acids or derivatives thereof.
   Most preferred for Z is H.

According to a preferred embodiment the acyl groups are C₁-C₆-acyl groups.

According to a further preferred embodiment the alk(yl) groups are C₁-C₆-alk(yl) groups, which may be branched or unbranched.

According to a still further preferred embodiment the alkoxy groups are C₁-C₆-alkoxy groups.

According to yet another preferred embodiment the aryl residues are C₅-C₁₂ aryl residues that have optionally fused rings.

According to a still further preferred embodiment the cycloalkyl residues (carbocycles) are C₃-C₈-cycloalkyl residues.

According to another preferred embodiment the heteroaryl residues are C₄-C₁₁ aryl residues that have optionally fused rings and, in at least one ring, additionally from 1 to 4 preferably 1 or 2 hetero atoms, such as O, N and/or S.

According to a further preferred embodiment peptide residues are corresponding residues containing from 2 to 50 amino acids.

According to another preferred embodiment the heterocyclic residues are C₂-C₇-cycloalkyl radicals that additionally have from 1 to 4, preferably 1 or 2 hetero atoms, such as O, N and/or S.

According to astill further preferred embodiment the carboxy groups are C₁ - C₆ carboxy groups, which may be branched or unbranched.

According to yet another preferred embodiment the oxycarbonyl groups are groups of the formula -O-(CH₂)₁₋₆COOH.

The amino acids can be any natural or synthetic amino acid, preferably natural alpha amino acids.

Preferred compounds of formula (4) are 2-Methylcarbonyl-1-N-[(L)-Alanyl-(L)-Valinyl]-(2S)-pyrrolidine hydrobromide; 2-Methyl)carbonyl-1-N-[(L)-Valinyl-(L)-Prolyl-(L)-Valinyl]-(2S)-pyrrolidine hydrobromide; 2-[(Acetyl-oxy-methyl)carbonyl]-1-N-[(L)-Alanyl-(L)-Valinyl]-(2S)-pyrrolidine hydrobromide; 2-[Benzoyl-oxy-methyl)carbonyl]-1-N-[{(L)-Alanyll-(L)-Valinyl]-(2S)-pyrrolidine hydrobromide; 2-{[(2,6-Dichlorbenzyl)thiomethyl]carbonyl}-1-N-[{(L)-Alanyl}-(L)-Valinyl]-(2S)-pyrrolidine; 2-[Benzoy-loxy-methyl)carbonyl]-1-N-[Glycyl-(L)-Valinyl]-(2S)-pyrrolidine hydrobromide; 2-[([1,3]-thiazole-2-yl)carbonyl]-1-N-[{(L)-Alanyl}-(L)-Valinyl]-(2S)-pyrrolidine trifluoracetat; 2-[(benzothiazole-2-yl)carbonyl]-1-N-[N-((L)-Alanyl)-(L)-Valinyl]-(2S)-pyrrolidin trifluoracetat; 2-[(-benzothiazole-2-yl)carbonyl]-1-N-[((L)-Alanyl)-Glycyl]-(2S)-pyrrolidine trifluoracetat; 2-[(pyridin-2-yl)carbonyl]-1-N-[N-((L)-Alanyl)-(L)-Valinyl]-(2S)-pyrrolidine trifluoracetat.

Further, according to the present invention DP IV-inhibitors of formula 13 including all stereoisomers and pharmaceutical acceptable salts thereof can be used for combination therapy of neuronal diseases:

**B-(CH**--R¹**)ₙ--C(=X²)-D** formula 13

wherein
n is 0 or 1,
R¹ stands for H, C₁-C₉ branched or straight chain alkyl, preferably H, n-butan-2-yl, n-prop-2-yl or isobutyl, C₂-C₉ branched or straight chain alkenyl, C₃-C₈ cycloalkyl, preferably cyclohexyl, C₅-C₇ cycloalkenyl, aryl, heteroaryl or a side chain of a natural amino acid or mimetics thereof,
X² stands for O, NR⁶, N⁺(R⁷)₂, or S,
B is selected from the following groups: 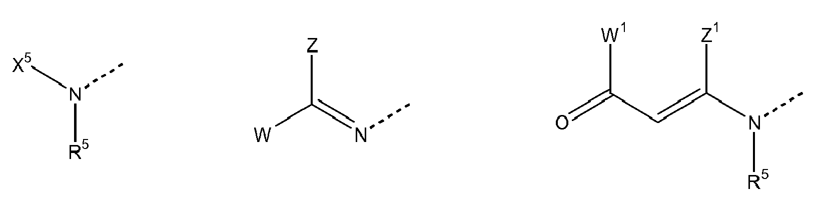 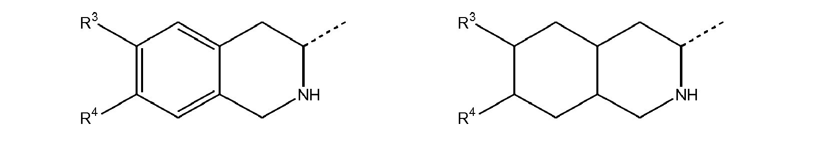 where X⁵ is H or an acyl or oxycarbonyl group including amino acids,
   R⁵ is H, C₁-C₉ branched or straight chain alkyl, preferably H, n-butan-2-yl, n-prop-2-yl or isobutyl, C₂-C₉ branched or straight chain alkenyl, C₃-C₈ cycloalkyl, preferably cyclohexyl, 3-hydroxyadamant-1-yl, C₅-C₇ cycloalkenyl, aryl, heteroaryl or a side chain of a natural amino acid or derivatives thereof, or a group of the formula -(CH)ₘ-NH-C₅H₃N-Y where m is an integer of 2-4, -C₅H₃N-Y is a divalent pyridyl moiety and Y is a hydrogen atom, a halogen atom, a nitro group or a cyano group,
   R⁶, R⁷ R⁸ and R⁹ are independently selected from H, optionally substituted C₁-C₉ branched or straight chain alkyl, preferably an optionally substituted C₂-C₅ branched or straight chain alkyl; or optionally substituted C₂-C₉ branched or straight chain alkenyl, preferably an C₂-C₅ branched or straight chain alkenyl; or optionally substituted C₃-C₈ cycloalkyl, preferably an optionally substituted C₄-C₇ cycloalkyl; or an optionally substituted C₅-C₇ cycloalkenyl, or an optionally substituted aryl residue,
   Z is selected from H, pyridyl or optionally substituted phenyl, optionally substituted alkyl groups, alkoxy groups, halogens, nitro, cyano and carboxy groups,
   W is selected from H, pyridyl or optionally substituted phenyl, optionally substituted alkyl groups, alkoxy groups, halogens, nitro, cyano and carboxy groups,
   W1 is H or optionally substituted alkyl, alkoxy or optionally substituted phenyl, and
   Z¹ is H, or optionally substituted alkyl,
   R³ and R⁴ are independently H, hydroxy, alkyl, alkoxy, aralkoxy, nitro, cyano or halogen,
D is an optionally substituted compound of the formula 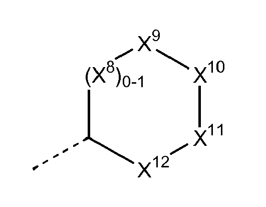 which can be saturated, or can have one, two or three double bonds,
   wherein
   X⁸ to X¹¹ are independently CH, N, N⁺(R⁷), or CR⁸, if unsaturated, or
   X⁸ to X¹¹ are independently CH₂, NH, NH⁺(R⁷), O, or S if saturated,
   X¹² is CHA, NA, CH₂, NH, NH⁺(R⁷), or CHR⁸, if saturated or
   X¹² is CA, NA⁺, CH, N, N⁺(R⁷), or CR⁸, if unsaturated and
   A is H or an isoster of a carboxylic acid such as CN, SO₃H, CONOH, PO₃R⁵R⁶, a tetrazole, an amide, an ester or an acid anhydride.

Throughout the application, D contains preferably at most two, further preferred at most one hetero atom in the ring.

According to preferred embodiments of the present invention, D stands for optionally substituted C₄-C₇ cycloalkyl, preferably C₄-C₆ cycloalkyl, optionally substituted C₄-C₇ cycloalkenyl, or optionally substituted (hetero)cycloalkyl of the formulae 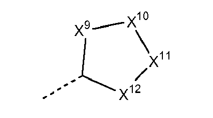 wherein the residues are as defined above,
or 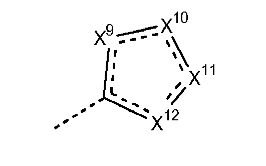 that is, a five-membered ring containing one or two double bonds in the ring,
wherein the residues are as defined above,
or 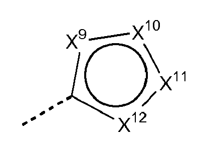 wherein the residues are as defined above,
or 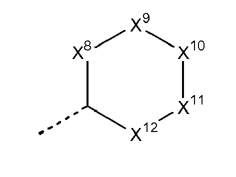 wherein the residues are as defined above,
or 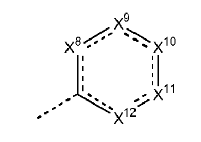 that is a six-membered ring containing one or two double bonds in the ring,
wherein the residues are as defined above,
or 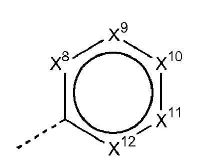 wherein the residues are as defined above.

According to a preferred embodiment, B has the following formula: 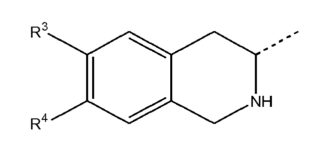 wherein the residues are as defined above.

According to another preferred embodiment, B has the following formula: 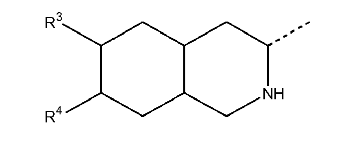 wherein the residues are as defined above.

Preferred compounds according to formula 13 are
1-cyclopentyl-3-methyl-1-oxo-2-pentanaminium chloride,
*1*-cyclopentyl-3-methyl-1-oxo-2-butanaminium chloride,
1-cyclopentyl-3,3-dimethyl-1-oxo-2-butanaminium chloride,
1-cyclohexyl-3,3-dimethyl-1-oxo-2-butanaminium chloride,
3-(cyclopentylcarbonyl)-1,2,3,4-tetrahydroisoquinolinium chloride, and
*N*-(2-cyclopentyl-2-oxoethyl)cyclohexanaminium chloride.

Because of the wide distribution of the protein in the body and the wide variety of mechanisms involving DP IV, DP IV-activity and DP IV-related proteins, systemic therapy (enteral or parenteral administration) with DP IV-inhibitors can result in a series of undesirable side-effects.

The problem to be solved was moreover, to provide DP IV-inhibitors that can be used in combination therapy of neuronal diseases, for targeted influencing of locally limited patho-physiological and physiological processes. The problem of the invention especially consists in obtaining locally limited and highly specific inhibition of DP IV or DP IV-analogous activity for the purpose of targeted intervention in the regulation of the activity of locally active substrates.

This problem is solved according to the invention by the use of the DP IV-inhibitors of the general formula 14 in combination therapy of neuronal disorders: 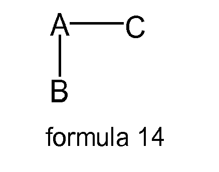 wherein
A is an amino acid having at least one functional group in the side chain,
B is a chemical compound covalently bound to at least one functional group of the side chain of A,
C is a thiazolidine, pyrrolidine, cyanopyrrolidine, hydroxyproline, dehydroproline or piperidine group amide-bonded to A.

In accordance with a preferred embodiment of the invention, pharmaceutical compositions are used comprising at least one compound of the general formula (6) and at least one customary adjuvant appropriate for the site of action.

Preferably A is an α-amino acid, especially a natural α-amino acid having one, two or more functional groups in the side chain, preferably threonine, tyrosine, serine, arginine, lysine, aspartic acid, glutamic acid or cysteine.

Preferably B is an oligopeptide having a chain length of up to 20 amino acids, a polyethylene glycol having a molar mass of up to 20 000 g/mol, an optionally substituted organic amine, amide, alcohol, acid or aromatic compound having from 8 to 50 C atoms.

Despite an extended side chain function, the compounds of formula 14 can still bind to the active centre of the enzyme dipeptidyl peptidase IV and analogous enzymes but are no longer actively transported by the peptide transporter PepT1. The resulting reduced or greatly restricted transportability of the compounds according to the invention leads to local or site directed inhibition of DP IV and DP IV-like enzyme activity.

By extending/expanding the side chain modifications, for example beyond a number of seven carbon atoms, it is accordingly possible to obtain a dramatic reduction in transportability. With increasing spatial size of the side chains, there is a reduction in the transportability of the substances. By spatially and sterically expanding the side chains, for example beyond the atom group size of a monosubstituted phenyl radical, hydroxylamine radical or amino acid residue, it is possible according to the invention to modify or suppress the transportability of the target substances.

Preferred compounds of formula 14 are compounds, wherein the oligopeptides have chain lengths of from 3 to 15, especially from 4 to 10, amino acids, and/or the polyethylene glycols have molar masses of at least 250 g/mol, preferably of at least 1500 g/mol and up to 15 000 g/mol, and/or the optionally substituted organic amines, amides, alcohols, acids or aromatic compounds have at least 12 C atoms and preferably up to 30 C atoms.

To prepare the pharmaceutical compositions of this invention, at least one effector of QC optionally in combination with at least one PEP-inhibitor and/or at least one DP IV-inhibitor and/or at least one NPY-receptor-ligand and/or at least one ACE-inhibitor, can be used as the active ingredient(s). The active ingredient(s) is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included.

Injectable suspensions may also prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient(s) necessary to deliver an effective dose as described above. The pharmaceutical compositions herein will contain, per dosage unit, e.g., tablet, capsule, powder, injection, suppository, teaspoonful and the like, from about 0.03 mg to 100 mg/kg (preferred 0.1 - 30 mg/kg) and may be given at a dosage of from about 0.1 - 300 mg/kg per day (preferred 1 - 50 mg/kg per day) of each active ingredient or combination thereof. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 500 mg of each active ingredient or combinations thereof of the present invention.

The tablets or pills of the compositions of the present invention can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

This liquid forms in which the compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinylpyrrolidone or gelatin.

Where the processes for the preparation of the compounds of the present invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their components enantiomers by standard techniques, such as the formation of diastereomeric pairs by salt formation with an optically active acid, such as (-)-di-p-toluoyl-d-tartaric acid and/or (+)-di-p-toluoyl-I-tartaric acid followed by fractional crystallization and regeneration of the free base. The compounds may also resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using conventional methods known from the art.

The method of treating neuronal disorders as described in the present invention, may also be carried out using a pharmaceutical composition of at least one effector of QC optionally in combination with at least one with at least one agent, selected from the group consisting of PEP-inhibitors, inhibitors of DP IV/DP IV-like enzymes, NPY-receptor ligands, NPY agonists, NPY antagonists, ACE-inhibitors, PIMT enhancers, inhibitors of beta secretases, inhibitors of gamma secretases and inhibitors of neutral endopeptidase or any other of the compounds as defined herein and a pharmaceutically acceptable carrier. The pharmaceutical composition may contain between about 0.01 mg and 100 mg, preferably about 5 to 50 mg, of each compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or betalactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitable flavored suspending or dispersing agents such as the synthetic and natural gums, for example, tragacanth, acacia, methyl-cellulose and the like. For parenteral administration, sterile suspensions and solutions are desired. Isotonic preparations which generally contain suitable preservatives are employed when intravenous administration is desired.

The compounds or combinations of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phosphatidylcholines.

Compounds or combinations of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamidephenol, polyhydroxyethylaspart-amidephenol, or polyethyl eneoxidepolyllysine substituted with palmitoyl residue. Furthermore, the compounds of the present invention may be coupled to a class of biodegradable polymers useful in achieving controlled release of a drug, for example, polyactic acid, polyepsilon caprolactone, polyhydroxy butyeric acid, polyorthoesters, polyacetals, polydihydropyrans, polycyanoacrylates and cross-linked or amphipathic block copolymers of hydrogels.

Compounds or combinations of this invention may be administered in any of the foregoing compositions and according to dosage regimens established in the art whenever treatment of the addressed disorders is required.

The daily dosage of the products may be varied over a wide range from 0.01 to 1.000 mg per mammal per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250 and 500 milligrams of each active ingredient or combinations thereof for the symptomatic adjustment of the dosage to the patient to be treated. An effective amount of the drug is ordinarily supplied at a dosage level of from about 0.1 mg/kg to about 300 mg/kg of body weight per day. Preferably, the range is from about 1 to about 50 mg/kg of body weight per day. The compounds or combinations may be administered on a regimen of 1 to 4 times per day.

Optimal dosages to be administered may be readily determined by those skilled in the art, and will vary with the particular compound used, the mode of administration, the strength of the preparation, the mode of administration, and the advancement of disease condition. In addition, factors associated with the particular patient being treated, including patient age, weight, diet and time of administration, will result in the need to adjust dosages.

Suitably, the particularly beneficial effect on glycaemic control provided by the treatment of the invention is an improved therapeutic ratio for the combination of the invention relative to the therapeutic ratio for one compound of the combination when used alone and at a dose providing an equivalent efficacy to the combination of the invention.

In a preferred aspect, the particularly beneficial effect on glycaemic control provided by the treatment of the invention is indicated to be a synergistic effect relative to the control expected from the effects of the individual active agents.

In a further aspect of the invention, combining doses of at least one QC-inhibitor with at least one PEP-inhibitor and/or at least one DP IV-inhibitor and/or at least one NPY-receptor-ligand will produce a greater beneficial effect than can be achieved for either agent alone at a dose twice that used for that agent in the combination.

In a preferred aspect, the dosage level of each of the active agents when used in accordance with the treatment of the invention will be less than would have been required from a purely additive effect upon the neuronal condition.

It is also considered that the treatment of the invention will effect an improvement, relative to the individual agents, in decreasing the intracellular deposition of pGlu-amyloid-β-peptides and thereby dramatically slowing down the plaque formation in the brain of a mammal, preferably in human brain.

In a further aspect, the invention also provides a process for preparing a pharmaceutical composition comprising at least one effector of QC optionally in combination with at least one PEP-inhibitor and/or at least one DP IV-inhibitor and/or at least one NPY-receptor-ligand and/or at least one ACE-inhibitor and a pharmaceutically acceptable carrier therefor, which process comprises admixing the QC effector and/or DP IV-inhibitor and/or the PEP-inhibitor and/or the NPY-receptor-ligand and/or the ACE-inhibitor and a pharmaceutically acceptable carrier.

The compositions are preferably in a unit dosage form in an amount appropriate for the relevant daily dosage.

Suitable dosages, including especially unit dosages, of the QC-inhibitor, the PEP-inhibitor, the DP IV-inhibitor and the NPY-receptor-ligand include the known dosages including unit doses for these compounds as described or referred to in reference text such as the British and US Pharmacopoeias, Remington's Pharmaceutical Sciences (Mack Publishing Co.), Martindale The Extra Pharmacopoeia (London, The Pharmaceutical Press) (for example see the 31 st Edition page 341 and pages cited therein) or the above mentioned publications.

### Examples of the invention

### Example 1: Solid-phase synthesis of peptides

The peptides used herein were synthesized with an automated synthesizer SYMPHONY (RAININ) using a modified Fmoc-protocol. Cycles were modified by using double couplings from the 15^{th} amino acid from the C-terminus of the peptide with fivefold excess of Fmoc-amino acids and coupling reagent. The peptide couplings were performed by TBTU/NMM-activation using a 0.23 mmol substituted NovaSyn TGR-resin or the corresponding preloaded Wang-resin at 25 µmol scale. The cleavage from the resin was carried out by a cleavage-cocktail consisting of 94.5 % TFA, 2.5 % water, 2.5 % EDT and 1 % TIS.

Analytical and preparative HPLC were performed by using different gradients on the LiChrograph HPLC system of Merck-Hitachi. The gradients were made up from two solvents: (A) 0.1 % TFA in H₂O and (B) 0.1 % TFA in acetonitrile. Analytical HPLC were performed under the following conditions: solvents were run (1 ml/min) through a 125-4 Nucleosil RP18-column, over a gradient from 5 %-50 % B over 15 min and then up to 95 % B until 20 min, with UV detection (λ = 220 nm). Purification of the peptides was carried out by preparative HPLC on either a 250-20 Nucleosil 100 RP8-column or a 250-10 LiChrospher 300 RP18-column (flow rate 6 ml/min, 220 nm) under various conditions depending on peptide chain length.

For the identification of the peptides and peptide analogues, laser desorption mass spectrometry was employed using the HP G2025 MALDI-TOF system of Hewlett-Packard.

### Example 2: Determination of IC₅₀-values of DP IV-inhibitors

100 µl inhibitor stock solution were mixed with 100 µl buffer (HEPES pH 7.6) and 50 µl substrate (Gly-Pro-pNA, final concentration 0.4 mM) and preincubated at 30°C. Reaction was started by addition of 20 µl purified porcine DP IV. Formation of the product pNA was measured at 405 nm over 10 min using the HTS 7000Plus plate reader (Perkin Elmer) and slopes were calculated. The final inhibitor concentrations ranged between 1 mM and 30 nM.

For calculation of IC₅₀-values GraFit 4.0.13 (Erithacus Software) was used.

### Example 3: Determination of Kᵢ-values of DP IV-inhibitors

For determination of the Kᵢ-values DP IV activity was measured in the same way as described in example 2 at final substrate concentrations of 0.05, 0.1, 0.2, and 0.4 mM and further 7 inhibitor concentrations covering the IC₅₀ concentration. Calculations were performed using the GraFit Software.

### Example 4: Prolyl endopeptidase (PEP) enzymatic activity assays

The enzymatic activity of PEP was quantified as described recently (Schulz et al., 2002, Modulation of inositol 1,4,5-triphosphate concentration by prolyl endopeptidase inhibition. Eur J Biochem 269: 5813-5820). Cellular extracts as described above were incubated in the assay buffer using the fluorogenic substrate Z-Gly-Pro-NHMec (10 µM; Bachem, Heidelberg, Germany) on a spectrofluorimeter SFM 25 (excitation wavelength 380 nm, emission wavelength 460 nm, Kontron, Neufahrn, Germany) equipped with a four-cell changer and controlled by an IBM-compatible personal computer. The data obtained were analyzed with the software FLUCOL (Machleidt et al., 1995).

### Example 5: Assays for glutaminyl cyclase activity

### Fluorometric assays

All measurements were performed with a BioAssay Reader HTS-7000Plus for microplates (Perkin Elmer) at 30 °C. QC activity was evaluated fluorometrically using H-Gln-βNA. The samples consisted of 0.2 mM fluorogenic substrate, 0.25 U pyroglutamyl aminopeptidase (Unizyme, Hørsholm, Denmark) in 0.2 M Tris/HCl, pH 8.0 containing 20 mM EDTA and an appropriately diluted aliquot of QC in a final volume of 250 µl. Excitation/emission wavelengths were 320/410 nm. The assay reactions were initiated by addition of glutaminyl cyclase. QC activity was determined from a standard curve of β-naphthylamine under assay conditions. One unit is defined as the amount of QC catalyzing the formation of 1 µmol pGlu-βNA from H-Gln-βNA per minute under the described conditions.

In a second fluorometric assay, QC was activity was determined using H-Gln-AMC as substrate. Reactions were carried out at 30°C utilizing the NOVOStar reader for microplates (BMG labtechnologies). The samples consisted of varying concentrations of the fluorogenic substrate, 0.1 U pyroglutamyl aminopeptidase (Qiagen) in 0.05 M Tris/HCl, pH 8.0 containing 5 mM EDTA and an appropriately diluted aliquot of QC in a final volume of 250 µl. Excitation/emission wavelengths were 380/460 nm. The assay reactions were initiated by addition of glutaminyl cyclase. QC activity was determined from a standard curve of 7-amino-4-methylcoumarin under assay conditions. The kinetic data were evaluated using GraFit sofware.

### Spectrophotometric assay of QC

This novel assay was used to determine the kinetic parameters for most of the QC substrates. QC activity was analyzed spectrophotometrically using a continuous method, that was derived by adapting a previous discontinuous assay (Bateman, R. C. J. 1989 J Neurosci Methods 30, 23-28) utilizing glutamate dehydrogenase as auxiliary enzyme. Samples consisted of the respective QC substrate, 0.3 mM NADH, 14 mM α-Ketoglutaric acid and 30 U/ml glutamate dehydrogenase in a final volume of 250 µl. Reactions were started by addition of QC and persued by monitoring of the decrease in absorbance at 340 nm for 8-15 min. Typical time courses of product formation are presented in Figure 1.

The initial velocities were evaluated and the enzymatic activity was determined from a standard curve of ammonia under assay conditions. All samples were measured at 30°C, using either the SPECTRAFluor Plus or the Sunrise (both from TECAN) reader for microplates. Kinetic data was evaluated using GraFit software.

### Inhibitor assay

For inhibitor testing, the sample composition was the same as described above, except of the putative inhibitory compound added. For a rapid test of QC-inhibition, samples contained 4 mM of the respective inhibitor and a substrate concentration at 1 K_{M}. For detailed investigations of the inhibition and determination of Kᵢ-values, influence of the inhibitor on the auxiliary enzymes was investigated first. In every case, there was no influence on either enzyme detected, thus enabling the reliable determination of the QC inhibition. The inhibitory constant was evaluated by fitting the set of progress curves to the general equation for competitive inhibition using GraFit software.

### Example 6: MALDI-TOF mass spectrometry

Matrix-assisted laser desorption/ionization mass spectrometry was carried out using the Hewlett-Packard G2025 LD-TOF System with a linear time of flight analyzer. The instrument was equipped with a 337 nm nitrogen laser, a potential acceleration source (5 kV) and a 1.0 m flight tube. Detector operation was in the positive-ion mode and signals were recorded and filtered using LeCroy 9350M digital storage oscilloscope linked to a personal computer. Samples (5 µl) were mixed with equal volumes of the matrix solution. For matrix solution we used DHAP/DAHC, prepared by solving 30 mg 2',6'-dihydroxyacetophenone (Aldrich) and 44 mg diammonium hydrogen citrate (Fluka) in 1 ml acetonitrile/0.1% TFA in water (1/1, v/v). A small volume (≈ 1 µl) of the matrix-analyte-mixture was transferred to a probe tip and immediately evaporated in a vacuum chamber (Hewlett-Packard G2024A sample prep accessory) to ensure rapid and homogeneous sample crystallization.

For long-term testing of Glu¹-cyclization, Aβ-derived peptides were incubated in 100µl 0.1 M sodium acetate buffer, pH 5.2 or 0.1 M Bis-Tris buffer, pH 6.5 at 30°C. Peptides were applied in 0.5 mM [Aβ(3-11)a] or 0.15 mM [Aβ(3-21)a] concentrations, and 0.2 U QC was added all 24 hours. In case of Aβ(3-21)a, the assays contained 1 % DMSO. At different times, samples were removed from the assay tube, peptides extracted using ZipTips (Millipore) according to the manufacturer's recommendations, mixed with matrix solution (1:1 v/v) and subsequently the mass spectra recorded. Negative controls did either contain no QC or heat deactivated enzyme. For the inhibitor studies the sample composition was the same as described above, with exception of the inhibitory compound added (5 mM benzimidazole or 2 mM 1,10-phenanthroline).

### Example 7: Formation of amyloid β-peptide (3-40/42) derivatives

The measurements were carried out with two short N-terminal peptide sequences of amyloid β-peptide(3-40/42), [Gln³]-amyloid β-peptide(1-11) (sequence: DAQFRHDSGYE) and [Gln³]-amyloid β-peptide(3-11), which contain a glutamine instead of an glutamic acid residue in the third position. Cleavage by DP IV and cyclization of the N-terminal glutamine residue by QC of the two peptides was tested using MALDI-TOF mass spectrometry. Measurements were carried out using purified DP IV (porcine kidney) or crude porcine pituitary homogenate as sources of QC as well as for both enzymes for measurements of consecutive catalysis.

### Results

### 1. Formation of [Gln³]-amyloid β-peptide(3-11) from [Gln³]-amyloid β-peptide(1-11) catalysed by DPIV and its prevention by the DP IV-inhibitor Val-Pyrrolidide (Val-Pyrr)

DPIV or DPIV-like activity is cleaving [Gln³]-amyloid β-peptide(1-11) under formation of [Gln³]-amyloid β-peptide(3-11) (Figure 2). The residue in the third position is uncovered by this cleavage and becomes therefore accessible for modification by other enzymes, i.e. QC. As expected, catalysis can be completely prevented by Val-Pyrr (Figure 3).

### 2. Formation of [pGlu³]- amyloid β-peptide(3-11) from [Gln³]-amyloid β-peptide(3-11) by catalysis of QC in pituitary homogenate and prevention by 1,10-phenanthroline

Glutaminyl cyclase present in the homogenate of porcine pituitary catalyzes conversion of [Gln³]-amyloid β-peptide(3-11) to [pGlu³]-amyloid β-peptide(3-11) (Figure 4). Formation of pyroglutamyl-amyloid β-peptide(3-11) was inhibited by addition of 1,10-phenanthroline (Figure 5).

### 3. Consecutive catalysis of DPIV and QC resulting in formation of [pGlu³]-amyloid β-peptide(3-11) and prevention by Val-Pyrr and 1,10-phenanthroline

Formation of [pGlu³]-amyloid β-peptide(3-11) from [Gln³]-amyloid β-peptide(1-11) takes place after consecutive catalysis by DP IV and QC, measured in crude homogenate of porcine pituitary with added DPIV from porcine kidney (Figure 6). [pGlu³]-amyloid β-peptide(3-11) was not formed when the QC-inhibitor 1,10-phenanthroline (Figure 7) or the DP IV-inhibitor Val-Pyrr was added (Figure 8). The slight appearance of [pGlu³]-amyloid β-peptide(3-11) is due to aminopeptidase cleavage and following cyclization of the glutamine residue, also indicated by formation of [Gln³]-amyloid β-peptide(2-11).

### 4. Formation of [pGlu³]-amyloid β-peptide(3-11) in crude pituitary homogenate by catalysis of aminopeptidase(s)

Due to the formation of [pGlu³]-amyloid β-peptide(3-11) that was not dependent on DPIV catalysis, degradation of [Gln³]-amyloid β-peptide(1-11) was investigated in crude pituitary homogenate without added DPIV (Figure 9). As expected from the data in section 4, formation of [pGlu³]-amyloid β-peptide(3-11) was observed. The data show that the degradation of [Gln³]-amyloid β-peptide(1-11) may also be catalyzed by aminopeptidase(s), resulting in [pGlu³]-amyloid β-peptide(3-11). Hence, the results show that pyroglutamyl formation is an endpoint of N-terminal peptide degradation in this tissue, further supporting the role of QC in plaque formation.

### Example 8: Turnover of Gln³-Aβ peptides 3-11a; 3-21 and 3-40 by recombinant human QC

All Gln³-Aβ derived peptides tested were efficiently converted by human QC into the corresponding pyroglutamyl forms (Table 4). Due to the poor solubility of Gln³-Aβ(3-21)a and Gln³-Aβ(3-40) in aqueous solution, the determinations were carried out in presence of 1 % DMSO. The better solubility of Gln³-Aβ(3-11)a, however, allowed the kinetic analysis of the QC-catalyzed turnover in presence and absence of DMSO (Table 4). Taken together, the investigation of the Aβ peptides as QC-substrates with chain-length of 8, 18 and 37 amino acids (see Table 4) confirmed the observation that human QC-activity increases with the length of its substrates. Accordingly, Gln¹-gastrin, Gln¹-neurotensin, Gln¹-GnRH are among the best QC-substrates taking the specificity constants into account. Similarly, Gln³-Aβ(3-40) and glucagon, the largest QC-substrates investigated thus far, exhibited high second order rate constants (449 mM⁻¹s⁻¹ and 526 mM⁻¹s⁻¹ respectively) even in presence of 1% DMSO (Table 4).

Interestingly, the kinetic parameters for the conversion of the investigated amyloid peptides did not change dramatically with increasing size, suggesting only moderate effects of the C-terminal part of Aβ on QC catalysis. Therefore, due to better solubility and experimental handling, the further investigations concerning N-terminal aminopeptidase processing of these peptides were performed using the smaller fragments of Aβ, Gln³-Aβ(1-11)a, Gln³-Aβ(3-11)a and Aβ(3-11)a.

**Table 4: Kinetic parameters for conversion of N-terminally Gln-containing peptides by recombinant human QC in buffer solution containing 1% DMSO**

| Peptide | K_{M} (µM) | k_{cat} (S⁻¹) | k_{cat}/K_{M} (mM⁻¹s⁻¹) |
|---|---|---|---|
| Gln³-Aβ(3-11)a | 87 ±3^{#} | 55 ±1^{#} | 632 ±10^{#} |
| Gln³-Aβ(3-11)a | 155 ±4 | 41.4 ±0.4 | 267 ±4 |
| Gln³-Aβ(3-21)a | 162 ±12 | 62 ±3 | 383 ±10 |
| Gln³-Aβ(3-40) | 89 ±10 | 40 ±2 | 449 ±28 |
| Glucagon(3-29) | 19 ±1 | 10.0 ±0.2 | 526 ±17 |

| | | | |
|---|---|---|---|
| ^{#} Determined in absence of DMSO | | | |

### Example 9: Turnover of Aβ(3-11)a and Aβ(3-21)a by recombinant human QC

The incubation of Aβ(3-11)a and Aβ(3-21)a in presence of QC revealed that in contrast to previous work, glutamate-containing peptides can also serve as QC-substrates (Figures 10C and D). The QC-catalyzed formation of pGlu³-Aβ(3-11)a and pGlu³-Aβ(3-21)a was investigated at pH 5.2 and 6.5, respectively. If the QC-inhibitor benzimidazole was added to the solution before starting the assay by addition of QC, substrate conversion resulting in pGlu³-Aβ(3-11)a or pGlu³-Aβ(3-21)a was suppressed (Figures 10E and F). If QC was boiled before addition, formation of the pGlu-peptides was negligible (Figures 10A and B).

### Example 10: pH-dependency of the papaya QC-catalyzed cyclization of Gln-βNA and Glu-βNA

Papaya QC converted Glu-βNA in a concentration range up to 2 mM (which was limited by substrate solubility) in accordance with Michaelis-Menten kinetics (Figure 11). Inspection of turnover versus substrate concentration diagrams for the QC-catalyzed conversion of Glu-βNA, studied between pH 6.1 and 8.5, revealed that for this Glu-substrate both parameters, K_{M} and k_{cat,} changed in a pH-dependent manner (Figure 11). This is in contrast to the previously described QC-catalyzed glutamine cyclization, for which only changes in K_{M} were observed over the given pH range (Gololobov, M. Y., Song, I., Wang, W., and Bateman, R. C. (1994) Arch Biochem Biophys 309, 300-307).

Subsequently, to study the impact of the proton concentration during Glu- and Gln-cyclization, the pH-dependence of cyclization of Glu-βNA and Gln-βNA under first-order rate-law conditions (i.e. substrate concentrations far below K_{M}-values) was investigated (Figure 12). The cyclization of glutamine has a pH-optimum at pH 8.0, in contrast to the cyclization of glutamic acid which showed a pH-optimum of pH 6.0. While the specificity constants at the respective pH-optima differ approximately 80,000-fold, the ratio of QC versus EC activity around pH 6.0, is only about 8,000. The nonenzymatic pGlu-formation from Gln-βNA investigated at pH 6.0, was followed for 4 weeks and revealed a first-order rate constant of 1.2*10⁻⁷ s⁻¹. However, during the same time period, no pGlu-βNA was formed from Glu-βNA, allowing to estimate a limiting rate constant for turnover of 1.0*10⁻⁹ s⁻¹.

### Example 11: Intracellular distribution of PEP

To identify suitable cell lines for planned localization studies, different human glioma and neuronal cell lines as well as rat primary neuronal and glial cells were investigated for PEP expression and activity. In all cell lines and primary cells studied, PEP was detected by Western blotting analysis using the specific polyclonal antibody PEP-S449 (figure 13A). Using an enzymatic assay with the specific substrate Z-Gly-Pro-AMC it was shown, that rat primary neurons exhibited the highest PEP enzymatic activity (figure 13B). Much lower specific activity was detected in primary astrocytes, microglia and oligodendroglial cells (figure 13B). Among the cell lines tested, the U-343 glioma cells and SH-SY5Y neuroblastoma cells displayed the highest specific PEP activities, which were about in the range of primary astrocytes (figure 13B). A 2.5 to 5-fold lower amount of specific PEP activity was detected in the glioma cell lines LN-405, LNZ-308, T98p31 and U138-MG (figure 13B). Therefore, U-343 as well as SH-SY5Y and - in some instances - LN-405 cells were selected for the subsequent experiments described below.

To reveal the subcellular localization of PEP, different independent methods were used. First, subcellular fractions of human glioma U-343 cells and human neuroblastoma SH-SY5Y cells separated by differential centrifugation were characterized by Western blotting analysis using different antibodies against cell compartment-specific marker proteins (figure 14A). PEP protein was found exclusively in the cell crude extract (CE) an in the soluble cytosolic fraction S100, which was confirmed by PEP enzymatic activity assay in individual fractions (figure 14B). In the SH-SY5Y and U-343 cells, approximately 99% and 87% of the total activity was found in the S100 fraction, respectively. Only small traces of PEP activity or no PEP activity were detected in the particular fraction and in the conditioned media, respectively.

To reveal the intracellular distribution of endogenous PEP protein by immunocytochemistry, the monoclonal PEP antibody 4D4D6 was used. In all cell lines and primary cells investigated, PEP protein was detected. PEP-immunoreactivity was mainly found in the perinuclear space (figure 15A). Additionally, in all LN-405 cells as well as in a significant number of SH-SY5Y and U-343 cells, a typical cytoskeleton-like PEP distribution was observed (figure 15A). Using the human PEP-antisense cell line U-343(as60) and the human glioma cell line T98p31, the specificity of the used PEP antibody was validated. Both cell types have a remaining PEP activity of lower than 50% as compared to human glioma U-343 cells and displayed an identical PEP staining pattern but significantly reduced PEP immunoreactivity as compared to U-343 cells (see figure 15A for U-343(as60)).

To validate this subcellular localization of PEP using a method not based on immunocytochemical detection, PEP-EGFP fusion proteins were employed. PEP wild-type and an inactive PEP-S554A mutant EGFP fusion protein were transformed in U-343, SH-SY5Y and LN-405 cells. The wild-type EGFP-fusion vector pEGFP-N3 was used as control. After 16 hours, in all transformation samples green fluorescent cells were observed. The overexpression of the wild-type EGFP led to a homogeneous staining of the whole cell body, including the nucleus (figure 15B). In contrast, the wild-type as well as the mutant PEP-EGFP fusion proteins showed an inhomogeneous distribution, mainly with high concentration in the perinuclear space. No differences in the distribution pattern were observed between the wild-type and the mutant PEP-EGFP-fusion protein. However, in all investigated cell lines a appropriate number of cells showed a fibrillary, cytoskeleton-like distribution pattern of the expressed PEP-EGFP-fusion proteins (figure 15B). This distribution pattern corresponds well to the immunocytochemical staining results as shown in figure 15A. In agreement with the activity measurements and the Western-blotting analysis, no secretion of PEP-EGFP-fusion proteins could be detected.

The overexpression of wild-type and mutant PEP-EGFP-fusion proteins resulted in the death of all transfected cells between 2 weeks. This fact precluded the generation of cell lines stably overexpressing PEP. Concerning both variants of fusion proteins, a large number of transfected cells displayed very strong cytosolic vacuolisation followed by formation of "apoptotic bodies". Additionally, all transfected cells showed no cell division during their short life time duration. In contrast, cells which expressed only the EGFP wild-type protein have a normal proliferation rate and it was possible to maintain stable cell lines.

The specific fibrillary cytoskeleton-like distribution of PEP was confirmed by the co-localization with tubulin, a main structural component of the cytoskeleton. In comparison to the typical fibrillary cytoskeleton pattern in tubulin-labeled LN-405 cells, a globular tubulin labeling was detected in most of the U-343 cells (figure 16A). This observation is in agreement with the staining patterns for endogenous PEP and for EGFP-PEP in these cells (compare to figures 15A and 15B, respectively). Both fibrillar and globular tubulin staining patterns co-localized almost completely with the corresponding PEP immunoreactivity (figure 16A).

To further verify the relationship between the cytoskeleton architecture and the localization of PEP, the microtubuli in U-343 and LN-405 cells were depolymerised by nocodazole (Sigma, Deisenhofen, Germany) treatment. In contrast to the co-localization study, non-treated and treated cells were single labeled with monoclonal tubulin (Sigma, Deisenhofen, Germany) and PEP (4D4D6) antibody (figure 16B). Under these conditions, most of the U-343 cells displayed the typical cytoskeleton structure as observed for LN-405 cells. After the treatment with nocodazole, the fibrillary structures were completely lost in both cell lines. To test the specificity of the nocodazole effect on the microtubuli structures, treated and non-treated cells were labeled with a monoclonal calnexin antibody (1:100, Stressgen, Victoria, Can.). We observed that nocodazole treatment had no effect on the distribution pattern of the specific ER-marker protein calnexin (data not shown).

In similarity to the tubulin labeling, the PEP immunoreactivity was no longer fibrillary after nocodazole treatment (figure 16B). In U-343 cells, after the microtubuli-depolymerisation, tubulin is distributed diffusely over the whole cytoplasm, mainly localized closely to the cellular membrane. In contrast, the PEP protein was found almost exclusively in large cell membrane puffs. In nocodazole-treated LN-405 cells, the tubulin protein was distributed over the whole cell body including the nucleus. The PEP protein was distributed like the tubulin protein, but not in the nucleus. In general, the formation of membrane puffs was considerably less than in U-343 cells.

To investigate the role of the enzymatic activity for the localization of PEP, U-343 and LN-405 cells were treated for 24 hours with 5µM of the specific PEP inhibitor, Fmoc-AlaPyrr-CN, and than labeled with the monoclonal PEP and tubulin (Sigma, Deisenhofen, Germany) antibodies. The complete inhibition of PEP enzymatic activity did not lead to any change in the tubulin or in the PEP localization pattern compared to non-treated cells.

### Example 12: Effects of PEP inhibition on protein secretion and on β-amyloid distribution

To test the effect of PEP inhibition on protein secretion, metabolic labeling experiments were performed under conditions of pharmacological inhibition of PEP activity (Schulz et al., 2002, Modulation of inositol 1,4,5-triphosphate concentration by prolyl endopeptidase inhibition. Eur J Biochem 269: 5813-5820).

Inhibition of PEP enzymatic activity resulted in a 2fold (197±27%) and 1,8fold (181±19%) increase in the protein secretion from U-343 and SH-SY5Y cells in a 24 hour period, respectively (figure 17A). Separation of secreted proteins by gel electrophoresis and subsequent detection of radioactive bands demonstrated that increased protein secretion includes many different proteins over a wide molecular weight range.

Because β-amyloid peptides are among the proteins processed in the secretory pathway and there has been a recent debate on the regulation of APP processing by PEP activity, the experimental system of PEP inhibition was applied to the analyses of β-amyloid secretion in U-343 and SH-SY5Y cells. Completely inhibition of PEP in human U-343 cells resulted in an increase of β-amyloid peptides in the conditioned medium (figure 18). After 24 hours, the amount of β-amyloid 1-40 and 1-42 (8,6±1,2 and 4,8±1,1 pg/ml per 10⁶ cells) was up to 4,3fold higher than the concentration measured in control samples (2,7±0,7 and 1,1±0,3 pg/ml per 10⁶ cells). Similar but less pronounced alterations in the secreted levels of β-amyloid peptides 1-40 and 1-42 (3,6±0,6 and 4,2±0,5 pg/ml per 10⁶ cells) were observed in treated SH-SY5Y cells in comparison to non-treated cells (2,2±0,4 and 1,9±0,6 pg/ml per 10⁶ cells). Independent from cell lines used, the intracellular concentration of β-amyloid 1-42 peptides were unaffected. In contrast, the amount of β-amyloid 1-40 peptides were lowered at 20 % in PEP inhibitor treated U343 and SH-SY5Y cells (86,7±9,9 and 156,7±28,5 pg/µg protein) in comparison to non-treated cells (111,2±11,4 and 127,0±12,7 pg/µg protein). Due to the large variance in background levels, decrease in beta-amyloid 1-40 was not significant in SH-SY5Y cells Togehther, the above experiments clearly demonstrate that β-amyloid peptides are among the proteins more abundantly secreted after inhibition of PEP enzymatic activity.

### Example 13: PEP expression in mouse brain

To reveal the distribution and the cellular source of PEP in brain, immunohistochemical labeling using the monoclonal PEP antibody 4D4D6 was performed in coronal mouse brain sections. PEP was primarily expressed by neurons and detected troughout the mouse brain. PEP-immunoreactivity was present in neuronal cytoplasm and axonal and dendritic processes, closely resembling the subcellular localization of PEP in rat primary neurons (figure 19A; compare figure 15A).

PEP expression in different brain regions was compared by Western blotting analysis and by an enzymatic PEP activity assay. Western blot analysis and densitometric quantification of optical density readings revealed the highest PEP expression in cerebellum of adult (8-months-old) mice and lower PEP expression in parietal cortex and hippocampus. In aged, 17-months-old mice, PEP protein levels were unchanged compared to the adult mice, with the exception of the hippocampus, which demonstrated an up-regulation of PEP expression by about 30% (figure 19B). These results were mirrored by those derived from the quantification of PEP enzymatic activity in different brain regions. In adult mice, the highest PEP activity was detected in cerebellum (16 mU/mg protein), followed by parietal cortex (11 mU/mg protein) and hippocampus (10 mU/mg protein). In aged mice, PEP enzymatic activity significantly increased in hippocampal tissue, but remained unchanged in the other brain regions studied (figure 19C).

### Example 14: PEP expression in human brain

In human brain PEP was selectively expressed by neurons as shown by immunohistochemistry. A perinuclear cytoplasmatic labeling and filamentous staining of neurites (figure 20A) was observed. In brain structures affected by β-amyloid plaque pathology in AD we detected fewer PEP-immunoreactive neurons, which were more intensely stained than in control brain and which appeared to be shrunken (figure 20A). In all AD cases investigated, a robust activation of microglial cells and astrocytes was observed in proximity to β-amyloid plaques. However, neither activated microglial cells nor reactive astroyctes expressed PEP as demonstrated by dual immunofluorescent labeling of PEP and glial markers using confocal laser scanning microscopy (figure 20A).

Total protein levels and enzymatic activity of PEP in parietal cortex were unaltered in AD brain as compared to age-matched control brain specimens (figure 20B and 20C).

### Example 15: β-secretase assay

The β-secretase assay was carried out using the BACE activity assay Kit (Calbiochem Cat.No. 565785) and the fluorescence quenched substrates RE(Edans)EVKMDAEFK(Dabcyl)Ra which corresponds to the wild type sequence of APP; and RE(Edans)EVKMisoDAEFK(Dabcyl)Ra which corresponds to the respective isoAsp form of APP. Cell extracts from SY5Y or U344 cells were prepared using the extraction buffer of the kit. Cell extraction and assay procedure were carried out according the manufacturer's protocol except the used substrate (see above). Hydrolysis of the substrate was monitored using a GENiusPro fluorescence microplate reader (TECAN) and excitation and emission wavelength of 340 and 495 nm, respectively. Activity in RFU/min was calculated by linear regression of the linear part of the time-response-curve.

## Claims

1. A pharmaceutical composition comprising at least one glutaminyl cyclase inhibitor in combination with at least one agent, selected from the group consisting of PEP-inhibitors, dipeptidyl aminopeptidases, NPY-receptor ligands, NPY agonists, NPY antagonists, ACE inhibitors, PIMT enhancers, inhibitors of beta secretases, inhibitors of gamma secretases and inhibitors of neutral endopeptidase, and at least one pharmaceutically acceptable carrier.

2. The pharmaceutical composition according to claim 1 for use in the treatment and/or prevention of neuronal diseases in a mammal.

3. Use of the pharmaceutical composition according to claim 1 for the preparation of a medicament for the treatment and/or prevention of neuronal diseases in a mammal.

4. The pharmaceutical composition or the use according to any one of claims 1, 2 or 3, wherein the neuronal disease is selected from the group consisting of Alzheimer's disease, Down Syndrome, Parkinson disease, Chorea Huntington, pathogenic psychotic conditions, schizophrenia, impaired food intake, sleep-wakefulness, impaired homeostatic regulation of energy metabolism, impaired autonomic function, impaired hormonal balance, impaired regulation, body fluids, hypertension, fever, sleep dysregulation, anorexia, anxiety related disorders including depression, seizures including epilepsy, drug withdrawal and alcoholism, neurodegenerative disorders including cognitive dysfunction and dementia.

5. The pharmaceutical composition or the use according to any one of the preceding claims, wherein the inhibitor of dipeptidyl aminopeptidases is an inhibitor of DP IV and/or DP IV-like enzymes.

6. The pharmaceutical composition or the use according to claim 5, wherein the inhibitor of DP IV and/or DP IV-like enzymes is selected from the group consisting of L-threo-isoleucyl pyrrolidine, L-allo-isoleucyl thiazolidine, L-allo-isoleucyl pyrrolidine, valine pyrrolidine, NVP-DPP728A (1-[ [ [ 2-[ {5-cyanopyridin-2-yl}amino]ethyl]amino]acetyl]-2-cyano-(S)-pyrrolidine) LAF-237 (1-[(3-hydroxy-adamant-1-ylamino)-acetyl]-pyrrolidine-2(S)-carbonitrile); TSL-225 (tryptophyl-1,2,3,4-tetrahydroisoquinoline-3-carboxylic acid), or FE-999011 ( [(2S)-1-([2'S]-2'-amino-3',3'dimethyl-butanoyl)-pyrrolidine-2-carbonitrile] ), MK-0431 ( (2R)-4-Oxo-4-[3-(trifluoromethyl)-5,6-dihydro[1,2,4]triazolo[4,3-a]pyrazin-7(8H)-yl]-1-(2,4,5-trifluorophenyl)butan-2-amine ) and pharmaceutical acceptable salts thereof, and at least one pharmaceutically acceptable carrier.

7. The pharmaceutical composition or the use according to any one of claims 1 to 4, wherein said NPY antagonist is selected from the group consisting of 3a,4,5,9b-tetrahydro-1h-benz[e]indo!-2-y! amine-derived compounds, BIBP3226 and, (R)-N2-(diphenylacetyl)-(R)-N-[1-(4-hydroxy- phenyl) ethyl] arginine amide.

8. The pharmaceutical composition or the use according to any one of claims 1 to 4, wherein said PEP-inhibitor is selected from the group consisting of chemical derivatives of proline or small peptides containing terminal prolines, e.g. benzyloxycarbonyl-prolyl-prolinal, N-terminal substituted L-proline or L-prolylpyrrolidine, substituted N-benzyloxycarbonyl (Z) dipeptides containing prolinal at the carboxy terminus, substituted thioprolines, substituted thiazolidines, substituted oxopyrrolidines, carboxy terminal modified prolines including fluorinated ketone derivatives, chloromethyl ketone derivatives of acyl-proline or acylpeptide-proline (Z-Gly-Pro-CH₂Cl) and 2-acylpyrrolidine derivatives.

9. The pharmaceutical composition or the use according to any one of claims 1 to 4, wherein said PEP-inhibitor is selected from the group consisting of Fmoc-Ala-Pyrr-CN, Z-321, ONO-1603, JTP-4819 and S-17092.

10. The pharmaceutical composition or the use according to any one of claims 1 to 4, wherein said ACE-inhibitor is SDZ ENA 713 (rivastigmine (+)-(S)-N-ethyl-3-[(1-dimethylamino)ethyl]-N-methylphenylcarbamate hydrogen tartrate.

11. The pharmaceutical composition or the use according to any one of claims 1 to 4, wherein said PIMT enhancer is a 10-aminoaliphatyl-dibenz[b, f] oxepines of the general formula 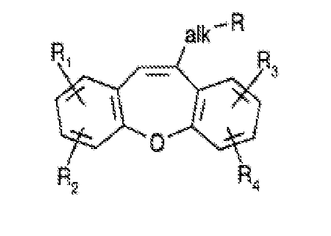 wherein alk is a divalent aliphatic radical, R is an amino group that is unsubstituted or mono- or di-substituted by monovalent aliphatic and/or araliphatic radicals or disubstituted by divalent aliphatic radicals, and R₁, R₂, R₃ and R₄ are each, independently of the others, hydrogen, lower alkyl, lower alkoxy, halogen or trifluoromethyl.

12. The pharmaceutical composition or the use according to any one of claims 1 to 4, wherein said gamma secretase inhibitor is (5S)-(t-Butoxycarbonylamino)-6-phenyl-(4R)hydroxy-(2R)benzylhexanoyl)-L-leu-L-phe-amide having the formula

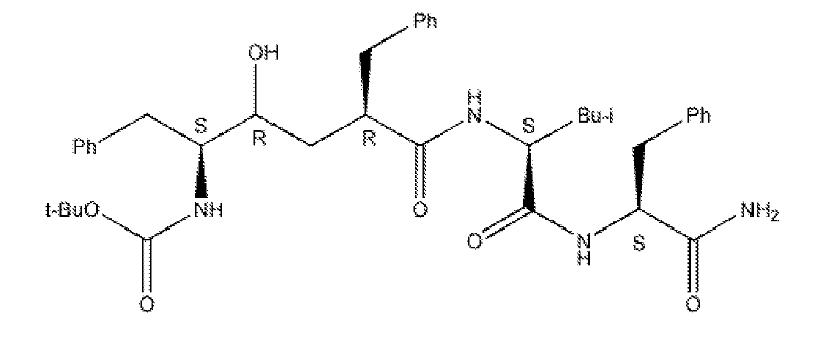

13. The pharmaceutical composition or the use according to any one of claims 1 to 4, wherein said beta secretase inhibitor is PNU-33312 having the formula

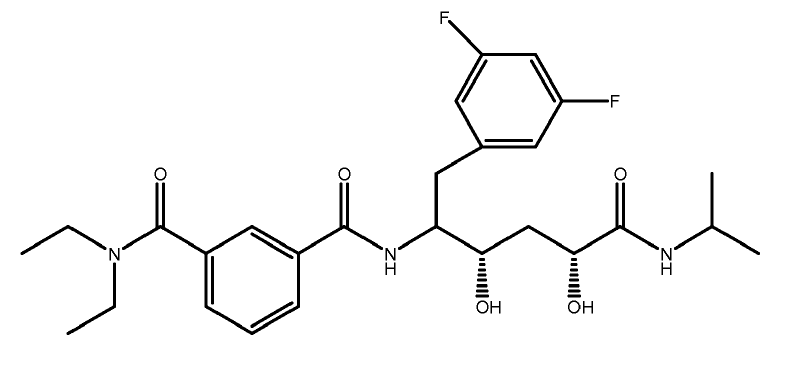

14. The pharmaceutical composition or the use according to any one of the preceding claims, wherein the glutaminyl cyclase inhibitor is a compound of formula 10: 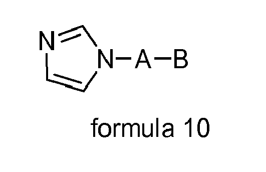 wherein
A is a branched or unbranched C₁-C₇ alkyl chain, a branched or unbranched C₁-C₇ alkenyl chain, a branched or unbranched C₁-C₇ alkynyl chain;
or A is a compound selected from the group consisting of : 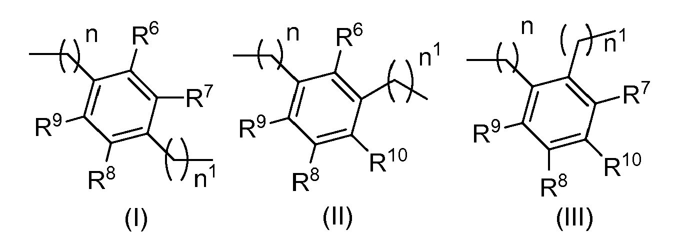 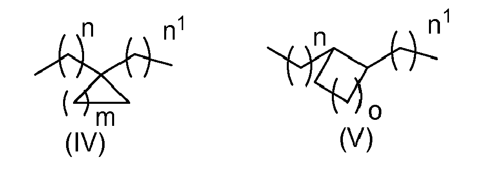 wherein
R⁶-R¹⁰ are independently H or a branched or unbranched alkyl chain, a branched or unbranched alkenyl chain, a branched or unbranched alkynyl chain, a carbocycle, aryl, heteroaryl, a heterocycle;
n and n¹ are independently 1 - 5, m is 1 - 5, o is 0 - 4;
B is a compound selected from the group consisting of 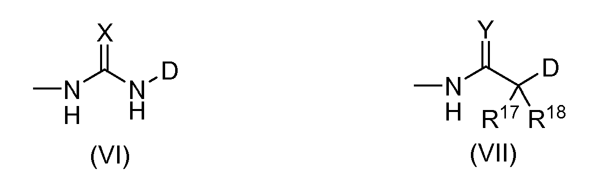 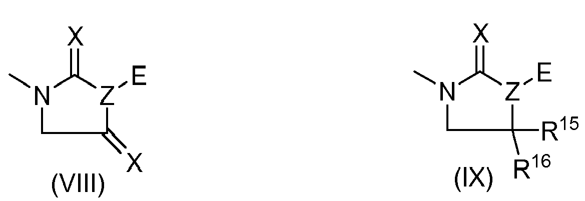 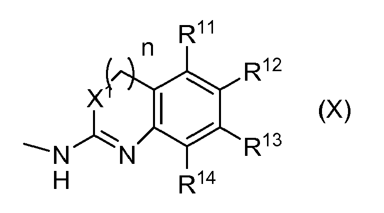 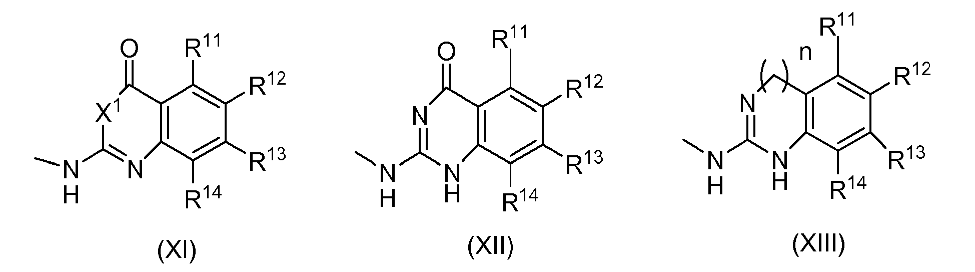 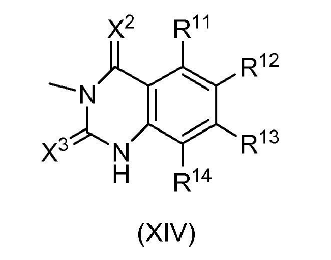 wherein
D and E are a branched or unbranched alkyl chain, a branched or unbranched alkenyl chain, a branched or unbranched alkynyl chain, a carbocycle, aryl, heteroaryl or a heterocycle;
Z is CH or N;
X can be O, S or N-CN, with the proviso for formulas (VIII) and (IX) that, if Z = CH,XisOorS;
X¹, X² and X³ are independently O or S;
Y is O or S;
R¹¹-R¹⁴ are independently H or a branched or unbranched alkyl chain, a branched or unbranched alkenyl chain, a branched or unbranched alkynyl chain, a carbocycle, aryl, heteroaryl, a heterocycle, halogenyl, oxyalkyl, thioalkyl, carboxyl, carboxylic acid ester, carbonyl, carbamide, carbimide, thiocarbamide or thiocarbonyl;
R¹⁵ and R¹⁶ are independently H or a branched or unbranched alkyl chain, or a branched or unbranched alkenyl chain;
R¹⁷ and R¹⁸ are independently H or a branched or unbranched alkyl chain, a branched or unbranched alkenyl chain, a branched or unbranched alkynyl chain, a carbocycle, aryl or can be connected to form a carbocycle with up to 6 ring atoms; and
n is 0 or 1 ;
all of the above residues being optionally substituted independently of each other.

15. The pharmaceutical composition or the use according to any one of the preceding claims, wherein the carrier is for parenteral or enteral application.
